# EUROPÄISCHE PATENTANMELDUNG

(11) **EP 1 431 393 A1**
(43) Veröffentlichungstag der Anmeldung: **23.06.2004**
(21) Anmeldenummer: 02028530.0
(22) Anmeldetag: 19.12.2002
(51) Int. Cl.: C12N 15/54, C12N 15/82, C12N 5/10, A01H 5/00, A01H 5/10, A23L 1/0522

(54) **Pflanzen, die eine Stärke mit erhöhter Endviskosität synthetisieren**

(71) Anmelder: Bayer CropScience GmbH, 65929 Frankfurt/Main (DE)
(72) Erfinder: Landschütze, Volker, Dr., 12203 Berlin (DE); Frohberg, Claus, Dr., 14432 Kleinmachnow (DE); Hoehne, Michaela, Dr., 1800 Vevey (CH)

(57) **Zusammenfassung**

Die vorliegende Erfindung betrifft eine Pflanzenzelle, die genetisch modifiziert ist, wobei die genetische Modifikation zur Verringerung der Aktivität eines oder mehrerer endogen in der Pflanzenzelle vorkommenden SSIII-Proteine und zur Verringerung der Aktivität eines oder mehrerer endogen in der Pflanzenzelle vorkommenden BEI-Proteine und zur Verringerung der Aktivität eines oder mehrerer endogen in der Pflanzenzelle vorkommenden BEII-Proteine führt im Vergleich zu entsprechenden nicht genetisch modifizierten Pflanzenzellen von Wildtyppflanzen. Weitere Aspekte der Erfindung betreffen solche Pflanzenzellen enthaltende Pflanzen, ein Verfahren zur Herstellung der Pflanzenzellen und Pflanzen sowie die daraus erhältliche Stärke.

## Beschreibung

### Beschreibung

Die vorliegende Erfindung betrifft Pflanzenzellen und Pflanzen, die genetisch modifiziert sind, wobei die genetische Modifikation zur Verringerung der Aktivität von SSIII- und BEI- und BEII-Proteinen im Vergleich zu entsprechenden nicht genetisch modifizierten Pflanzenzellen von Wildtyppflanzen führt. Ferner betrifft die vorliegende Erfindung Mittel und Verfahren zur Herstellung solcher Pflanzenzellen und Pflanzen. Derartige Pflanzenzellen und Pflanzen synthetisieren eine modifizierte Stärke, die dadurch gekennzeichnet ist, dass sie einen Amylosegehalt von mindestens 30% und einen im Vergleich zu Stärke von entsprechenden nicht genetisch modifizierten Wildtyp-Pflanzen einen erhöhten Phosphatgehalt und eine gegenüber dem Stand der Technik erhöhte Endviskosität in der RVA Analyse und/oder eine veränderte Seitenkettenverteilung und/oder eine erhöhte Gelfestigkeit im Texture Analyser und/oder eine veränderte Stärkekornmorphologie und/oder eine veränderte mittlere Stärkekorngröße aufweist. Die vorliegende Erfindung betrifft daher auch die von den erfindungsgemäßen Pflanzenzellen und Pflanzen synthetisierte Stärke sowie Verfahren zur Herstellung dieser Stärke.

Im Hinblick auf die zunehmende Bedeutung, die pflanzlichen Inhaltsstoffen als erneuerbaren Rohstoffquellen zur Zeit beigemessen wird, ist es eine der Aufgaben der biotechnologischen Forschung, sich um eine Anpassung dieser pflanzlichen Rohstoffe an die Anforderungen der verarbeitenden Industrie zu bemühen. Um eine Anwendung von nachwachsenden Rohstoffen in möglichst vielen Einsatzgebieten zu ermöglichen, ist es darüber hinaus erforderlich, eine große Stoffvielfalt zu erreichen.

Das Polysaccharid Stärke ist ein Polymer aus chemisch einheitlichen Grundbausteinen, den Glukosemolekülen. Es handelt sich dabei jedoch um ein sehr komplexes Gemisch aus unterschiedlichen Molekülformen, die sich hinsichtlich ihres Polymerisationsgrades und des Auftretens von Verzweigungen der Glukoseketten unterscheiden. Daher stellt Stärke keinen einheitlichen Rohstoff dar. Man unterscheidet zwei chemisch unterschiedliche Komponenten der Stärke: die Amylose und das Amylopektin. In typischen für die Stärkeproduktion verwendeten Pflanzen, wie z.B. Mais, Weizen oder Kartoffel, besteht die synthetisierte Stärke zu ca. 20% - 30% aus Amylose-Stärke und zu ca. 70% - 80% aus Amylopektin-Stärke.
Amylose wurde lange als lineares Polymer, bestehend aus α-1,4-glycosidisch verknüpften α-D-Glukose-Monomeren, angesehen. In neueren Studien wurde jedoch die Anwesenheit von α-1,6-glycosidischen Verzweigungspunkten (ca. 0,1%) nachgewiesen (Hizukuri und Takagi, Carbohydr. Res. 134, (1984), 1-10; Takeda et al., Carbohydr. Res. 132, (1984), 83-92).

Es stehen unterschiedliche Methoden zur Bestimmung des Amylosegehaltes zur Verfügung. Einige dieser Methoden basieren auf dem lodbindevermögen der Amylose, das potentiometrisch (Banks & Greenwood, in W. Banks & C.T. Greenwood, Starch and its components (pp. 51-66), Edinburgh, Edinburgh University Press), amperometrisch (Larson et al., Analytical Chemistry 25(5), (1953), 802-804) oder spektrophotometrisch (Morrison & Laignelet, J. Cereal Sc. 1, (1983), 9-20) bestimmt werden kann. Die Bestimmung des Amylosegehaltes kann auch kalorimetrisch mittels DSC-(Differential Scanning Calorimetry)-Messungen erfolgen (Kugimiya & Donovan, Journal of Food Science 46, (1981), 765-770; Sievert & Holm, Starch/Stärke 45 (4), (1993), 136-139). Ferner besteht die Möglichkeit, den Amylosegehalt über den Einsatz von SEC-(size exclusion chromatography)- Chromatographie von nativer oder entzweigter Stärke zu bestimmen. Diese Methode wurde insbesondere zur Bestimmung des Amylosegehaltes gentechnisch modifizierter Stärken empfohlen (Gérard et al., Carbohydrate Polymers 44, (2001), 19-27).
Im Gegensatz zur Amylose ist das Amylopektin stärker verzweigt und weist ca. 4% Verzweigungspunkte auf, die durch das Auftreten von zusätzlichen α-1,6-glycosidischen Verknüpfungen zustande kommen. Das Amylopektin stellt ein komplexes Gemisch aus unterschiedlich verzweigten Glukoseketten dar.

Ein weiterer wesentlicher Unterschied zwischen Amylose und Amylopektin liegt im Molekulargewicht. Während Amylose, je nach Herkunft der Stärke, ein Molekulargewicht von 5x10⁵ - 10⁶ Da besitzt, liegt das des Amylopektins zwischen 10⁷ und 10⁸ Da. Die beiden Makromoleküle können durch ihr Molekulargewicht und ihre unterschiedlichen physiko-chemischen Eigenschaften differenziert werden, was am einfachsten durch ihre unterschiedlichen Jodbindungseigenschaften sichtbar gemacht werden kann.

Die funktionellen Eigenschaften der Stärke werden neben dem Amylose/Amylopektin-Verhältnis und dem Phosphatgehalt stark beeinflußt durch das Molekulargewicht, das Muster der Seitenkettenverteilung, den Gehalt an Ionen, den Lipid- und Proteingehalt, die mittlere Stärkekorngröße sowie die Stärkekornmorphologie etc. Als wichtige funktionelle Eigenschaften sind hierbei beispielsweise zu nennen die Löslichkeit, das Retrogradationsverhalten, das Wasserbindevermögen, die Filmbildungseigenschaften, die Viskosität, die Verkleisterungseigenschaften, die Gefrier-Tau-Stabilität, die Säurestabilität, die Gelfestigkeit etc.. Auch die Stärkekorngröße kann für verschiedene Anwendungen von Bedeutung sein.

Die Verkleisterungseigenschaften, denen auch die Endviskositat zuzurechnen ist, werden häufig vom Fachmann mit unterschiedlichen Methoden bestimmt. Je nach verwendeter Methode können sich insbesondere absolute, aber auch relative Werte für ein und dieselbe Stärkeprobe unterscheiden. Eine schnelle und effiziente Methode zur Analyse der Verkleisterungseigenschaften bietet die RVA Analyse. Je nach Wahl der Parameter und des Temperaturprofils bei der RVA Analyse erhält man unterschiedliche RVA-Profile für ein und dieselbe Probe. Es sei darauf hingewiesen, dass im folgend aufgeführten Stand der Technik teilweise unterschiedliche Profile zur Bestimmung der Verkleisterungseigenschaften verwendet wurden.

Eine Übersicht zu verschiedenen Pflanzenspezies, die eine Reduktion von an der Stärkebiosynthese beteilligten Enzymen aufweisen, findet sich bei Kossmann und Lloyd (2000, Critical Reviews in Plant Sciences 19(3), 171-126).
Bisher sind Pflanzen beschrieben, bei welchen die Aktivität eines SSIII Proteins (Abel et al., 1996, The Plant Journal 10(6), 9891-991; Lloyd et al., 1999, Biochemical Journal 338, 515-521) bzw. die Aktivität eines BEI Proteins (Kossmann et al. 1991,Mol Gen Genet 230, 39-44); Safford et al., 1998, Carbohydrate Polymers 35, 155-168, bzw. die Aktivität eines BEII Proteins (Jobling et al., 1999, The Plant Journal 18, bzw. die Aktivität eines BEI und BEII Proteins (Schwall et al., 2000, Nature Biotechnology 18, 551- 554; WO 96/34968), bzw. die Aktivität eines BEI und eines SSIII (WO 00/08184) Proteins reduziert sind .
Pflanzen, bei welchen die Aktivität eines SSIII Proteins reduziert ist, zeigen, verglichen mit entsprechenden Wildtyppflanzen, eine relative Verschiebung der Seitenketten des Amylopektins von längeren Ketten zu kurzen Ketten (Lloyd et al., 1999, Biochemical Journal 338, 515-521), einen um 70% erhöhten Phosphatgehalt, keine Veränderung des Amylosegehaltes (Abel et al., 1996, The Plant Journal 10(6), 9891-991) und eine erniedrigte Endviskosität in der RVA Analyse (Abel, 1995, Dissertation der Freien Universität Berlin). Solche Pflanzen, die auch in WO 00/08184 beschrieben sind, weisen in der isolierten Stärke gegenüber nicht transformierten Wildtyppflanzen einen um 197% gesteigerten Phosphatgehalt, einen um 123% gesteigerten Amylosegehalt und eine Endviskosität in der RVA Analyse, die auf 76% des Wildtyps sinkt, auf. Außerdem sinkt die Gelfestigkeit der betreffenden Stärke auf 84% des Wildtyps.

Pflanzen, die eine verringerte Aktivität sowohl eines BEI -, als auch eines BE II Proteins aufweisen, zeigen in der spektrophotometrischen Analyse nach Morrison & Laignelet (1983, J. Cereal Sc. 1, 9-20) einen Amylosegehalt von maximal 89,14% (entspricht 344% des Wildtyps) und einen Phosphatgehalt der Stärke, der maximal 522% desjenigen von Stärke, isoliert aus entsprechenden Wildtyp-Pflanzen, entspricht. Diese Stärken zeigten in der RVA Analyse einen um maximal 237% gesteigerten Wert der Endviskosität. Des Weiteren zeigen Stärkekörner, die aus solchen Pflanzen isoliert wurden, eine veränderte Stärkekornmorphologie, die sich dadurch auszeichnet, dass die Stärkekörner in der Mikroskopie unter polarisiertem Licht große Furchen im Zentrum des jeweiligen Korns aufwiesen.

Daraus ergibt sich, dass dem Fachmann Pflanzenzellen und Pflanzen und von diesen synthetisierte Stärken bekannt sind, welche einen erhöhten Amylose- und Phosphgatgehalt aufweisen, deren Endviskosität in der RVA Analyse jedoch nicht mehr als maximal 256% gegenüber nicht genetisch modifizierten Wildtyp Pflanzen erhöht ist. Höhere Endviskositäten in der RVA Analyse konnten bisher nicht erreicht werden. Dieses wäre jedoch wünschenswert, weil z.B. bei Verwendung einer solchen Stäke als Dickungs-, Gelier- oder Bindemittel weniger Feststoff an Stärke eingesetzt werden muß, um den gewünschten Effekt zu erzielen. Dadurch kann z.B. die Menge an Zusatzstoffen in menschlichen und tierischen Nahrungsmitteln, in die Gesundheit erhaltenden Produkten (Healthcare products) und Kosmetika reduziert werden. Auch bei der Verwendung einer solchen Stärke in Klebstoffen können geringere Mengen Stärke eingesetzt werden, was zu einer Reduzierung der Kosten z.B. bei der Herstellung von z.B. Papier, Pappe oder Dämmplatten führt.
Der vorliegenden Erfindung liegt somit die Aufgabe zugrunde, Pflanzenzellen, Pflanzen und Stärke aus entsprechenden Pflanzenzellen bzw. Pflanzen zur Verfügung zu stellen, die einen erhöhten Amylosegehalt sowie einen erhöhten Phosphatgehalt und in der RVA Analyse eine um mindestens 270% erhöhte Endviskosität und/oder eine erhöhte Gelfestigkeit der gelatinisierten Stärke und/oder eine veränderte Stärkekornmorphologie aufweisen .

Diese Aufgabe wird durch die Bereitstellung der in den Patentansprüchen bezeichneten Ausführungsformen gelöst.

Somit betrifft ein erster Aspekt der vorliegenden Erfindung eine Pflanzenzelle, die genetisch modifiziert ist, wobei die genetische Modifikation zur Verringerung der Aktivität eines oder mehrerer endogen in der Pflanzenzelle vorkommenden SSIII-Proteine und zur Verringerung der Aktivität eines oder mehrerer endogen in der Pflanzenzelle vorkommenden BEI-Proteine und zur Verringerung der Aktivität eines oder mehrerer endogen in der Pflanzenzelle vorkommenden BEII-Proteine führt im Vergleich zu entsprechenden nicht genetisch modifizierten Pflanzenzellen von Wildtyppflanzen.

Die genetische Modifikation kann dabei jede genetische Modifikation sein, die zu einer Verringerung der Aktivität eines oder mehrerer endogen in der Pflanzenzelle vorkommenden SSIII-Proteine und zur Verringerung der Aktivität eines oder mehrerer endogen in der Pflanzenzelle vorkommenden BEI-Proteine und zur Verringerung der Aktivität eines oder mehrerer endogen in der Pflanzenzelle vorkommenden BEII-Proteine führt im Vergleich zu entsprechenden nicht genetisch modifizierten Pflanzenzellen von Wildtyppflanzen.

Im Zusammenhang mit der Erfindung kann die genetische Modifikation beispielsweise die Herstellung von erfindungsgemäßen Pflanzenzellen durch Mutagenese eines oder mehrerer Gene umfassen. Die Art der Mutation ist dafür unerheblich, solange sie zu einer Reduktion der Aktivität eines SSIII-Proteins und/oder eines BEI-Proteins und/oder eines BEII-Proteins führt. Unter dem Begriff "Mutagenese" soll im Zusammenhang mit der vorliegenden Erfindung jegliche Art von Mutation verstanden werden, wie z.B. Deletionen, Punktmutationen (Nukleotidaustausche), Insertionen, Inversionen, Genkonversionen oder Chromosomentranslokation.

Die Mutation kann dabei durch den Einsatz chemischer Agentien oder energiereicher Strahlung (z.B. Röntgen-, Neutronen-, Gamma- UV-Strahlung) erzeugt werden.
Agentien, die zur Erzeugung chemisch induzierter Mutationen eingesetzt werden können und die durch Einwirkung der entsprechenden Mutagene dabei entstehenden Mutationen sind z.B. beschrieben bei Ehrenberg und Husain, 1981, (Mutation Research 86, 1-113), Müller, 1972 (Biologisches Zentralblatt 91 (1), 31-48). Die Erzeugung von Reismutanten unter Verwendung von gamma Strahlen, Ethyl-Methan-Sulfonat (EMS), N-methyl-N-Nitrosurea oder Natriumazid (NaN3) ist z.B. beschrieben in Jauhar und Siddiq (1999, Indian Journal of Genetics, 59 (1), 23-28), bei Rao (1977, Cytologica 42, 443-450), Gupta und Sharma (1990, Oryza 27, 217-219) und Satoh und Omura (1981, Japanese Journal of Breeding 31 (3), 316-326). Die Erzeugung von Weizenmutanten unter Verwendung von NaN₃ bzw. Maleic hydrazide ist in Arora et al. (1992, Anals of Biology 8 (1), 65-69) beschrieben. Eine Übersicht zur Erzeugung von Weizenmutanten unter Verwendung von verschiedenen Arten energiereicher Strahlung und chemischer Agenzien ist in Scarascia-Mugnozza et al. (1993, Mutation Breeding Review 10, 1-28) dargestellt. Svec et al. (1998, Cereal Research Communications 26 (4), 391-396) beschreibt die Anwendung von N-ethyl-N-Nitrosurea zur Erzeugung von Mutanten in Triticale. Die Verwendung von MMS und gamma-Strahlung zur Erzeugung von Hirsemutanten ist in Shashidhara et al. (1990, Journal of Maharashtra Agricultural Universities 15 (1), 20-23) beschrieben.
Die Herstellung von Mutanten in Pflanzenspezies, die sich hauptsächlich vegetativ vermehren, wurde z.B. für Kartoffeln, die eine veränderte Stärke produzieren (Hovenkamp-Hermelink et al. (1987, Theoretical and Applied Genetics 75, 217-221) und für Minze mit erhöhtem Ölertrag bzw. veränderter Ölqualität (Dwivedi et al., 2000, Journal of Medicinal and Aromatic Plant Sciences 22, 460-463) beschrieben. Alle diese Methoden sind grundsätzlich geeignet, die erfindungsgemäßen Pflanzenzellen und die von ihnen produzierte Stäke herzustellen.

Das Auffinden von Mutationen in den entsprechenden Genen, insbesondere in Genen codierend ein BEI Protein und/oder ein BEII Protein und/oder ein SSIII Protein, kann mit Hilfe von dem Fachmann bekannten Methoden geschehen. Insbesondere können hierzu Analysen, basierend auf Hybridisierungen mit Sonden (Southern Blot), der Amplifikation mittels Polymerasekettenreaktion (PCR), der Sequenzierung betreffender genomischer Sequenzen und die Suche nach einzelnen Nukleotidaustauschen angewandt werden. Eine Methode, um Mutationen anhand von Hybridisierungsmustern zu identifizieren, ist z.B. die Suche nach Restriktionsfragment Längen-Unterschieden (Restriction Fragment Length Polymorphism, RFLP) (Nam et al., 1989, The Plant Cell 1, 699-705; Leister and Dean, 1993, The Plant Journal 4 (4), 745-750). Eine auf PCR basierende Methode ist z.B. die Analyse von amplifizierten Fragment Längenunterschieden (Amplified Fragment Length Polymorphism, AFLP) (Castiglioni et al., 1998, Genetics 149, 2039-2056; Meksem et al., 2001, Molecular Genetics and Genomics 265, 207-214; Meyer et al., 1998, Molecular and General Genetics 259, 150-160). Auch die Verwendung von mit Restriktionsendonukleasen geschnittenen amplifizierten Fragmenten (Cleaved Amplified Polymorphic Sequences, CAPS) kann zur Identifizierung von Mutationen herangezogen werden (Konieczny und Ausubel, 1993, The Plant Journal 4, 403-410; Jarvis et al., 1994, Plant Molecular Biology 24, 685-687; Bachem et al., 1996, The Plant Journal 9 (5), 745-753). Methoden zur Ermittlung von SNPs sind u.a. von Qi et al. (2001, Nucleic Acids Research 29 (22), e116) Drenkard et al. (2000, Plant Physiology 124, 1483-1492) und Cho et al. (1999, Nature Genetics 23, 203-207) beschrieben worden. Insbesondere sind Methoden, die es erlauben, viele Pflanzen innerhalb kurzer Zeit auf Mutationen in bestimmten Genen hin zu untersuchen, geeignet. Solch eine Methode, das sogenannte TILLING (Targetting Induced Local Lesions IN Genomes), ist von McCallum et al. (2000, Plant Physiology 123, 439-442) beschrieben worden.

Die Verwendung aller dieser Methoden ist grundsätzlich im Rahmen der vorliegenden Erfindung geeignet.

Hoogkamp et al. (2000, Potato Research 43, 179-189) haben stabile Mutanten in Kartoffel isoliert, die eine amylosefreie Stärke enthalten. Diese Pflanzen synthetisieren kein aktives Enzym mehr für eine stärkekorngebundene Stärkesynthase (GBSS I). Nach erneuter Mutagenese dieser Pflanzen könnten solche selektiert werden, die zusätzliche Mutationen in Genen, die an der Stärkebiosynthese beteiligt sind, aufweisen. Dadurch könnten Pflanzen erzeugt werden, die Stärke mit verbesserten Eigenschaften synthetisieren. Nach der entsprechenden Methode können auch die erfindungsgemäßen Pflanzenzellen, die eine erfindungsgemäße Stärke produzieren, identifiziert und isoliert werden.

Weiterhin können die erfindungsgemäßen Pflanzenzellen auch mit Hilfe von homologen, d.h. natürlicherweise in den entsprechenden Pflanzenzellen enthaltenen, Transposons erzeugt werden. Ein detailierte Darstellung dieser Methode erfogt weiter unten.

Alle zuvor genannten Methoden sind grundsätzlich dazu geeignet, erfindungsgemäße Pflanzenzellen und die von ihnen synthetisierte modifizierte Stärke herzustellen. Daher sind Verfahren zur Herstellung genetisch modifizierter Pflanzenzellen, die eine modifizierte Stärke synthetisieren, wobei diese Stärke dadurch gekennzeichnet ist, dass sie einen Amylosegehalt von mindestens 30% enthält, im Vergleich zu Stärke aus entsprechenden nicht genetisch modifizierten Wildtyp-Pflanzenzellen einen erhöhten Phosphatgehalt aufweist und in der RVA Analyse im Vergleich zu Stärke aus entsprechenden nicht genetisch modifizierten Wildtyp-Pflanzenzellen eine erhöhte Endviskosität aufweist, auch Gegenstand der vorliegenden Erfindung.

Ein weiterer Aspekt der vorliegenden Erfindung betrifft Verfahren zur Herstellung einer Pflanzenzelle, die eine modifizierte Stärke synthetisiert, umfassend die genetische Modifikation der Pflanzenzelle, wobei die genetische Modifikation zur Verringerung der Aktivität eines oder mehrerer endogen in der Pflanzenzelle vorkommenden SSIII-Proteine und zur Verringerung der Aktivität eines oder mehrerer endogen in der Pflanzenzelle vorkommenden BEI-Proteine und zur Verringerung der Aktivität eines oder mehrerer endogen in der Pflanzenzelle vorkommenden BEII-Proteine führt im Vergleich zu entsprechenden nicht genetisch modifizierten Pflanzenzellen von Wildtyp-Pflanzen.

Noch ein weiterer Aspekt der vorliegenden Erfindung betrifft Verfahren zur Herstellung einer genetisch modifizierten Pflanze, die eine modifizierte Stärke synthetisiert, wobei
a) eine Pflanzenzelle wie vorstehend beschrieben hergestellt wird;
b) aus oder mit der gemäß a) hergestellten Pflanzenzelle eine Pflanze regeneriert wird; und
c) gegebenenfalls aus der gemäß Schritt b) erzeugten Pflanze weitere Pflanzen erzeugt werden.

Der Begriff "genetisch modifiziert" bedeutet im Zusammenhang mit der vorliegenden Erfindung, dass die Pflanzenzelle in ihrer genetischen Information verändert ist.

Dabei weisenn die erfindungsgemäßen Pflanzenzellen eine Verringerung der Aktivität eines oder mehrerer endogen in der Pflanzenzelle vorkommenden SSIII-Proteine und eine Verringerung der Aktivität eines oder mehrerer endogen in der Pflanzenzelle vorkommenden BEI-Proteine und eine Verringerung der Aktivität eines oder mehrerer endogen in der Pflanzenzelle vorkommenden BEII-Proteine auf im Vergleich zu entsprechenden nicht genetisch modifizierten Pflanzenzellen von Wildtyppflanzen.

Die genetischen Modifikationen zur Erzeugung der erfindungsgemäßen Pflanzenzellen können gleichzeitig oder in aufeinanderfolgenden Schritten erfolgen. Dabei kann jede genetische Modifikation zur Verringerung der Aktivität eines oder mehrerer SSIII-Proteine und/oder eines oder mehrerer BEI-Proteine und/oder eines oder mehrerer BEII-Proteine führen. Es kann sowohl von Wildtyp-Pflanzen bzw. -Pflanzenzellen ausgegangen werden, in denen noch keine vorherige genetische Modifikation zur Verringerung der Aktivität eines oder mehrerer SSIII-Proteine und/oder eines oder mehrerer BEI-Proteine und/oder eines oder mehrerer BEII-Proteine erfolgt ist, oder von bereits genetisch modifizierten Pflanzenzellen bzw. Pflanzen, in denen bereits durch eine genetische Modifikation die Aktivität eines oder mehrerer SSIII-Proteine und/oder eines oder mehrerer BEI-Proteine und/oder eines oder mehrerer BEII-Proteine erfolgt ist. Soweit bereits von solchen genetisch modifizierten Pflanzen(zellen) ausgegangen wird, betreffen die weiter durchgeführten genetischen Modifikationen vorzugsweise nur die Aktivität jeweils eines oder mehrerer der noch nicht in ihrer Aktivität verringerten Proteine (SSIII, BEI bzw. BEII).

Beispielsweise zeigen genetisch modifizierte erfindungsgemäße Pflanzenzellen eine Verringerung der Expression eines oder mehrerer endogen in der Pflanzenzelle vorkommenden SSIII-Gene und eine Verringerung der Expression eines oder mehrerer endogen in der Pflanzenzelle vorkommenden BEI-Gene und eine Verringerung der Expression eines oder mehrerer endogen in der Pflanzenzelle vorkommenden BEII-Gene und/oder eine Verringerung der Aktivität jeweils eines oder mehrerer der vorgenannten in der Pflanzenzelle vorkommenden Proteine im Vergleich zu entsprechenden nicht genetisch modifizierten Pflanzenzellen von Wildtyppflanzen.

Der Begriff "Verringerung der Aktivität" bedeutet dabei im Rahmen der vorliegenden Erfindung eine Verringerung der Expression endogener Gene, die SSIII-, BEI- und/oder BEII-Proteine codieren, und/oder eine Verringerung der Menge an SSIII-, BEI- und/oder BEII-Protein in den Zellen und/oder eine Verringerung der enzymatischen Aktivität der SSIII-, BEI- und/oder BEII-Proteine in den Zellen.

Die Verringerung der Expression kann beispielsweise bestimmt werden durch Messung der Menge an SSIII-, BEI- oder BEII-Proteinen codierenden Transkripten, z.B. durch Northern-Blot-Analyse oder RT-PCR. Eine Verringerung bedeutet dabei vorzugsweise eine Verringerung der Menge an Transkripten im Vergleich zu entsprechenden nicht genetisch modifizierten Zellen um mindestens 50%, insbesondere um mindestens 70%, bevorzugt um mindestens 85% und besonders bevorzugt um mindestens 95%.
Die Verringerung der Menge an SSIII-, BEI- und/oder BEII-Proteinen, die eine verringerte Aktivität dieser Proteine in den betreffenden Pflanzenzellen zur Folge hat, kann beispielsweise bestimmt werden durch immunologische Methoden wie Western-Blot-Analyse, ELISA (Enzyme Linked Immuno Sorbent Assay) oder RIA (Radio Immune Assay). Eine Verringerung bedeutet dabei vorzugsweise eine Verringerung der Menge an SSIII-, BEI- und/oder BEII-Protein im Vergleich zu entsprechenden nicht genetisch modifizierten Zellen um mindestens 50%, insbesondere um mindestens 70%, bevorzugt um mindestens 85% und besonders bevorzugt um mindestens 95%.

Unter SSIII-Protein ist im Zusammenhang mit der vorliegenden Erfindung eine Klasse von löslichen Stärkesynthasen (ADPGlukose 1,4-alpha-D-glucan 4-alpha-Dglucosyltransferase; EC 2.4.1.21) zu verstehen. Lösliche Stärkesynthasen katalysieren eine Glycosylierungsreaktion, bei der Glukosereste des Substrates ADP-Glukose unter Bildung einer alpha 1,4-Verknüpfung auf alpha-1,4-verknüpfte Glukanketten übertragen werden. (ADP-Glukose + {(1,4)-alpha-D-glucosyl}(N) <=> ADP + {(1,4)-alpha-Dglucosyl}(N+1)).

Beschrieben sind SSIII Proteine zum Beispiel bei Marshall et al. (The Plant Cell 8; (1996); 1121-1135), Li et al. (2000, Plant Physiology 123, 613-624), Abel et al. (The Plant Journal 10(6); (1996); 981-991) und in WO 0066745. SSIII Proteine weisen häufig in ihrem Aufbau eine Abfolge von Domänen auf. SSIII Proteine haben am N-Terminus ein Signalpeptid für den Transport in Plastiden. In Richtung C-Terminus folgen eine N-terminale Region, eine SSIII spezifische Region und eine katalytische Domäne (Li et al., 2000, Plant Physiology 123, 613-624). Weitere Analysen, basierend auf Primärsequenzvergleichen (http://hits.isb-sib.ch/cgi-bin/PFSCAN), ergaben, dass das SSIII Protein aus Kartoffel eine sogenannte Kohlenhydrat Bindedomäne (CBM von Carbohydrate Binding Domain) aufweist. Diese Domäne (Pfam Motiv cbm 25) umfasst die Aminosären 377 bis 437 der in Seq ID Nr. 2 dargestellten Sequenz des SSIII-Proteins aus Kartoffel. Unter einem SSIII Protein sollen daher im Zusammenhang mit der vorliegenden Erfindung Stärkesynthasen verstanden werden, die zu der in Seq ID Nr. 3 dargestellten Sequenz eine Identität von mindestens 50% aufweisen, bevorzugt von mindestens 60%, besonders bevorzugt von mindestens 70%, weiter bevorzugt von mindestens 80%, und insbesondere von mindestens 90%.

Unter dem Begriff Homologie bzw. Identität soll die Anzahl der übereinstimmenden Aminosäuren (Identität) mit anderen Proteinen, ausgedrückt in Prozent verstanden werden. Bevorzugt wird die Identität durch Vergleiche der Seq. ID Nr. 3 zu anderen Proteinen mit Hilfe von Computerprogrammen ermittelt. Weisen Sequenzen, die miteinander verglichen werden, unterschiedliche Längen auf, ist die Identität so zu ermitteln, dass die Anzahl an Aminosäuren, welche die kürzere Sequenz mit der längeren Sequenz gemeinsam hat, den prozentualen Anteil der Identität bestimmt. Die Identität kann standardmäßig mittels bekannten und der Öffentlichkeit zur Verfügung stehenden Computerprogrammen wie z.B. ClustalW (Thompson et al., Nucleic Acids Research 22 (1994), 4673-4680) ermittelt werden. ClustalW wird öffentich zur Verfügung gestellt von Julie Thompson (Thompson@EMBL-Heidelberg.DE) und Toby Gibson (Gibson@EMBL-Heidelberg.DE), European Molecular Biology Laboratory, Meyerhofstrasse 1, D 69117 Heidelberg, Germany. ClustalW kann ebenfalls von verschiedenen Internetseiten, u.a. beim IGBMC (Institut de Génétique et de Biologie Moléculaire et Cellulaire, B.P.163, 67404 Illkirch Cedex, France; ftp://ftp-igbmc.ustrasbg.fr/pub/) und beim EBI (ftp://ftp.ebi.ac.uk/pub/software/) sowie bei allen gespiegelten Internetseiten des EBI (European Bioinformatics Institute, Wellcome Trust Genome Campus, Hinxton, Cambridge CB10 1SD, UK), heruntergeladen werden. Wenn das ClustalW Computerprogramm der Version 1.8 benutzt wird, um die Identität zwischen z.B. dem Referenzprotein der vorliegenden Amneldung und anderen Proteinen zu bestimmen, sind folgende Parameter einzustellen: KTUPLE=1, TOPDIAG=5, WINDOW=5, PAIRGAP=3, GAPOPEN=10, GAPEXTEND=0.05, GAPDIST=8, MAXDIV=40, MATRIX=GONNET, ENDGAPS(OFF), NOPGAP, NOHGAP. Eine Möglichkeit zum Auffinden von ähnlichen Sequenzen, ist die Durchführung von Sequenzdatenbankrecherchen. Hierbei wird eine oder werden mehrere Sequenzen als sogenannte Abfrage (= query) vorgegeben. Diese Abfragesequenz wird dann mittels statistischen Computerprogrammen mit Sequenzen, die in den ausgewählten Datenbanken enthalten sind, verglichen. Solche Datenbankabfragen (blast searches) sind dem Fachmann bekannt und können bei verschiedenen Anbietern durchgeführt werden. Wird eine solche Datenbankabfrage z.B. beim NCBI (National Center for Biotechnology Information, http://www.ncbi.nlm.nih.gov/) durchgeführt, so sollen die Standardeinstellungen, die für die jeweilige Vergleichsanfrage vorgegeben sind, benutzt werden. Für Proteinsequenzvergleiche (blastp) sind dieses folgende Einstellungen: Limit entrez = nicht aktiviert; Filter = low compexity aktiviert; Expect value = 10; word size = 3; Matrix = BLOSUM62; Gap costs: Existence = 11, Extension = 1. Als Ergebnis einer solchen Abfrage werden neben anderen Parametern auch der Anteil an Identität zwischen der Abfragesequenz und den in den Datenbanken aufgefundenen ähnlichen Sequenzen dargestellt.
Unter einem SSIII Protein sollen daher im Zusammenhang mit der vorliegenden Erfindung Stärkesynthasen verstanden werden, die bei der Verwendung mindestens einer der vorstehend beschriebenen Methoden zur Identitätsbestimmung zu der in Seq ID Nr. 3 dargestellten Sequenz eine Identität von mindestens 50% aufweisen, bevorzugt von mindestens 60%, besonders bevorzugt von mindestens 70%, weiter bevorzugt von mindestens 80%, und insbesondere von mindestens 90%.

Unter dem Begriff SSIII-Gen soll im Rahmen der vorliegenden Erfindung ein Nukleinsäuremolekül (DNA, cDNA, RNS) verstanden werden, welches ein SSIII-Protein, vorzugsweise aus Kartoffel, codiert. Nukleinsäuremoleküle codierend für ein SSIII-Protein sind für verschiedene Pflanzenspezies wie z.B. Kartoffel (Abel et al., The Plant Journal 10(6); (1996); 981-991), Weizen (WO 00/66745, Li et al., 2000, Plant Physiology 123, 613-624; Genbank Acc. No AF258608; Genbank Acc. No AF258609), Mais (Gao et al., 1998, Plant Cell 10 (3), 399-412; Genbank Acc. No AF023159), *Vignia* (Genbank Acc. No AJ225088), Reis (Genbank Acc. No AY100469; Genbank Acc. No AF43291) und *Arabidopsis* (Genbank Acc. No AC007296) beschrieben.

Im Rahmen der vorliegenden Erfindung wird unter dem Begriff "Branching Enzyme" oder "BE-Protein" (α-1,4-Glucan: α-1,4-Glucan 6-Glycosyltransferase, E.C. 2.4.1.18) ein Protein verstanden, das eine Transglycosylierungsreaktion katalysiert, in der α-1,4-Verknüpfungen eines α-1,4-Glucandonors hydrolysiert und die dabei freigesetzten α-1,4-Glucanketten auf eine α-1,4-Glucanakzeptorkette transferiert und dabei in α-1,6-Verknüpfungen überführt werden.

Unter dem Begriff "BEI-Protein" soll im Zusammenhang mit der vorliegenden Erfindung ein Verzweigungsenzym (branching enzyme = BE) der Isoform I verstanden werden. Vorzugsweise stammt das BEI-Protein aus Kartoffelpflanzen.
Die Bezeichnung der Isoformen lehnt dabei an der von Smith-White und Preiss vorgeschlagenen Nomenklatur an (Smith-White und Preiss, Plant Mol Biol. Rep. 12, (1994), 67-71, Larsson et al., Plant Mol Biol. 37, (1998), 505-511). Diese Nomenklatur geht davon aus, dass alle Enzyme, die zum BEI-Protein aus Mais (GenBank Acc. No. D11081; Baba et al., Biochem. Biophys. Res. Commun. 181 (1), (1991), 87-94; Kim et al. Gene 216, (1998), 233-243) eine höhere Homologie (Identität) auf Aminosäureebene aufweisen als zum BEII-Protein aus Mais (Genbank Acc. No AF072725 , U65948), als Branching Enzyme der Isoform I oder kurz als BEI-Proteine bezeichnet werden.

Unter dem Begriff "BEII-Protein" soll im Zusammenhang mit der vorliegenden Erfindung ein Verzweigungsenzym (branching enzyme = BE) der Isoform II verstanden werden. Vorzugsweise stammt dieses aus Kartoffelpflanzen. Im Zusammenhang mit der vorliegenden Erfindung sollen alle Enzyme, die auf Aminosäureebene zum BEII-Protein aus Mais (Genbank Acc. No AF072725, U65948) eine höhere Homologie (Identität) aufweisen als zum BEI-Protein aus Mais (Genbank Acc. No. D 11081, AF 072724 ), als BEII-Protein bezeichnet werden.

Unter dem Begriff "BEI-Gen" soll im Zusammenhang mit der vorliegenden Erfindung ein Nukleinsäuremolekül (cDNA, DNA) verstanden werden, das ein "BEI-Protein", vorzugsweise ein BEI-Protein aus Kartoffelpflanzen, codiert. Derartige Nukleinsäuremoleküle sind für zahlreiche Pflanzen beschrieben worden, beispielsweise für Mais (Genbank Acc. No. D 11081, AF 072724), Reis (Genbank Acc. No. D11082), Erbse (Genbank Acc. No. X80010) und Kartoffel. Verschiedene Formen des BEI-Gens bzw. des BEI-Proteins aus Kartoffel wurden beispielsweise beschrieben bei Khoshnoodi et al., Eur. J. Biochem. 242 (1), 148-155 (1996), Genbank Acc. No. Y 08786 und bei Kossmann et al., Mol. Gen. Genet. 230, (1991), 39-44). In Kartoffelpflanzen wird das BEI-Gen hauptsächlich in den Knollen und kaum in den Blättern exprimiert (Larsson et al., Plant Mol. Biol. 37, (1998), 505-511).

Unter dem Begriff "BEII-Gen" soll im Zusammenhang mit der vorliegenden Erfindung ein Nukleinsäuremolekül (z.B. cDNA, DNA) verstanden werden, das ein "BEII-Protein" codiert, vorzugsweise ein BEII-Protein aus Kartoffelpflanzen. Derartige Nukleinsäuremoleküle sind für zahlreiche Pflanzen beschrieben worden, beispielsweise für Kartoffel (GenBank Acc. No. AJ000004, AJ011888, AJ011889, AJ011885, AJ011890, EMBL GenBank A58164), Mais (AF 072725, U65948), Gerste (AF064561), Reis (D16201) und Weizen (AF 286319). In Kartoffelpflanzen wird das BEII-Gen hauptsächlich in den Blättern und kaum in den Knollen exprimiert (Larsson et al., Plant Mol. Biol. 37, (1998), 505-511).

Der Begriff "transgen" bedeutet in diesem Zusammenhang, dass die erfindungsgemäßen Pflanzenzellen aufgrund der Einführung eines fremden Nukleinsäuremoleküls oder mehrerer fremder Nukleinsäuremolekülein die Zelle in ihrer genetischen Information von entsprechenden nicht genetisch modifizierten Pflanzenzellen abweichen.

In einer weiteren Ausführungsform der vorliegenden Erfindung besteht die genetische Modifikation der erfindungsgemäßen transgenen Pflanzenzelle in der Einführung eines oder mehrerer fremder Nukleinsäuremoleküle, deren Vorhandensein und/oder Expression zur Verringerung der Aktivität von SSIII-und BEI- und BEII-Proteinen führt im Vergleich zu entsprechenden nicht genetisch modifizierten Pflanzenzellen von Wildtyppflanzen. Im speziellen versteht man unter dem Begriff der genetischen Modifikation das Einbringen von homologen und/oder heterologen und/oder mutagenisierten fremden Nukleinsaüremolekülen in eine Pflanzenzelle, wobei besagtes Einbringen dieser Moleküle zur Reduktion der Aktivität eines SSIII-Proteins und/oder eines BEI-Proteins und/oder BEII Proteins führt.

Unter dem Begriff "fremdes Nukleinsäuremolekül" bzw. "fremder Nukleinsäuremoleküle" versteht man im Zusammenhang mit der vorliegenden Erfindung ein solches Molekül, das entweder natürlicherweise in entsprechenden Pflanzenzellen nicht vorkommt, oder das in der konkreten räumlichen Anordnung nicht natürlicherweise in den Pflanzenzellen vorkommt oder das an einem Ort im Genom der Pflanzenzelle lokalisiert ist, an dem es natürlicherweise nicht vorkommt. Bevorzugt ist das fremde Nukleinsäuremolekül ein rekombinantes Molekül, das aus verschiedenen Elementen besteht, deren Kombination oder spezifische räumliche Anordnung natürlicherweise in pflanzlichen Zellen nicht auftritt.
Bei dem bzw. den zur genetischen Modifikation verwendeten fremden Nukleinsäuremolekül(en) kann es sich um ein zusammengefügtes oder mehrere getrennte Nukleinsäurekonstrukte handeln, insbesondere sogenannte Einfach-, Zweifach- und Dreifachkonstrukte. So kann das fremde Nukleinsäuremolekül beispielsweise ein sogenanntes "Dreifachkonstrukt" sein, worunter man einen einzigen Vektor zur Pflanzentransformation versteht, der sowohl die genetische Information zur Inhibierung der Expression eines oder mehrerer endogener SSIII-Gene als auch zur Inhibierung der Expression eines oder mehrerer BEI- und eines oder mehrerer BEII-Gene enthält oder dessen Vorhandensein bzw. dessen Expression zur Verringerung der Aktivität eines oder mehrerer SSIII-, BEI- und BEII-Proteine führt.

In einer weiteren Ausführungsform kann das fremde Nukleinsäuremolekül ein sogenanntes "Doppelkonstrukt" sein, worunter man einen Vektor zur Pflanzentransformation versteht, der die genetische Information zur Inhibierung der Expression von zwei der drei Zielgene (SSIII-, BEI-, BEII-Gen) enthält oder dessen Vorhandensein bzw. dessen Expression zur Verringerung der Aktivität von zwei der drei Zielproteine (SSIII-, BEI-, BEII-Proteine) führt. Die Inhibierung der Expression des dritten Zielgens und/oder die Verringerung der Aktivität des dritten Zielproteins erfolgt in dieser Ausführungsform der Erfindung mit Hilfe eines gesonderten fremden Nukleinsäuremoleküls, das die entsprechende genetische Information zur Inhibierung dieses dritten Zielgens enthält.

In einer weiteren Ausführungsform der Erfindung wird in das Genom der Pflanzenzelle nicht ein Dreifachkonstrukt, sondern es werden mehrere unterschiedliche fremde Nukleinsäuremoleküle eingeführt, wobei eines dieser fremden Nukleinsäuremoleküle beispielsweise ein DNA-Molekül ist, das z.B. ein Cosuppressions-Konstrukt darstellt, das eine Verringerung der Expression von einem oder mehreren endogenen SSIII-Genen bewirkt, und ein weiteres fremdes Nukleinsäuremolekül ein DNA-Molekül ist, das z.B. eine antisense-RNA codiert, die eine Verringerung der Expression von einem oder mehreren endogenen BEI- und/oder BEII-Genen bewirkt. Grundsätzlich ist bei der Konstruktion der fremden Nukleinsäuremoleküle aber auch die Verwendung jeder Kombination aus antisense-, cosuppressions-, Ribozym- und doppelsträngigen RNA Konstrukten oder in-vivo-Mutagenese geeignet, die zu einer gleichzeitigen Verringerung der Genexpression endogener Gene führt, die für ein oder mehrere SSIII-, BEI- und BEII-Proteine codieren, oder die zu einer gleichzeitigen Verringerung der Aktivität eines oder mehrerer SSIII-, BEI- und BEII-Proteine führt.
Die fremden Nukleinsäuremoleküle können hierbei zeitgleich ("Cotransformation") oder auch nacheinander, d.h. zeitlich aufeinanderfolgend ("Supertransformation") in das Genom der Pflanzenzelle eingeführt werden.

Die fremden Nukleinsäuremoleküle können auch in verschiedene individuelle Pflanzen einer Spezies eingeführt werden. Es können dabei Pflanzen erzeugt werden, bei welchen die Aktivität von einem Zielprotein oder zwei Zielproteinen (BEI, BEII, SSIII) reduziert ist. Durch anschließendes Kreuzen können dann Pflanzen erzeugt werden, bei welchen die Aktivität aller drei Zielproteine reduziert ist.

Weiterhin kann zur Einführung eines fremden Nukleinsäuremoleküls bzw. zur Erzeugung der erfindungsgemäßen Pflanzenzellen oder Pflanzen anstelle einer Wildtyp Pflanzenzelle bzw. -Pflanze eine Mutante, die sich dadurch auszeichnet, dass sie bereits eine verminderte Aktivität für eines oder mehrerer Zielproteine (BEI, BEII, SSIII) aufweist, herangezogen werden. Bei den Mutanten kann es sich sowohl um spontan auftretende Mutanten, als auch um solche handeln, die durch den gezielten Einsatz von Mutagenen erzeugt wurden. Möglichkeiten zur Erzeugung von solchen Mutanten sind weiter oben beschrieben worden.

Die erfindungsgemäßen Pflanzenzellen und deren Stärke können durch die Verwendung der sogenannten Insertionsmutagenese (Übersichtsartikel: Thorneycroft et al., 2001, Journal of experimental Botany 52 (361), 1593-1601) hergestellt werden. Unter Insertionsmutagenese ist insbesondere das Inserieren von Transposons oder sogenannter transfer DNA (T-DNA) in ein Gen codierend für ein BEI Protein und/oder BEII Protein und/oder ein SSIII Protein zu verstehen, wobei dadurch die Aktivität der besagten Proteine in der betreffenden Zelle reduziert wird.

Bei den Transposons kann es sich dabei sowohl um solche handeln, die in der Zelle natürlicherweise vorkommen (endogene Transposons), als auch um solche, die natürlicherweise nicht in besagter Zelle vorkommen, sondern mittels gentechnischer Methoden, wie z.B. Transformation der Zelle, in die Zelle eingeführt wurden (heterologe Transposons). Die Veränderung der Expression von Genen mittels Transposons ist dem Fachmann bekannt. Eine Übersicht über die Nutzung von endogenen und heterologen Transposons als Werkzeuge in der Pflanzenbiotechnologoie ist in Ramachandran und Sundaresan (2001, Plant Physiology and Biochemistry 39, 234-252) dargestellt. Die Möglichkeit, Mutanten zu identifizieren, bei welchen spezifische Gene durch Transposoninsertionsmutagenese inaktiviert wurden, ist in einer Übersicht von Maes et al. (1999, Trends in Plant Science 4 (3), 90-96) dargestellt. Die Erzeugung von Reismutanten mit Hilfe endogener Transposons ist von Hirochika (2001, Current Opinion in Plant Biology 4, 118-122) beschrieben. Die Identifizierung von Maisgenen, mit Hilfe endogener Retrotransposons wird z.B. von Hanley et al. (2000, The Plant Journal 22 (4), 557-566) dargestellt. Die Möglichkeit Mutanten mit Hilfe von Retrotransposons herzustellen und Methoden, Mutanten zu identifizieren, sind von Kumar und Hirochika (2001, Trends in Plant Science 6 (3), 127-134) beschrieben. Die Aktivität von heterologen Transposons in unterschiedlichen Spezies, ist sowohl für dikotelydone, als auch für monkotyledone Pflanzen beschrieben worden: z.B. für Reis (Greco et al., 2001, Plant Physiology 125, 1175-1177; Liu et al., 1999, Molecular and General Genetics 262, 413-420; Hiroyuki et al., 1999, The Plant Journal 19 (5), 605-613; Jeon und Gynheung, 2001, Plant Science 161, 211-219), Gerste (2000, Koprek et al., The Plant Journal 24 (2), 253-263) *Arabidopsis thaliana* (Aarts et al., 1993, Nature 363, 715-717, Schmidt und Willmitzer, 1989, Molecular and General Genetics 220, 17-24; Altmann et al., 1992, Theoretical and Applied Gentics 84, 371-383; Tissier et al., 1999, The Plant Cell 11, 1841-1852), Tomate (Belzile und Yoder, 1992, The Plant Journal 2 (2), 173-179) und Kartoffel (Frey et al., 1989, Molecular and General Genetics 217, 172-177; Knapp et al., 1988, Molecular and General Genetics 213, 285-290).

Grundsätzlich können die erfindungsgemäßen Pflanzenzellen und Pflanzen und die von ihnen produzierte Stärke sowohl mit Hilfe homologer, als auch heterologer Transposons hergestellt werden, wobei unter Verwendung von homologen Transposons auch solche zu verstehen sind, die bereits natürlicherweise im Pflanzengenom vorhanden sind.

Die T-DNA Insertionsmutagenese beruht darauf, dass bestimmte Abschnitte (T-DNA) von Ti-Plasmiden aus *Agrobacterium* in das Genom von pflanzlichen Zellen integrieren können. Der Ort der Integration in das pflanzliche Chromosom ist dabei nicht festgelegt, sondern kann an jeder beliebigen Stelle erfolgen. Integriert die T-DNA in einen Abschnitt des Chromosoms, der eine Genfuktion darstellt, so kann dieses zur Veränderung der Genexpression und damit auch zur Änderung der Aktivität eines durch das betreffende Gen codierte Protein führen. Insbesondere führt die Integration einer T-DNA in den codierenden Bereich eines Proteins häufig dazu, dass das entsprechende Protein von der betreffenden Zelle gar nicht mehr oder nicht mehr in aktiver Form synthetisiert werden kann. Die Verwendung von T-DNA Insertionen zur Erzeugung von Mutanten ist z.B. für *Arabidopsis thaliana* (Krysan et al., 1999, The Plant Cell 11, 2283-2290; Atipiroz-Leehan und Feldmann, 1997, Trends in genetics 13 (4), 152-156; Parinov und Sundaresan, 2000, Current Opinion in Biotechnology 11, 157-161) und Reis (Jeon und An, 2001, Plant Science 161, 211-219; Jeon et al., 2000, The Plant Journal 22 (6), 561-570) beschrieben. Methoden zur Identifizierung von Mutanten, die mit Hilfe der T-DNA Insertionsmutagenese erzeugt wurden, sind u.a. beschrieben von Young et al.,(2001, Plamt Physiology 125, 513-518), Parinov et al. (1999, The Plant cell 11, 2263-2270), Thorneycroft et al. (2001, Journal of Experimental Botany 52, 1593-1601), und McKinney et al. (1995, The Plant Journal 8 (4),613-622).

Die T-DNA Mutagenese ist grundsätzlich zur Erzeugung der erfindungsgemäßen Pflanzenzellen und der von diesen produzierten Stärke geeignet.

In einer weiteren Ausführungsform der vorliegende Erfindung führt das Vorhandensein und/oder die Expression eines oder mehrerer fremder Nukleinsäuremoleküle zur Inhibierung der Expression von endogenen Genen, die SSIII-Proteine, BEI-Proteine und BEII-Proteine codieren.

Die Herstellung erfindungsgemäßer Pflanzenzellen kann durch verschiedene, dem Fachmann bekannte Verfahren erzielt werden, z.B. durch solche, die zu einer Inhibierung der Expression endogener Gene führen, die ein SSIII-, BEI- bzw. BEII-Protein codieren. Hierzu zählen beispielsweise die Expression einer entsprechenden antisense-RNA, oder eines doppelsträngigen RNA Konstruktes, die Bereitstellung von Molekülen oder Vektoren, die einen Cosuppressionseffekt vermitteln, die Expression eines entsprechend konstruierten Ribozyms, das spezifisch Transkripte spaltet, die ein SSIII-, BEI- bzw. BEII-Protein codieren, oder die sogenannte "in-vivo-Mutagenese". Ferner kann die Verringerung der SSIII- und/oder der BEI- und/oder der BEII-Aktivität in den Pflanzenzellen auch durch die simultane Expression von sense und antisense RNA Molekülen des jeweiligen zu reprimierenden Zielgens, vorzugsweise des SSIIIund/oder des BEI- und/oder des BEII-Gens, hervorgerufen werden. Diese Methoden sind dem Fachmann geläufig.
Darüberhinaus ist bekannt, dass *in planta* die Bildung von doppelsträngigen RNA-Molekülen von Promotorsequenzen *in trans* zu einer Methylierung und einer transkriptionellen Inaktivierung homologer Kopien dieses Promotors führen kann (Mette et al., EMBO J. 19, (2000), 5194-5201).
Weitere Verfahren zur Verringerung der Aktivität von Proteinen werden weiter unten beschrieben.
Alle diese Verfahren basieren auf der Einführung eines fremden oder mehrerer fremder Nukleinsäuremoleküle in das Genom von Pflanzenzellen.

Zur Inhibierung der Genexpression mittels antisense- oder cosuppressions-Technologie kann beispielsweise ein DNA-Molekül verwendet werden, das die gesamte für ein SSIIIund/oder BEI- und/oder BEII-Protein codierende Sequenz einschließlich eventuell vorhandener flankierender Sequenzen umfaßt, als auch DNA-Moleküle, die nur Teile der codierenden Sequenz umfassen, wobei diese Teile lang genug sein müssen, um in den Zellen einen antisense-Effekt bzw. cosuppressions-Effekt zu bewirken. Geeignet sind im allgemeinen Sequenzen bis zu einer Mindestlänge von 15 bp, vorzugsweise einer Länge von 100-500 bp, für eine effiziente antisense- bzw. cosuppressions-Inhibition insbesondere Sequenzen mit einer Länge über 500 bp.

Für antisense- oder cosuppressions-Ansätze geeignet ist auch die Verwendung von DNA-Sequenzen, die einen hohen Grad an Homologie zu den endogen in der Pflanzenzelle vorkommenden Sequenzen haben, die SSIII-, BEI- bzw. BEII-Proteine codieren. Die minimale Homologie sollte größer als ca. 65 % sein. Die Verwendung von Sequenzen mit Homologien von mindestens 90%, insbesondere zwischen 95 und 100% ist zu bevorzugen.

Ferner ist zur Erzielung eines antisense- oder eines cosuppressions-Effektes auch die Verwendung von Introns, d.h. von nicht-codierenden Bereichen von Genen, die für SSIII-, BEI- und/oder BEII-Proteine codieren, denkbar.
Die Verwendung von Intron-Sequenzen zur Inhibierung der Genexpression von Genen, die für Proteine der Stärkebiosynthese codieren, wurde beschrieben in den internationalen Patentanmeldungen WO97/04112, WO97/04113, WO98/37213, W098/37214.
Dem Fachmann ist bekannt, wie er einen antisense- und einen cosuppressions- Effekt erzielen kann. Das Verfahren der cosuppressions-Inhibierung wurde beispielsweise beschrieben in Jorgensen (Trends Biotechnol. 8 (1990), 340-344), Niebel et al., (Curr. Top. Microbiol. Immunol. 197 (1995), 91-103), Flavell et al. (Curr. Top. Microbiol. Immunol. 197 (1995), 43-46), Palaqui und Vaucheret (Plant. Mol. Biol. 29 (1995), 149-159), Vaucheret et al., (Mol. Gen. Genet. 248 (1995), 311-317), de Borne et al. (Mol. Gen. Genet. 243 (1994), 613-621).

Auch die Expression von Ribozymen zur Verringerung der Aktivität von bestimmten Enzymen in Zellen ist dem Fachmann bekannt und ist beispielsweise beschrieben in EP-B1 0321201. Die Expression von Ribozymen in pflanzlichen Zellen wurde z.B. beschrieben in Feyter et al. (Mol. Gen. Genet. 250, (1996), 329-338).

Ferner kann die Verringerung der SSIII- und/oder der BEI- und/oder der BEII-Aktivität in den Pflanzenzellen auch durch die sogenannte "in vivo-Mutagenese" erreicht werden, bei der durch Transformation von Zellen ein hybrides RNA-DNA-Oligonucleotid ("Chimeroplast") in Zellen eingeführt wird (Kipp, P.B. et al., Poster Session beim " 5^{th} International Congress of Plant Molecular Biology, 21.-27. September 1997, Singapore; R. A. Dixon und C.J. Arntzen, Meeting report zu "Metabolic Engineering in Transgenic Plants", Keystone Symposia, Copper Mountain, CO, USA, TIBTECH 15, (1997), 441-447; internationale Patentanmeldung WO 9515972; Kren et al., Hepatology 25, (1997), 1462-1468; Cole-Strauss et al., Science 273, (1996), 1386-1389; Beetham et al., 1999, PNAS 96, 8774-8778).
Ein Teil der DNA-Komponente des RNA-DNA-Oligonucleotids ist homolog zu einer Nukleinsäuresequenz eines endogenen SSIII-, BEI- und/oder BEII-Gens, weist jedoch im Vergleich zur Nukleinsäuresequenz eines endogenen SSIII-, BEI- und/oder BEII-Gens eine Mutation auf oder enthält eine heterologe Region, die von den homologen Regionen umschlossen ist.
Durch Basenpaarung der homologen Regionen des RNA-DNA-Oligonucleotids und des endogenen Nukleinsäuremoleküls, gefolgt von homologer Rekombination, kann die in der DNA-Komponente des RNA-DNA-Oligonucleotids enthaltene Mutation oder heterologe Region in das Genom einer Pflanzenzelle übertragen werden. Dies führt zu einer Verringerung der Aktivität eines oder mehrerer SSIII-, BEI- und/oder BEII-Proteine.
Ferner kann die Verringerung der SSIII- und/oder der BEI- und/oder der BEII-Aktivität in den Pflanzenzellen auch durch die simultane Expression von sense und antisense RNA Molekülen des jeweiligen zu reprimierenden Zielgens, vorzugsweise des SSIIIund/oder des BEI- und/oder des BEII-Gens, hervorgerufen werden.
Dies kann beispielsweise durch die Verwendung von chimären Konstrukten erreicht werden, die "inverted repeats" des jeweiligen Zielgens oder Teilen des Zielgens enthalten. Hierbei codieren die chimären Konstrukte für sense und antisense RNA Moleküle des jeweiligen Zielgens. Sense und antisense RNA werden *in planta* gleichzeitig als ein RNA-Molekül synthetisiert, wobei sense und antisense RNA durch einen Spacer voneinander getrennt sein und ein doppelsträngiges RNA-Molekül bilden können.
Es konnte gezeigt werden, dass die Einführung von inverted-repeat-DNA-Konstrukten in das Genom von Pflanzen eine sehr effiziente Methode ist, um die zu den inverted-repeat-DNA-Konstrukten korrespondierenden Gene zu reprimieren (Waterhouse et al., Proc. Natl. Acad. Sci. USA 95, (1998), 13959-13964; Wang and Waterhouse, Plant Mol. Biol. 43, (2000), 67-82;Singh et al., Biochemical Society Transactions Vol. 28 part 6 (2000), 925- 927; Liu et al., Biochemical Society Transactions Vol. 28 part 6 (2000), 927-929); Smith et al., (Nature 407, (2000), 319-320; internationale Patentanmeldung WO99/53050 A1). Sense und antisense Sequenzen des Zielgens bzw. der Zielgene können auch getrennt voneinander mittels gleicher oder unterschiedlicher Promotoren exprimiert werden (Nap, J-P et al, 6^{th} International Congress of Plant Molecular Biology, Quebec, 18.-24. Juni, 2000; Poster S7-27, Vortrag Session S7).

Die Verringerung der SSIII- und/oder der BEI- und/oder der BEII-Aktivität in den Pflanzenzellen kann somit auch durch die Erzeugung doppelsträngiger RNA-Moleküle von SSIII- und/oder BEI- und/oder BEII-Genen erreicht werden. Vorzugsweise werden hierzu "inverted repeats" von DNA-Molekülen von SSIII- und/oder BEI- und/oder BEII-Genen oder -cDNAs in das Genom von Pflanzen eingeführt, wobei die zu transkribierenden DNA-Moleküle (SSIII-, BEI- oder BEII-Gen oder -cDNA oder Fragmente dieser Gene oder cDNAs) unter Kontrolle eines Promotors stehen, der die Expression besagter DNA-Moleküle steuert.

Darüberhinaus ist bekannt, dass die Bildung von doppelsträngigen RNA-Molekülen von Promotor-DNA-Molekülen in Pflanzen *in trans* zu einer Methylierung und einer transkriptionellen Inaktivierung homologer Kopien dieser Promotoren führen kann, die im folgenden als Zielpromotoren bezeichnet werden sollen (Mette et al., EMBO J. 19, (2000), 5194-5201).
Über die Inaktivierung des Zielpromotors ist es somit möglich, die Genexpression eines bestimmten Zielgens (z.B. SSIII, BEI oder BEII-Gen), das natürlicherweise unter der Kontrolle dieses Zielpromotors steht, zu verringern.
D.h., die DNA-Moleküle, die die Zielpromotoren der zu reprimierenden Gene (Zielgene) umfassen, werden in diesem Fall, im Gegensatz zur ursprünglichen Funktion von Promotoren in Pflanzen, nicht als Steuerelemente zur Expression von Genen oder cDNAs, sondern selbst als transkribierbare DNA-Moleküle verwendet.
Zur Erzeugung der doppelsträngigen Zielpromotor-RNA-Moleküle *in planta*, die dort als RNA-Haarnadel-Moleküle (RNA hairpin) vorliegen können, werden vorzugsweise Konstrukte verwendet, die "inverted repeats" der Zielpromotor-DNA-Moleküle enthalten, wobei die Zielpromotor-DNA-Moleküle unter Kontrolle eines Promotors stehen, der die Genexpression besagter Zielpromotor-DNA-Moleküle steuert. Anschließend werden diese Konstrukte in das Genom von Pflanzen eingeführt. Die Expression der "inverted repeats" besagter Zielpromotor-DNA-Moleküle führt *in planta* zur Bildung doppelsträngiger Zielpromotor-RNA-Moleküle (Mette et al., EMBO J. 19, (2000), 5194-5201). Hierdurch kann der Zielpromotor inaktiviert werden.
Die Verringerung der SSIII- und/oder der BEI- und/oder der BEII-Aktivität in den Pflanzenzellen kann somit auch durch die Erzeugung doppelsträngiger RNA-Moleküle von Promotorsequenzen von SSIII- und/oder BEI- und/oder BEII-Genen erreicht werden. Vorzugsweise werden hierzu "inverted repeats" von Promotor-DNA-Molekülen von SSIII- und/oder BEI- und/oder BEII-Promotoren in das Genom von Pflanzen eingeführt, wobei die zu transkribierenden Zielpromotor-DNA-Moleküle (SSIII-, BEIund/oder BEII-Promotor) unter Kontrolle eines Promotors stehen, der die Expression besagter Zielpromotor-DNA-Moleküle steuert.

Ferner ist dem Fachmann bekannt, dass er die Aktivität eines oder mehrerer SSIII-, BEI- und/oder BEII-Proteine durch die Expression von nicht-funktionellen Derivaten insbesondere trans-dominanten Mutanten solcher Proteine und/oder durch die Expression von Antagonisten/Inhibitoren solcher Proteine erreichen kann.
Antagonisten/Inhibitoren solcher Proteine umfassen beispielsweise Antikörper, Antikörperfragmente oder Moleküle mit ähnlichen Bindungseigenschaften. Beispielsweise wurde ein cytoplasmatischer scFv Antikörper eingesetzt, um die Aktivität des Phytochrom A Proteins in gentechnisch veränderten Tabakpflanzen zu modulieren (Owen, Bio/Technology 10 (1992), 790-4; Review: Franken, E, Teuschel, U. und Hain, R., Current Opinion in Biotechnology 8, (1997), 411-416; Whitelam, Trends Plant Sci. 1 (1996), 268-272).

Sinnvolle Promotoren für die Expression von Nukleinsäuren, die die Aktivität eines Zielgens verringern, sind z.B. der Promotor der 35S RNA des Cauliflower Mosaic Virus und der Ubiquitin-Promotor aus Mais für eine konstitutive Expression, der Patatingen-Promotor B33 (Rocha-Sosa et al., EMBO J. 8 (1989), 23-29), der MCPI-Promotor des Metallocarboypeptidase-Inhibitor-Gens aus Kartoffel (ungarische Patentanmeldung HU9801674) oder der GBSSI-Promotor aus Kartoffel (internationale Patentanmeldung WO 92/11376) für eine knollenspezifische Expression in Kartoffeln oder ein Promotor, der eine Expression lediglich in photosynthetisch aktiven Geweben sicherstellt, z.B. der ST-LS1-Promotor (Stockhaus et al., Proc. Natl. Acad. Sci. USA 84 (1987), 7943-7947; Stockhaus et al., EMBO J. 8 (1989), 2445-2451), der Ca/b-Promotor (s. beispielsweise US 5656496, US 5639952, Bansal et al., Proc. Natl. Acad. Sci.USA 89, (1992), 3654-3658) und der Rubisco SSU-Promotor (s. beispielsweise US 5034322, US 4962028) oder für eine endosperm-spezifische Expression der Glutelin-Promotor (Leisy et al., Plant Mol. Biol. 14, (1990), 41-50; Zheng et al., Plant J. 4, (1993), 357-366; Yoshihara et al., FEBS Lett. 383, (1996), 213-218), der Shrunken-1 Promotor (Werr et al., EMBO J. 4, (1985), 1373-1380), der HMG-Promotor aus Weizen, der USP-Promotor, der Phaseolinpromotor oder Promotoren von Zein-Genen aus Mais (Pedersen et al., Cell 29, (1982), 1015-1026; Quatroccio et al., Plant Mol. Biol. 15 (1990), 81-93).
Die Expression des fremden Nukleinsäuremoleküls (der fremden Nukleinsäuremoleküle) ist insbesondere in solchen Organen der Pflanze von Vorteil, die Stärke speichern. Solche Organe sind z.B. die Knolle der Kartoffelpflanze oder die Körner bzw. das Endosperm von Mais-, Weizen- oder Reispflanzen. Bevorzugt werden daher Promotoren verwendet, die die Expression in diesen Organen vermitteln.
Es können jedoch auch Promotoren verwendet werden, die nur zu einem durch äußere Einflüsse determinierten Zeitpunkt aktiviert werden (siehe beispielsweise WO 93/07279). Von besonderem Interesse können hierbei Promotoren von heat-shock Proteinen sein, die eine einfache Induktion erlauben. Ferner können samenspezifische Promotoren, wie z.B. der USP-Promoter aus Vicia faba, der eine samenspezifische Expression in Vicia faba und anderen Pflanzen gewährleistet (Fiedler et al., Plant Mol. Biol. 22, (1993), 669-679; Bäumlein et al., Mol. Gen. Genet. 225, (1991), 459-467). Ferner können auch fruchtspezifische Promotoren eingesetzt werden, wie z.B. beschrieben in der W091/01373.
Weiterhin kann eine Terminationssequenz vorhanden sein, die der korrekten Beendigung der Transkription dient sowie der Addition eines Poly-A-Schwanzes an das Transkript, dem eine Funktion bei der Stabilisierung der Transkripte beigemessen wird. Derartige Elemente sind in der Literatur beschrieben (vgl. z.B. Gielen et al., EMBO J. 8 (1989), 23-29) und sind beliebig austauschbar.

Die erfindungsgemäßen transgenen Pflanzenzellen synthetisieren eine modifizierte Stärke, die in ihren physikalisch-chemischen Eigenschaften, insbesondere dem Amylosegehalt bzw. dem Amylose/Amylopektin-Verhältnis, dem Phosphatgehalt, dem Viskositätsverhalten, der Gelfestigkeit, der Stärkekorngröße und/oder der Stärkekornmorphologie im Vergleich zu in Wildtyppflanzen synthetisierter Stärke verändert ist, so dass diese für spezielle Verwendungszwecke besser geeignet ist.

Gegenstand der vorliegenden Erfindung ist daher auch eine erfindungsgemäße genetisch modifizierte Pflanzenzelle, insbesondere eine transgene Pflanzenzelle, die eine modifizierte Stärke synthetisiert.

Es wurde überraschenderweise gefunden, dass bei den erfindungsgemäßen Pflanzenzellen die Zusammensetzung der Stärke in der Weise verändert ist, dass sie einen Amylosegehalt von mindestens 30% und einen erhöhten Phosphatgehalt und in der RVA Analyse eine erhöhte Endviskosität im Vergleich zu Stärke aus Pflanzenzellen von entsprechenden Wildtyppflanzen aufweist, so dass diese Stärke für spezielle Verwendungszwecke besser geeignet ist.
Die erfindungsgemäßen Stärken weisen insbesondere den Vorteil auf, dass sie trotz des erhöhten Amylosegehaltes unter Standardbedingungen vollständig verkleistern. Dadurch wird die Verarbeitbarkeit der Stärke gegenüber anderen Stärken mit erhöhtem Amylosegehalt deutlich verbessert. Es ist daher zum Gelatinisieren der erfindungsgemäßen Stärke keine erhöhte Temperatur oder erhöhter Druck notwendig. Daher kann zum Aufschluß dieser Stärken auf den Einsatz spezieller Geräte wie z.B. Jetcooker, Extruder oder Autoklaven verzichtet werden. Ein weiterer Vorteil der erfindungsgemäßen Stärken liegt darin, dass sie bei Verarbeitungsprozessen mit Heißwalzen als Suspension auf diese aufgebracht werden können. Andere Stärken mit erhöhtem Amylosegehalt würden bei dieser Verarbeitung, wenn überhaupt, nur begrenzt verkleistern und daher auch nicht als Paste oder Film auf die entsprechenden Walzen aufgebracht werden können.
Besonders geeignet sind die erfindungsgemäßen Stärken für alle Anwendungen, bei welchen die Dickungsleistung, die Geliereigenschaften oder die Bindungseigenschaften zugesetzter Substanzen von Bedeutung sind. Daher eignet sich die erfindungsgemäße Stärke besonders zur Herstellung von Lebensmitteln wie z.B. Backwaren, Fertignahrungsmitteln (Instant Food), Pudding, Suppen, Konfekt, Schokolade, Eiskrem, Panaden für Fisch- oder Fleisch, gefrorene Dessets oder extrudierte Snacks. Weiterhin ist die erfindungsgemäße Stärke geeignet für die Herstellung von Klebstoffen, Anwendungen bei der Textilverarbeitung, als Zusatz zu Baustoffen, für Anwendungen im Bereich der Tierernährung, als Zusatzstoff für Kosmetika und in der Papierverarbeitung.
Insbesondere eignet sich die Stärke, welche aus erfindungsgemäßen Pflanzenzellen isoliert wurde zur Herstellung von Quellstärken.
Quellstärken sind physikalisch modifizierte Stärken, die vorwiegend durch naßthermischen Aufschluß hergestellt werden. Im Unterschied zu nativer Stärke bilden sie mit kaltem Wasser Dispersionen bzw. Pasten oder Gele, je nach eingesetzter Konzentration der Quellstärke und in Abhängigkeit von der zur Herstellung der Quellstärke verwendeten Stärkeart. Aufgrund dieser Eigenschaften ergeben sich für Quellstärken eine Reihe von Anwendungsmöglichkeiten in der Lebensmittelindustrie und außerdem in vielen technischen Bereichen. Die Verwendung von Quellstärke, die auch als kaltquellende Stärke bezeichnet wird, anstelle von nativer Stärke hat in verschiedenen Fällen den Vorteil, dass Produktionsverfahren vereinfacht und verkürzt werden können.
Für die Herstellung beispielsweise von Instant-Desserts und -Puddings werden Quellstärken benötigt, die nach dem Einrühren in kalte Flüssigkeit, wie z.B. Wasser oder Milch, innerhalb kurzer Zeit schnittfeste Gele bilden, wie beispielsweise im Falle eines Kochpuddings. Diese Anforderungen erfüllen die kommerziellen Quellstärken aus Weizen-, Kartoffel- oder Maisstärke nicht. Zur Erzielung der vorgenannten Eigenschaften sind bei den bisher kommerziell verfügbaren Quellstärken Zusätze zur Quellstärke wie Gelatine, Alginat, Carrageenan (Carrageen) und/oder anorganische Salze notwendig. Dieses Zusetzen von sogenannten Hilfsstoffen ist z.B. nach Herstellung von Quellstärken mit erfindungsgemäßen Stärken, isoliert aus erfindungsgemäßen Pflanzenzellen nicht notwendig.

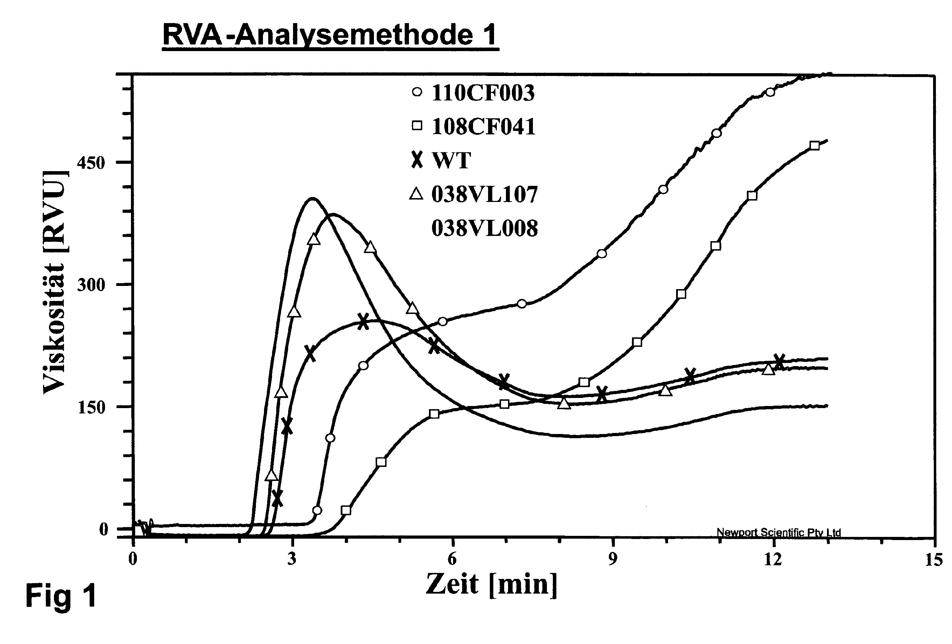

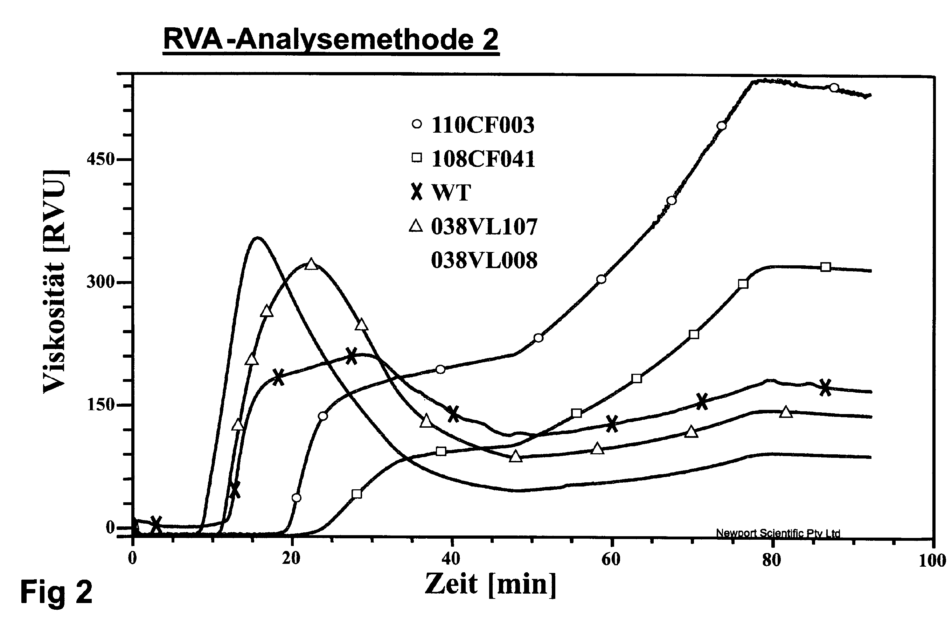

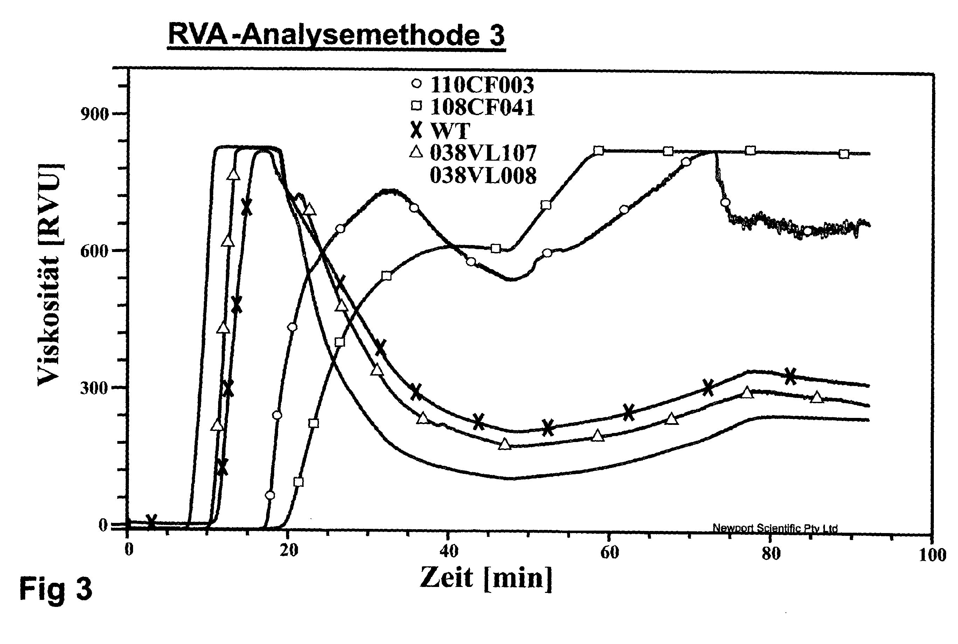

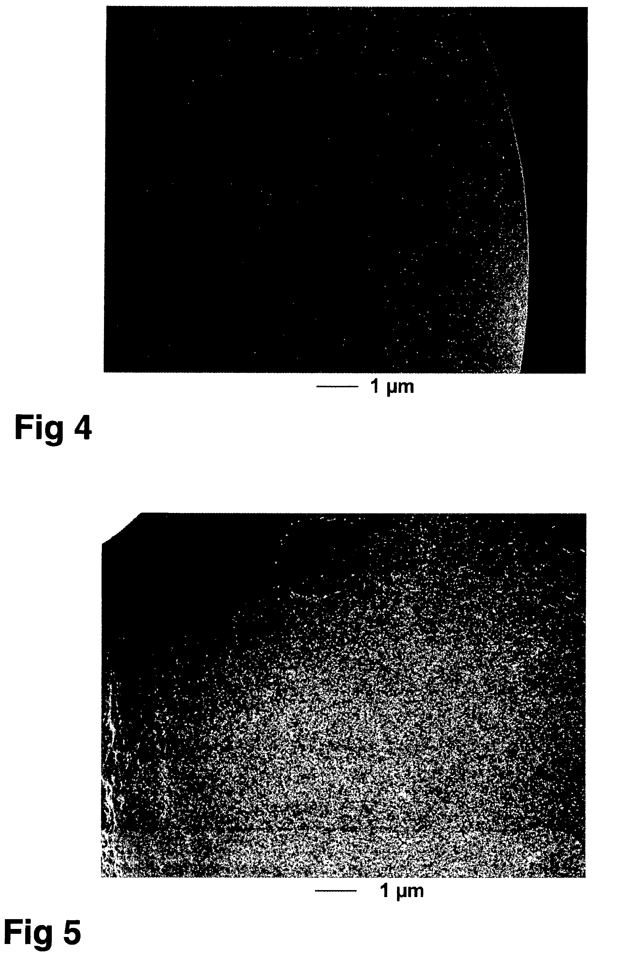

Gegenstand der vorliegenden Erfindung ist auch eine erfindungsgemäße Pflanzenzelle, die eine modifizierte Stärke mit veränderter Stärkekornmorphologie aufweist.
Unter dem Begriff Stärkekornmorphologie soll im Zusammenhang mit der vorliegenden Erfindung die Größe, und die Oberflächenstruktur von nativen Stärkekörnern verstanden werden. Stärke wird in den Speicherorganen wie z.B. Knollen, Wurzeln, Embryonen oder Endosperm von Pflanzen als kristalline Struktur in granulärer From gespeichert. Stärkekörner, bei welchen diese granulären Strukturen nach Isolierung der Stärke aus Pflanzenzellen erhalten bleiben, werden als native Stärke bezeichnet. Die mittlere Korngröße (bestimmt nach der weiter unten beschriebenen Methode) der erfindungsgemäßen nativen Stärke ist deutlich geringer, als diejenige von nativer Stärke, die aus Wildtyp-Pflanzen isoliert wurde. In der Aufnahme mit dem Rasterelektronenmikroskop (siehe Fig. 4 und 5) ist deutlich zu erkennen, dass erfindungsgemäße native Stärkekörner überraschenderweise eine rauhe Oberfläche mit vielen Poren aufweisen. Die Oberflächenstruktur von nativen Stärkekömern, isoliert aus Wildtyp-Pflanzen, zeigt hingegen eine überwiegend glatte Struktur, die keine Poren erkennen läßt.
Sowohl das Vorliegen von kleineren Körnern, als auch die rauhe, mit Poren versehene Oberfläche führen dazu, dass die Oberfläche von erfindungsgemäßen Stärkekörnern bei gleichem Volumen wesentlich größer ist, als diejenige von Stärkekörnern, isoliert aus Wildtyppflanzen. Dadurch eignet sich die erfindungsgemäße Stärke besonders gut für den Einsatz als Trägerstoff für z.B. Geschmackstoffe, pharmakologisch aktive Substanzen, präbiotische Substanzen, probiotische Mikroorganismen, Enzyme oder Farbstoffe. Auch zum Koagulieren von Substanzen und in der Papierherstellung sind diese Stärken besonders geeignet.
Eine weitere Anwendungsmöglichkeit für die erfindungsgemäßen Stärken findet sich auf dem Gebiet der Förderung von Rohstoffen unter Einsatz von Bohrern. So ist es z.B. bei der Förderung von Rohöl notwendig, Hilfstoffe und/oder Schmiermittel einzusetzen, die eine Überhitzung des Bohrers, bzw. des Bohrgestänges vermeiden. Durch ihre besonderen Gelatinisierungseigenschaften ist die erfindungsgemäße Stärke daher auch für die Anwendung auf diesem Gebiet besonders geeignet.

Gegenstand der vorliegenden Erfindung ist auch eine erfindungsgemäße Pflanzenzelle, die eine modifizierte Stärke mit einem Amylosegehalt von mindestens 30% enthält und im Vergleich zu Stärke aus entsprechenden nicht genetisch modifizierten Pflanzenzellen von Wildtyppflanzen einen erhöhten Phosphatgehalt und in der RVA Analyse erhöhte Endviskosität aufweist.

Der Amylosegehalt wird im Zusammenhang mit der vorliegenden Erfindung nach der weiter unten für Kartoffelstärke beschriebenen Methode von Hovenkamp-Hermelink et al. (Potato Research 31, (1988), 241-246) bestimmt. Diese Methode ist auch auf isolierte Stärken anderer Pflanzenspezies anwendbar. Verfahren zur Isolierung von Stärken sind dem Fachmann bekannt.

Der Begriff "Phosphatgehalt" der Stärke bezeichnet im Sinne der vorliegenden Erfindung den Gehalt an kovalent in Form von Stärkephosphatmonoestern gebundenem Phosphat.
Der Begriff "erhöhter Phosphatgehalt" bedeutet im Zusammenhang mit der vorliegenden Erfindung, dass der Gesamtphosphatgehalt an kovalent gebundenem Phosphat und/oder der Phosphatgehalt in C-6-Position der in den erfindungsgemäßen Pflanzenzellen synthetisierten Stärke erhöht ist, vorzugsweise um mindestens 270%, weiter bevorzugt um mindestens 300%, besonders bevorzugt um mindestens 350% erhöht ist im Vergleich zu Stärke aus Pflanzenzellen von entsprechenden Wildtyppflanzen.

Unter dem Begriff "Phosphatgehalt in C6-Position" versteht man im Sinne der vorliegenden Erfindung den Gehalt an Phosphatgruppen, die an Kohlenstoffatomposition "6" der Glukosemonomere der Stärke gebunden sind. Grundsätzlich können in der Stärke *in vivo* die Positionen C2, C3 und C6 der Glukoseeinheiten phosphoryliert sein. Im Zusammenhang mit der vorliegenden Erfindung kann die Bestimmung des Phosphatgehaltes in C6-Position (= C6-P-Gehalt) über eine Glukose-6-phosphat-Bestimmung mittels eines optisch-enzymatischen Tests (Nielsen et al., Plant Physiol. 105, (1994), 111-117) erfolgen (siehe unten).

Unter dem Begriff "Gesamtphosphatgehalt" der Stärke versteht man im Zusammenhang mit der vorliegenden Erfindung den Gehalt an kovalent in Form von Stärkephosphatmonoestern gebundenem Phosphat in C2-, C3- und C6-Position der Glukoseeinheiten. Der Gehalt an phosphorylierten Nicht-Glukanen, wie z.B. Phospholipiden, ist erfindungsgemäß von dem Begriff "Gesamtphosphatgehalt" nicht umfaßt. Phosphorylierte Nicht-Glukane müssen daher vor Bestimmung des Gesamtphosphatgehaltes quantitativ abgetrennt werden. Verfahren zur Trennung der phosphorylierten Nicht-Glukane (z.B. Phospholipide) von der Stärke sind dem Fachmann bekannt. Methoden zur Bestimmung des Gesamtphosphatgehaltes sind dem Fachmann bekannt und unten beschrieben.

In einer weiteren Ausführungsform der Erfindung synthetisieren die erfindungsgemäßen Pflanzenzellen eine Stärke, die in C6-Position der Glukosemonomere der Stärke einen Phosphatgehalt von 40 - 120 nmol, insbesondere von 60 - 110 nmol, bevorzugt von 80 - 100 C6-P pro mg Stärke aufweisen.

Ein Protokoll zur Durchführung der RVA Analyse ist weiter unten beschrieben. Insbesondere ist darauf hinzuweisen, dass in der RVA Analyse von Kartoffelstärken häufig eine 8%ige Stärkesuspension (w/w) eingesetzt wird. In den zum zum Gerät "RVAsuper3" beigefügten Unterlagen (Gebrauchsanweisung, Newport Scientific Pty Ltd., Investment Support Group, Warried NSW 2102, Australien) wird eine Suspension enthaltend ca. 10% Stärke zur Analyse von Kartoffelstärke empfohlen.
Überraschenderweise wurde im Falle der Stärke aus Kartoffelpflanzen, betreffend die vorliegende Erfindung, gefunden, dass die Verwendung einer 8%igen Stärkesuspension (2g Stärke in 25 ml Wasser) für die Analyse nicht möglich war, weil die Endviskosität Werte erreichte, die von dem Gerät nicht mehr erfasst werden konnten. Darum wurden für die RVA Analyse anstelle von 8%igen Stärkesuspensionen nur 6%ige Stärkesuspensionen ( 1,5g Stärke in 25 ml Wasser) eingesetzt. Im Zusammenhang mit der vorliegenden Erfindung soll daher unter erhöhter Endviskosität in der RVA Analyse eine Erhöhung um mindestens 150%, besonders um mindestens 200%, insbesondere um mindestens 250% gegenüber nicht genetisch modifizierten Wildtyp Pflanzen verstanden werden. Die Erhöhung der Endviskositäten ist dabei auf 6%ige Stärkesuspensionen zu beziehen.
Weiterhin soll im Zusammenhang mit der vorliegenden Erfindung eine Kartoffelstärke verstanden werden, die eine Endviskosität in der RVA Analyse mit 6%igem Stärkegehalt von mindestens 300 RVU, besonders von 400 RVU, insbesondere von 500 RVU aufweisen. Auf die Bestimmung der RVUs wird nachstehend noch eingegangen.
In einer weiteren bevorzugten Ausführungsform betrifft die vorliegende Erfindung erfindungsgemäße Pflanzenzellen, die eine modifizierte Stärke synthetisieren, die nach Verkleisterung in Wasser ein Gel bildet, das im Vergleich zu einem Gel aus Stärke von entsprechenden nicht genetisch modifizierten Wildtyp-Pflanzenzellen eine erhöhte Gelfestigkeit aufweist.

Unter dem Begriff "erhöhte Gelfestigkeit" versteht man im Sinne der vorliegenden Erfindung eine Erhöhung der Gelfestigkeit, vorzugsweise um mindestens 300%, insbesondere um mindestens 500%, weiter bevorzugt um mindestens 700% und besonders bevorzugt um mindestens 800%, maximal um höchstens 2000% oder um höchstens 1500% im Vergleich zur Gelfestigkeit von Stärke aus entsprechenden nicht genetisch modifizierten Wildtyp-Pflanzenzellen.
Die Bestimmung der Gelfestigkeit soll im Zusammenhang mit der vorliegenden Erfindung mit Hilfe eines Texture Analyzers unter den unten beschriebenen Bedingungen erfolgen.
Um Stärkegele herzustellen muß die kristalline Struktur von nativer Stärke zunächst durch Erhitzen in wässriger Suspension unter ständigem Rühren zerstört werden. Dieses wurde mit Hilfe eines Rapid Visco Analyser (Newport Scientific Pty Ltd., Investmet Support Group, Warriewod NSW 2102, Australien) durchgeführt. Wie weiter oben bereits ausgeführt, wurde dabei für Stärke aus Kartoffelpflanzen nur eine 6%ige Stärkesuspension anstatt einer 8%igen eingesetzt, weil die Endviskositäten der 8%igen Suspensionen von dem Gerät nicht mehr erfasst werden konnten. Zur Bestimmung der Gelfestigkeit werden die im Rapid Visco Analyser verkleisterten Stärkesuspensionen für eine gewisse Zeit gelagert und dann einer Analyse mit einem Texture Analyser unterzogen. Folglich sind auch zur Bestimmung der Gelfestigkeit 6%ige anstelle von 8%igen verkleisterten Stärkesuspensionen eingesetzt worden.

In einer weiteren Ausführungsform der vorliegende Erfindung zeichnet sich die in den erfindungsgemäßen Pflanzenzellen synthetisierte modifizierte Stärke nicht nur durch einen im Vergleich zu Stärke von entsprechenden Wildtyp-Pflanzen erhöhten Amylosegehalt und einen erhöhten Phosphatgehalt und eine erhöhte Endviskosität in der RVA Analyse aus, sondern auch durch eine veränderte Seitenkettenverteilung.

In einer weiteren Ausführungsform betrifft die vorliegende Erfindung somit erfindungsgemäße Pflanzenzellen, die eine modifizierte Stärke synthetisieren, wobei die modifizierte Stärke dadurch gekennzeichnet ist, dass sie eine veränderte Seitenkettenverteilung aufweist.

Unter dem Begriff "veränderte Seitenkettenverteilung" soll in einer Ausführungsform der vorliegenden Erfindung eine Verringerung des Anteils an kurzen Seitenketten mit einem DP (= Degree of Polymerisation) von 6 bis 11 um mindestens 10%, bevorzugt um mindestens 15%, insbesondere um mindestens 30% und besonders bevorzugt um mindestens 50% im Vergleich zum Anteil an kurzen Seitenketten mit einem DP von 6 bis 11 von Amylopektin aus Wildtyp-Pflanzen verstanden werden und/oder eine Erhöhung des Anteils an kurzen Seitenketten mit einem DP von 16 bis 22 um mindestens 5%, bevorzugt um mindestens 10%, insbesondere um mindestens 15% und besonders bevorzugt um mindestens 30% im Vergleich zum Anteil an kurzen Seitenketten mit einem DP von 16 bis 22 von Amylopektin aus Wildtyp-Pflanzen.

Die Bestimmung des Anteils an kurzen Seitenketten erfolgt über die Bestimmung des prozentualen Anteils einer bestimmten Seitenkette am Gesamtanteil aller Seitenketten. Der Gesamtanteil aller Seitenketten wird ermittelt über die Bestimmung der Gesamtfläche unter den Peaks, die im HPLC-Chromatogramm die Polymerisationsgrade von DP 6 bis 26 repräsentieren. Der prozentuale Anteil einer bestimmten Seitenkette am Gesamtanteil aller Seitenketten wird ermittelt über die Bestimmung des Verhältnisses der Fläche unter dem Peak, der diese Seitenkette im HPLC-Chromatogramm repräsentiert, zur Gesamtfläche. Zur Bestimmung der Peakflächen kann beispielsweise das Programm Chromelion 6.20 der Firma Dionex, USA, verwendet werden.

Die erfindungsgemäßen Pflanzenzellen können zur Regeneration ganzer Pflanzen verwendet werden.

Die durch Regeneration der erfindungsgemäßen transgenen Pflanzenzellen erhältlichen Pflanzen sind ebenfalls Gegenstand der vorliegenden Erfindung.

Die erfindungsgemäßen Pflanzenzellen können zu jeder beliebigen Pflanzenspezies gehören, d.h. sowohl zu monokotyledonen als auch zu dikotyledonen Pflanzen. Bevorzugt handelt es sich um Pflanzenzellen aus landwirtschaftlichen Nutzpflanzen, d.h. aus Pflanzen, die vom Menschen kultiviert werden für Zwecke der Ernährung oder für technische, insbesondere industrielle Zwecke. Vorzugsweise betrifft die Erfindung faserbildende (z.B. Flachs, Hanf, Baumwolle), ölspeichernde (z.B. Raps, Sonnenblume, Sojabohne), zuckerspeichernde (z.B. Zuckerrübe, Zuckerrohr, Zuckerhirse) und proteinspeichernde Pflanzen (z.B. Leguminosen).
In einer weiteren bevorzugten Ausführungsform betrifft die Erfindung Futterpflanzen, insbesondere Futter- und Weidegräser (Alfalfa, Klee etc.), und Gemüsepflanzen (z.B. Tomate, Salat, Chicoree).

In einer weiteren bevorzugten Ausführungsform betrifft die Erfindung Pflanzenzellen aus stärkespeichernden Pflanzen (z.B. Weizen, Gerste, Hafer, Roggen, Kartoffel, Mais, Reis, Erbse, Maniok), besonders bevorzugt sind Pflanzenzellen aus Kartoffel.

Für die Einführung von DNA in eine pflanzliche Wirtszelle stehen eine Vielzahl von Techniken zur Verfügung. Diese Techniken umfassen die Transformation pflanzlicher Zellen mit T-DNA unter Verwendung von *Agrobacterium tumefaciens* oder *Agrobacterium rhizogenes* als Transformationsmittel, die Fusion von Protoplasten, die Injektion, die Elektroporation von DNA, die Einbringung der DNA mittels des biolistischen Ansatzes sowie weitere Möglichkeiten.
Die Verwendung der Agrobakterien-vermittelten Transformation von Pflanzenzellen ist intensiv untersucht und ausreichend in EP 120516; Hoekema, IN: The Binary Plant Vector System Offsetdrukkerij Kanters B.V., Alblasserdam (1985), Chapter V; Fraley et al., Crit. Rev. Plant Sci. 4, 1-46 und bei An et al. EMBO J. 4, (1985), 277-287 beschrieben worden. Für die Transformation von Kartoffel, siehe z.B. Rocha-Sosa et al., EMBO J. 8, (1989), 29-33.).

Auch die Transformation monokotyler Pflanzen mittels auf Agrobakterium Transformation basierender Vektoren wurde beschrieben (Chan et al., Plant Mol. Biol. 22, (1993), 491-506; Hiei et al., Plant J. 6, (1994) 271-282; Deng et al, Science in China 33, (1990), 28-34; Wilmink et al., Plant Cell Reports 11, (1992), 76-80; May et al., Bio/Technology 13, (1995), 486-492; Conner und Domisse, Int. J. Plant Sci. 153 (1992), 550-555; Ritchie et al, Transgenic Res. 2, (1993), 252-265). Alternatives System zur Transformation von monokotylen Pflanzen ist die Transformation mittels des biolistischen Ansatzes (Wan und Lemaux, Plant Physiol. 104, (1994), 37-48; Vasil et al., Bio/Technology 11 (1993), 1553-1558; Ritala et al., Plant Mol. Biol. 24, (1994), 317-325; Spencer et al., Theor. Appl. Genet. 79, (1990), 625-631), die Protoplastentransformation, die Elektroporation von partiell permeabilisierten Zellen, die Einbringung von DNA mittels Glasfasern. Insbesondere die Transformation von Mais wird in der Literatur mehrfach beschrieben (vgl. z. B. WO95/06128, EP0513849, EP0465875, EP0292435; Fromm et al., Biotechnology 8, (1990), 833-844; Gordon-Kamm et al., Plant Cell 2, (1990), 603-618; Koziel et al., Biotechnology 11 (1993), 194-200; Moroc et al., Theor. Appl. Genet. 80, (1990), 721-726).

Auch die erfolgreiche Transformation anderer Getreidearten wurde bereits beschrieben, z.B. für Gerste (Wan und Lemaux, s.o.; Ritala et al., s.o.; Krens et al., Nature 296, (1982), 72-74) und für Weizen (Nehra et al., Plant J. 5, (1994), 285-297). Alle vorstehenden Methoden sind im Rahmen der vorliegenden Erfindung geeignet.
Generell kommt für die Expression des fremden Nukleinsäuremoleküls (der fremden Nukleinsäuremoleküle) jeder in pflanzlichen Zellen aktive Promotor in Frage. Der Promotor kann dabei so gewählt sein, dass die Expression in den erfindungsgemäßen Pflanzen konstitutiv erfolgt oder nur in einem bestimmten Gewebe, zu einem bestimmten Zeitpunkt der Pflanzenentwicklung oder zu einem durch äußere Einflüsse determinierten Zeitpunkt. In Bezug auf die Pflanze kann der Promotor homolog oder heterolog sein.

In einer weiteren Ausführungsform der Erfindung wird zur Reduzierung der Aktivität eines oder mehrerer SSIII-Proteine und/oder BEI-Proteine und/oder BEII-Proteine in pflanzlichen Zellen mindestens eine antisense-RNA exprimiert.

Gegenstand der vorliegenden Erfindung ist daher auch eine erfindungsgemäße Pflanzenzelle, worin besagte fremde Nukleinsäuremoleküle ausgewählt sind aus der Gruppe bestehend aus
a) DNA-Molekülen, die mindestens eine antisense-RNA codieren, welche eine Verringerung der Expression von mindestens einem endogenen Gen bewirkt, das SSIII-Proteine und/oder BEI-Proteine und/oder BEII-Proteine codiert;
b) DNA-Molekülen, die über einen Cosuppressionseffekt zu Verringerung der Expression von mindestens einem endogenen Gen führen, das SSIII-Protein(e) und/oder BEI-Protein(e) und/oder BEII-Protein(e) codiert;
c) DNA-Molekülen, die mindestens ein Ribozym codieren, das spezifisch Transkripte von mindestens einem endogenen Gen spaltet, das SSIII-Proteine und/oder BEI-Proteine und/oder BEII-Proteine codiert; und
d) Mittels in vivo-Mutagenese eingeführte Nukleinsäuremoleküle, die zu einer Mutation oder einer Insertion einer heterologen Sequenz in mindestens einem endogenen SSIII-Protein(e) und/oder BEI-Protein(e) und/oder BEII-Protein(e) codierenden Gen führen, wobei die Mutation oder Insertion eine Verringerung der Expression von mindestens einem SSIII-Protein(e) und/oder BEI-Protein(e) und/oder BEII-Protein(e) codierenden Gen bewirkt, oder die Synthese von inaktiven SSIII- und/oder BEI- und/oder BEII-Proteinen;
e) DNA-Molekülen, die simultan mindestens eine antisense-RNA und mindestens eine sense-RNA codieren, wobei besagte antisense-RNA und besagte sense-RNA ein doppelsträngiges RNA-Molekül ausbilden, das eine Verringerung der Expression von mindestens einem endogenen Gen bewirkt, das SSIII-Protein(e) und/oder BEI-Protein(e) und/oder BEII-Protein(e) codiert;
f) DNA Molekülen, die Transposons enthalten, wobei die Integration der Transposonsequenzen zu einer Mutation oder einer Insertion in mindestens einem endogenen SSIII-Protein(e) und/oder BEI-Protein(e) und/oder BEII-Protein(e) codierenden Gen führt, welche zu einer Verringerung der Expression von mindestens einem SSIII-Protein(e) und/oder BEI-Protein(e) und/oder BEII-Protein(e) codierenden Gen bewirkt, oder die Synthese von inaktiven SSIIIund/oder BEI- und/oder BEII-Proteinen zur Folge hat; und
g) T-DNA Moleküle, die durch Insertion in mindestens einem endogenen SSIII-Protein(e) und/oder BEI-Protein(e) und/oder BEII-Protein(e) codierenden Gen eine Verringerung der Expression von mindestens einem endogenen SSIII-Protein(e) und/oder BEI-Protein(e) und/oder BEII-Protein(e) codierenden Gen bewirkt, oder die Synthese von inaktiven SSIII- und/oder BEI- und/oder BEII-Proteinen zur Folge haben.

Nach einem weiteren Aspekt betrifft die vorliegende Erfindung Vermehrungsmaterial jeglicher Art von erfindungsgemäßen Pflanzen.

Ein weiterer Aspekt der vorliegenden Erfindung betrifft die Verwendung der hierin beschriebenen Nukleinsäuremoleküle zur Herstellung der erfindungsgemäßen Pflanzenzellen und Pflanzen.

Ein weiterer Aspekt der vorliegenden Erfindung betrifft eine Zusammensetzung, enthaltend mindestens eines der vorstehenden Nukleinsäuremoleküle, wobei das mindestens eine Nukleinsäuremolekül nach Einführung in eine Pflanzenzelle zur Verringerung mindestens eines endogen in der Pflanzenzelle vorkommenden SSIII-Proteins und mindestens eines endogen in der Pflanzenzelle vorkommenden BEII-Proteins, und vorzugsweise weiterhin zur Verringerung mindestens eines endogen in der Pflanzenzelle vorkommenden BEI-Proteins führt. Die Zusammensetzung kann ein oder mehrere Nukleinsäurekonstrukte (vgl. oben) enthalten.

Ein weiterer Aspekt der vorliegenden Erfindung betrifft die Verwendung der erfindungsgemäßen Zusammensetzungen zur Herstellung der erfindungsgemäßen Pflanzenzellen und Pflanzen sowie eine Wirtszelle, insbesondere eine Pflanzenzelle, enthaltend die erfindungsgemäße Zusammensetzung.

Noch ein weiterer Aspekt der vorliegenden Erfindung betrifft ein Transformationssystem für Pflanzenzellen, enthaltend mindestens ein Nukleinsäuremolekül und mindestens eine Pflanzenzelle, wobei das mindestens eine Nukleinsäuremolekül zur Verringerung der Aktivität jeweils mindestens eines der endogen in der Pflanzenzelle vorkommenden SSIII-, BEI- und BE-II-Proteine führt, soweit diese nicht bereits durch eine vorherige genetische Modifikation der besagten Pflanzenzelle in ihrer Aktivität verringert wurden. Unter "Transformationssystem" wird im Rahmen der vorliegenden Erfindung also eine Kombination aus mindestens einer zu transformierenden Pflanzenzelle und mindestens einem zur Transformation verwendeten Nukleinsäuremolekül wie vorstehend beschrieben verstanden. Es können weitere, dem Fachmann auf dem Gebiet der Transformation von Pflanzenzellen geläufige Komponenten, die bei der Transformation nützlich sind, einschließlich von Puffern und dergleichen, im erfindungsgemäßen Transformationssystem enthalten sein.

### Beschreibung der Abbildungen

Fig 1:
   Graphische Darstellung der Viskositätseigenschaften von Stärke aus Kartoffelpflanzen. Die Analyse wurde mit einem Rapid Visco Analyser (Newport Scientific Pty Ltd., Investmet Support Group, Warriewod NSW 2102, Australien) durchgeführt. Die Bedingungen, unter welchen die Analyse durchgeführt wurde, sind unter RVA-Analysemethode 1 im Abschnitt "Allgemeine Methoden" beschrieben. Die untersuchte Stärke wurde aus Knollen von Wildtyp-Pflanzen (WT), Pflanzen mit einer reduzierten Aktivität eines SSIII-Proteins und eines BEI-Proteins (038VL008 und 038VL107) oder aus Pflanzen, die eine reduzierte Aktivität eines SSIII-Proteins und eines BEI-Proteins und eines BEII-Proteins (110CF003 und 108CF041) aufweisen, isoliert. Die Isolierung der Stärke erfolgte nach dem unter "Beispiele", "Extraktionsprozeß der Stärke aus Kartoffeln" beschriebenen Verfahren.
Fig 2:
   Graphische Darstellung der Viskositätseigenschaften von Stärke aus Kartoffelpflanzen. Die Analyse wurde mit einem Rapid Visco Analyser (Newport Scientific Pty Ltd., Investmet Support Group, Warriewod NSW 2102, Australien) durchgeführt. Die Bedingungen, unter welchen die Analyse durchgeführt wurde, sind unter RVA-Analysemethode 2 im Abschnitt "Allgemeine Methoden" beschrieben. Die untersuchte Stärke wurde aus Knollen von Wildtyp-Pflanzen (WT), Pflanzen mit einer reduzierten Aktivität eines SSIII-Proteins und eines BEI-Proteins (038VL008 und 038VL107) oder aus Pflanzen, die eine reduzierte Aktivität eines SSIII-Proteins und eines BEI-Proteins und eines BEII-Proteins (110CF003 und 108CF041 ) aufweisen, isoliert. Die Isolierung der Stärke erfolgte nach dem unter "Beispiele", "Extraktionsprozeß der Stärke aus Kartoffeln" beschriebenen Verfahren.
Fig 3:
   Graphische Darstellung der Viskositätseigenschaften von Stärke aus Kartoffelpflanzen. Die Analyse wurde mit einem Rapid Visco Analyser (Newport Scientific Pty Ltd., Investmet Support Group, Warriewod NSW 2102, Australien) durchgeführt. Die Bedingungen, unter welchen die Analyse durchgeführt wurde, sind unter RVA-Analysemethode 3 im Abschnitt "Allgemeine Methoden" beschrieben. Die untersuchte Stärke wurde aus Knollen von Wildtyp-Pflanzen (WT), Pflanzen mit einer reduzierten Aktivität eines SSIII-Proteins und eines BEI-Proteins (038VL008 und 038VL107) oder aus Pflanzen, die eine reduzierte Aktivität eines SSIII-Proteins und eines BEI-Proteins und eines BEII-Proteins (110CF003 und 108CF041) aufweisen, isoliert. Die Isolierung der Stärke erfolgte nach dem unter "Beispiele", "Extraktionsprozeß der Stärke aus Kartoffeln" beschriebenen Verfahren.
Fig 4:
   Rasterelektronenmikroskopische Aufnahme eines Kartoffelstärkekorns, welches aus Wildtyp-Pflanzen isoliert wurde.
Fig 5:
   Rasterelektronenmikroskopische Aufnahme eines Kartoffelstärkekorns, welches aus Pflanzen isoliert wurde, die eine verringerte Aktivität eines SSIII-Proteins und eines BEI-Proteins und eines BEII-Proteins aufweisen (110CF003).
Fig 6:
   Schematische Darstellung des Vektors pGSV71-α-BEII-basta, welcher zur erneuten Transformation von Pflanzen, die bereits eine reduzierte Aktivität eines SSIII-Proteins und eines BEI-Proteins aufwiesen, benutzt wurde.
   (RB, linke T-DNA Border, LB, rechte T-DNA Border; CaMV35, 35S Promotor des Blumenkohl Mosaik Virus; NOS, Polyadenylierungssequenz des Nopalinsynthase Gens aus *Agrobacterium tumefaciens*; OCS, Polyadenylierungssequenz des Octopinsynthase Gens aus *Agrobacterium tumefaciens*; B33, Promotor des Patatin Gens aus Kartoffel; BEII, kodierende Sequenzen des BEII Gens aus Kartoffel; bar, Sequenz kodierend eine Phosphinothricinacetyltransferase aus *Streptomyces hygroscopicus*)
Fig 7:
   Schematische Darstellung des Vektors pB33-α-BE-α-SSIII-Kan, welcher zur Herstellung von transgenen Pflanzen mit reduzierter Aktivität eines SSIII-Proteins und eines BEI-Proteins benutzt wurde. (RB, linke T-DNA Border, LB, rechte T-DNA Border; nos5', Promotor des Nopalin Synthase Gens aus *Agrobacterium tumefaciens*; nptll, Gen kodierend die Aktivität einer Neomycinphosphotransferase; nos3' Polyadenylierungssequenz des Nopalinsynthase Gens aus *Agrobacterium tumefaciens*; OCS Polyadenylierungssequenz des Octopinsynthase Gens aus *Agrobacterium tumefaciens*; B33, Promotor des Patatin Gens aus Kartoffel; BE, kodierende Sequenzen des BEI Gens aus Kartoffel; SSIII kodierende Sequenzen des SSIII Gens aus Kartoffel)

### Beschreibung der Sequenzen

Seq ID 1:
   Nukleinsäuresequnz der Stärkesynthase SSIII aus Kartoffel *(Solanum tuberosum)* mit Angabe der Sequenzen, die für das entsprechende SSIII Protein kodieren.
Seq ID 2:
   Aminosäuresequenz eines SSIII Proteins aus Kartoffel.
Seq ID 3:
   Aminosäuresequenz der Pfam cbm25 Bindedömäne des SSIII-Proteins aus Kartoffel (*Solanum tuberosum).*
Seq ID 4:
   Kodierende Nukleinsäuresequenz des Verzweigungsenzyms BEI aus Kartoffel (*Solanum tuberosum).*
Seq ID 5:
   Aminosäuresequenz des Verzweigungsenzyms BEI aus Kartoffel (*Solanum tuberosum)*
Seq ID 6:
   Kodierende Nukleinsäuresequenz des Verzweigungsenzyms BEII aus Kartoffel (*Solanum tuberosum)*.
Seq ID 7:
   Aminosäuresequenz des Verzweigungsenzyms BEII aus Kartoffel (*Solanum tuberosum)*
Seq ID 8:
   Mittels PCR amplifizierte Nukleinsäuresequenz des Verzweigungsenzyms BEII aus Kartoffel (*Solanum tuberosum)*

### Allgemeine Methoden

In den Beispielen wurden die folgenden Methoden verwendet:

### Stärkeanalytik

### a) Bestimmung des Amylosegehaltes bzw. des Amylose/Amylopektinverhältnisses

Stärke wurde nach Standardmethoden aus Kartoffelpflanzen isoliert, und der Amylosegehalt sowie das Verhältnis Amylose zu Amylopektin wurde nach der von Hovenkamp-Hermelink et al. beschriebenen Methode (Potato Research 31, (1988), 241-246) bestimmt.

### b) Bestimmung des Phosphatgehaltes

In der Stärke können die Positionen C2, C3 und C6 der Glukoseeinheiten phosphoryliert sein. Zur Bestimmung des C6-P-Gehaltes der Stärke werden 50 mg Stärke in 500 µl 0,7 M HCl 4 h bei 95°C hydrolysiert. Anschließend werden die Ansätze für 10 min bei 15500 g zentrifugiert und die Überstände abgenommen. Von den Überständen werden 7µl mit 193 µl Imidazol-Puffer (100 mM Imidazol, pH 7,4; 5 mM MgCl₂, 1 mM EDTA und 0,4 mM NAD) gemischt. Die Messung wurde im Photometer bei 340 nm durchgeführt. Nach der Etablierung einer Basisabsorption wurde die Enzymreaktion durch die Zugabe von 2 Einheiten (units) Glukose-6-Phosphat Dehydrogenase (von Leuconostoc mesenteroides, Boehringer Mannheim) gestartet. Die Absorptionsänderung ist direkt proportional zur Konzentration des G-6-P Gehaltes der Stärke.

Die Bestimmung des Gesamtphosphatgehaltes erfolgte nach der Methode von Ames (Methods in Enzymology VIII, (1966), 115-118).
Es werden ca. 50 mg Stärke mit 30 µl ethanolischer Magnesiumnitrat-Lösung versetzt und drei Stunden bei 500°C im Muffelofen verascht. Der Rückstand wird mit 300 µl 0,5 M Salzsäure versetzt und 30 min bei 60°C inkubiert. Anschließend wird ein Aliquot auf 300 µl 0,5 M Salzsäure aufgefüllt, zu einer Mischung aus 100 µl 10%iger Ascorbinsäure und 600 µl 0,42% Ammoniummolybdat in 2 M Schwefelsäure gegeben und 20 min bei 45°C inkubiert.

Es folgt eine photometrische Bestimmung bei 820nm unter Berücksichtigung einer Phosphat-Eichreihe als Standard.

### c) Bestimmung der Gelfestigkeit (Texture Analyser)

1,5 g Stärke (TS) werden in 25 ml einer wäßrigen Suspension im RVA-Gerät verkleistert (Temperaturprogramm: s. Punkt d) "Bestimmung der Viskositätseigenschaften mittels eines Rapid Visco Analyzers (RVA)") und anschließend für 24 h bei Raumtemperatur in einem geschlossenen Gefäß gelagert. Die Proben werden unter der Sonde (zylindrischer Stempel mit planer Oberfläche) eines Texture Analysers TA-XT2 der Firma Stable Micro Systems (Surrey, UK) fixiert und die Gelfestigkeit mit folgenden Parametern bestimmt:
- Test-Geschwindigkeit 0,5 mm/s
- Eindringtiefe 7 mm
- Kontaktfläche 113 mm²
- Druck 2 g

### d) Bestimmung der Viskositätseigenschaften mittels eines Rapid Visco Analyzers (RVA)

### Standardmethode

2 g Stärke (TS) werden in 25 ml H₂O (VE Wasser, Leitfähigkeit von mindestens 15 mega Ohm) aufgenommen und für die Analyse in einem Rapid Visco Analyser (Newport Scientific Pty Ltd., Investmet Support Group, Warriewod NSW 2102, Australien) verwendet. Der Betrieb des Gerätes erfolgt nach den Angaben des Herstellers. Dabei erfolgt die Angabe der Viskositätswerte in RVUs gemäß der Betriebsanleitung des Herstellers, die hiermit insoweit durch Bezugnahme in die Beschreibung aufgenommen wird. Zur Bestimmung der Viskosität der wäßrigen Lösung der Stärke wird die Stärkesuspension zunächst für eine Minute auf 50°C erhitzt (Schritt 1), danach von 50°C auf 95°C mit einer Geschwindigkeit von 12°C pro Minute (Schritt 2) erhitzt. Anschließend wird die Temperatur für 2.5 Min bei 95°C gehalten (Schritt 3). Danach wird die Lösung von 95°C auf 50°C abgekühlt, mit einer Geschwindigkeit von 12°C pro Minute (Schritt 4). Während der gesamten Dauer wird die Viskosität bestimmt. Insbesondere in den Fällen, wo bei der Einwage von 2,0 g (TS) Stärke in 25 ml H₂O (VE Wasser, Leitfähigkeit von mindestens 15 mega Ohm) die Grenzen des Meßbereichs des RVA nicht ausreichten, wurden nur 1,5g Stärke (TS) in 25 ml H₂O (VE Wasser, Leitfähigkeit von mindestens 15 mega Ohm) aufgenommen.

Um eine Vergleichbarkeit mit dem Stand der Technik herzustellen wurde zudem in einigen Fällen ein geändertes Temperaturprofil benutzt.
Folgende Temperaturprofile wurden verwendet:

### RVA-Analysemethode 1:

Zur Bestimmung der Viskosität einer 6%igen wässrigen Lösung der Stärke wird die Stärkesuspension zunächst für 10 Sekunden mit 960 rpm gerührt, um anschließend bei einer Rührgeschwindigkeit von 160 rpm für zunächst eine Minute auf 50 °C erhitzt zu werden (Schritt 1). Danach folgt eine Erhöhung der Temperatur von 50 °C auf 95 °C mit einer Heizgeschwindigkeit von 12°C pro Minute (Schritt 2). Die Temperatur wird für 2.5 Minuten bei 95 °C gehalten (Schritt 3) und danach mit 12 °C pro Minute von 95 °C auf 50 °C gekühlt (Schritt 4). Der letzte Schritt (Schritt 5) hält die Temperatur von 50°C für 2 Minuten.
Nach Beendigung des Programms wird der Rührer entfernt und der Becher abgedeckt. Die verkleisterte Stärke steht nun für die Texturanalyse nach 24 h zur Verfügung.

### RVA- Analysemethode 2:

Zur Bestimmung der Viskosität einer 6%igen wässrigen Lösung der Stärke wird die Stärkesuspension zunächst für 10 Sekunden mit 960 rpm gerührt, um anschließend bei einer Rührgeschwindigkeit von 160 rpm für zunächst zwei Minuten auf 50 °C erhitzt zu werden (Schritt 1). Danach erfolgt eine Erhöhung der Temperatur von 50 °C auf 95 °C mit einer Heizgeschwindigkeit von 1,5 °C pro Minute (Schritt 2). Die Temperatur wird für 15 Minuten bei 95 °C gehalten (Schritt 3) und danach mit 1.5 °C pro Minute von 95 °C auf 50 °C gekühlt (Schritt 4). Der letzte Schritt (Schritt 5) hält die Temperatur von 50°C für 15 Minuten.
Nach Beendigung des Programms wird der Rührer entfernt und der Becher abgedeckt. Die verkleisterte Stärke steht nun für die Texturanalyse nach 24 h zur Verfügung.

### RVA Analysemethode 3:

Zur Bestimmung der Viskosität einer 10%igen wässrigen Lösung der Stärke wird die Stärkesuspension zunächst für 10 Sekunden mit 960 rpm gerührt, um anschließend bei einer Rührgeschwindigkeit von 160 rpm für zunächst zwei Minuten auf 50 °C erhitzt zu werden (Schritt 1). Danach folgt eine Erhöhung der Temperatur von 50 °C auf 95 °C mit einer Heizgeschwindigkeit von 1,5 °C pro Minute (Schritt 2). Die Temperatur wird für 15 Minuten bei 95 °C gehalten (Schritt 3) und danach mit 1,5 °C pro Minute von 95 °C auf 50 °C gekühlt (Schritt 4). Der letzte Schritt (Schritt 5) hält die Temperatur von 50°C für 15 Minuten. Dieses Profil der RVA Analyse entspricht desjenigen, welches in WO 96 34968 angewendet wurde.
Nach Beendigung des Programms wird der Rührer entfernt und der Becher abgedeckt. Die verkleisterte Stärke steht nun für die Texturanalyse nach 24 h zur Verfügung.

Im Profil der RVA Analyse gibt es charakteristische Werte, die zum Vergleich unterschiedlicher Messungen und Substanzen dargestellt werden. Die folgenden Begriffe sollen im Zusammenhang mit der vorliegenden Erfinung wie folgt verstanden werden:
1. Maximale Viskosität (RVA Max)
   Unter der maximalen Viskosität versteht man den höchsten Viskositätswert, gemessen in RVUs, der in Schritt 2 oder 3 des Temperaturprofils erreicht wird.
2. Minimale Viskosität (RVA Min)
   Unter der minimalen Viskosität versteht man den geringsten Viskositätswert, gemessen in RVUs, der nach der Maximalen Viskosität im Temeraturprofil auftritt. Normalerweise erfolgt dieses in Schritt 3 des Temperaturprofils.
3. Finale Viskosität (RVA Fin)
   Unter der Finalen Viskosität versteht man den Viskositätswert, gemessen in RVUs, der am Ende der Messung auftritt.
4. Setback (RVA Set)
   Der sogenannte "Setback" wird berechnet, indem man den Wert der Finalen Viskosoität von der desjenigen Minimums, welches im Kurvenverlauf nach erreichen der Maximalen Viskosität auftritt, subtrahiert.
5. Verkleisterungstemperatur (RVA T)
   Unter der Verkleisterungstemperatur versteht man die Zeit im Temperaturprofil, zu welcher die Viskosität zuerst stark für eine kurze eine Periode ansteigt.

### e) Analyse der Seitenkettenverteilung des Amylopektins mittels lonenaustauschchromatographie

Zur Trennung von Amylose und Amylopektin werden 200mg Stärke in 50ml Reaktionsgefäßen mit 12ml 90% (v/v) DMSO in H₂O gelöst. Nach Zugabe von 3 Volumen Ethanol wird das Präzipitat durch 10 minütige Zentrifugation bei etwa 1800g bei Raumtemperatur (RT) abgetrennt. Das Pellet wird dann mit 30ml Ethanol gewaschen, getrocknet und in 40ml 1% (w/v) NaCl Lösung bei 75°C gelöst. Nach Abkühlung der Lösung auf 30°C werden langsam etwa 90mg Thymol zugegeben und diese Lösung für mindestens 60h bei 30°C inkubiert. Anschließend wird die Lösung für 30 min bei 2000g (RT) zentrifugiert. Der Überstand wird dann mit 3 Volumen Ethanol versetzt und das ausfallende Amylopektin mittels 5 minütiger Zentrifugation bei 2000g (RT) abgetrennt. Das Pellet (Amylopektin) wird dann mit Ethanol gewaschen und mittels Aceton getrocknet. Durch Zugabe von DMSO zum Pellet wird eine 1% Lösung hergestellt von der 200µl mit 345µl Wasser, 10µl 0.5M Natriumacetat (pH3.5) und 5µl Isoamylase (1:10 verdünnt; Megazyme) versetzt und bei 37°C für etwa 16h inkubiert werden. Eine wässrige 1:5 Verdünnung dieses Verdaus wird anschließend mit einem 0.2µm Filter filtriert und 100µl des Filtrats ionenchromatografisch analysiert (HPAEC-PAD, Dionex). Die Separation erfolgte mit einer PA-100 Säule (mit entsprechender Vorsäule), die Detektion amperometrisch. Die Elutionsbedingungen waren wie folgt:
Lösung A - 0.15M NaOH
Lösung B - 1 M Natriumacetat in 0.15M NaOH

**Tabelle 1:**

| Zusammensetzung des Elutionspuffers für die Seitenkettenanalyse des Amylopektins zu verschiedene Zeiten während der HPEAC-PAD Dionex Analyse. Zwischen den angegebenen Zeitpunkten verändert der Elutionspuffer seine Zusammensetzung jeweils einem linearen Verlauf folgend. | | |
|---|---|---|
| t (min) | Lösung A (%) | Lösung B (%) |
| 5 | 0 | 100 |
| 35 | 30 | 70 |
| 45 | 32 | 68 |
| 60 | 100 | 0 |
| 70 | 100 | 0 |
| 72 | 0 | 100 |
| 80 | 0 | 100 |
| stop | | |

Die Bestimmung des relativen Anteils an kurzen Seitenketten am Gesamtanteil aller Seitenketten erfolgt über die Bestimmung des prozentualen Anteils einer bestimmten Seitenkette am Gesamtanteil aller Seitenketten. Der Gesamtanteil aller Seitenketten wird ermittelt über die Bestimmung der Gesamtfläche unter den Peaks, die im HPCL-Chromatogramm die Polymerisationsgrade von DP6 bis 26 repräsentieren.

Der prozentuale Anteil einer bestimmten Seitenkette am Gesamtanteil aller Seitenketten wird ermittelt über die Bestimmung des Verhältnisses der Fläche unter dem Peak, der diese Seitenkette im HPLC-Chromatogramm repräsentiert, zur Gesamtfläche. Zur Bestimmung der Peakflächen wurde das Programm Chromelion 6.20 Version 6.20 der Firma Dionex, USA, verwendet werden.

### f) Korngrößenbestimmung

Stärke wurde nach Standardmethoden aus Kartoffelknollen extrahiert (siehe Beispiele).
Die Korngrößenbestimmung wurde dann mit einem Fotosedimentometer des Typs "Lumosed FS1" der Firma Retsch GmbH, Deutschland unter Verwendung der Software V.2.3 durchgeführt. Die Softwareeinstellungen wurden wie folgt festgelegt:

| | | |
|---|---|---|
| Stoff-Daten | Kalibration Nr. 0 | |
| | Dichte [kg/m³] 1500 | |
| Sedimentationsflüssigkeit | Typ Wasser | |
| | Viskosität [Pa s] | 0.001 |
| | Dichte [kg/m³] | 1000 |
| | Zusatz | - |
| | Mess-Daten | 5 min |
| | Siebschnitt [µm] | 250 |
| | Durchgang [%] | 100 |
| | Messbereich | 4.34 - 117.39 µm |
| | Eichung | N |
| | Temperatur | 20°C |

Die Körngrößenverteilung wurde in wäßriger Lösung bestimmt und erfolgte nach Herstellerangaben sowie basierend auf der Literatur von z.B. H. Pitsch, Korngrößenbestimmung; LABO-1988/3 Fachzeitschrift für Labortechnik, Darmstadt.

### g) Aufnahmen mit dem Rasterelektronenmikroskop (REM)

Für die Untersuchung der Oberfläche der Stärkeproben wurden diese auf Probenträger mit leitfähigem Kleber aufgestäubt. Zur Vermeidung von Aufladung wurden die Probenträger abschließend mit einer 4 nm Pt-Schicht besputtet. Die Untersuchungen der Stärkeproben wurden mit dem Feldemissions-Rasterelektronenmikroskop JSM 6330 F (Jeol) bei einer Beschleunignungsspannung von 5 kV durchgeführt.

### h) Bestimmung der Aktivität der SSIII, BEI- und BEII-Proteine

Die Bestimmungen erfolgten wie in den Beispielen angegeben.

### Beispiele

### Herstellung des Expressionsvektors ME5/6

pGSV71 ist ein Derivat des Plasmides pGSV7, welches sich vom intermediären Vektor pGSV1 ableitet. pGSV1 stellt ein Derivat von pGSC1700 dar, dessen Konstruktion von Cornelissen und Vanderwiele (Nucleic Acid Research 17, (1989), 19-25) beschrieben wurde. pGSV1 wurde aus pGSC1700 erhalten, durch Deletion des Carbenicillin Resistenzgen, sowie Deletion der T-DNA-Sequenzen der TL-DNA-Region des Plasmides pTiB6S3.
pGSV7 enthält den Replikationsursprung des Plasmides pBR322 (Bolivar et al., Gene 2, (1977), 95-113) sowie den Replikationsursprung des *Pseudomonas*-Plasmides pVS1 (Itoh et al., Plasmid 11, (1984), 206). pGSV7 enthält außerdem das selektierbare Markergen *aadA*, aus dem Transposon Tn1331 aus *Klebsiella pneumoniae*, welches Resistenz gegenüber den Antibiotika Spectinomycin und Streptomycin verleiht (Tolmasky, Plasmid 24 (3), (1990), 218-226; Tolmasky and Crosa, Plasmid 29(1), (1993), 31-40)
Das Plasmid pGSV71 wurde erhalten durch Klonierung eines chimären *bar*-Gens zwischen die Borderregionen von pGSV7. Das chimäre *bar*-Gen enthält die Promotorsequenz des Blumenkohlmosaikvirus zur Initiation der Transkription (Odell et al., Nature 313, (1985), 180), das *bar*-Gen aus *Streptomyces hygroscopicus* (Thompson et al., Embo J. 6, (1987), 2519-2523) und den 3'-untranslatierten Bereich des Nopalinsynthasegens der T-DNA von pTiT37, zur Termination der Transkription und Polyadenylierung. Das *bar*-Gen vermittelt Toleranz gegenüber dem Herbizid Glufosinat-Ammonium.
Die T-DNA enthält an Position 198-222 die rechte Randsequenz der TL-DNA aus dem Plasmid pTiB6S3 (Gielen et al., EMBO J. 3, (1984), 835-846). Zwischen Nukleotid 223-249 befindet sich eine Polylinker-Sequenz. Die Nukleotide 250-1634 enthalten die P35S3 Promotor-Region des Blumenkohl-Mosaik-Virus (Odell et al., siehe oben). Die codierende Sequenz des Phosphinothricin-Resistenzgen (*bar*) aus *Streptomyces hygroscopicus* (Thompson et al. 1987, siehe oben) ist zwischen den Nukleotiden 1635-2186 enthalten. Dabei wurden die zwei endständigen Codons am 5'-Ende des *bar*-Wildtyp-Gens ersetzt durch die Codons ATG und GAC. Zwischen den Nukleotiden 2187-2205 befindet sich eine Polylinker-Sequenz. Das 260 bp lange Taql-Fragment des nicht-translatierten 3'-Endes des Nopalinsynthase-Gens (3'nos) aus der T-DNA des Plasmides pTiT37 (Depicker et al., J. Mol. Appl. Genet. 1, (1982), 561-573) befindet sich zwischen den Nukleotiden 2206 und 2465. Die Nukleotide 2466-2519 enthalten eine Polylinker-Sequenz. Die linke Randsequenz der TL-DNA aus pTiB6S3 (Gielen et al., EMBO J. 3, (1984), 835-846) befindet sich zwischen den Nukleotiden 2520-2544. Der Vektor pGSV71 wurde dann mit dem Enzym Pstl aufgeschnitten und geglättet. Aus dem Vektor pB33-Kan wurde der B33 Promotor sowie die ocs-Kassette als *Eco*RI-*Hind*lll-Fragment ausgeschnitten und geglättet und in den mit *Pst*l aufgeschnittenen und geglätteten Vektor pGSV71 eingefügt. Der erhaltene Vektor diente als Ausgangsvektor zur Konstruktion von ME5/6: In die zwischen B33-Promotor und *ocs*-Element gelegene *Pst*l-Schnittstelle des Vektors ME4/6 wurde ein Oligonukleotid, enthaltend die Schnittstellen *Eco*RI, *Pac*I, *Spe*I, *Srf*I, *Spe*I, *Not*I, *Pac*I und *Eco*RI, unter Verdopplung der *Pst*l-Schnittstelle eingeführt. Der erhaltene Expressionsvektor wurde als ME5/6 bezeichnet.

### Beschreibung des Vektors pSK-Pac:

pSK-Pac ist ein Derivat des pSK-Bluescript (Stratagene, USA) bei dem flankierend zur multiplen Klonierungsstelle (MCS) je eine *Pac*I Schnittstelle eingeführt wurde.

### Herstellung transgener Kartoffelpflanzen, die eine verringerte Genexpression eines BEI- , SSIII-, und eines BEII-Gens aufweisen

Zur Erzeugung transgener Pflanzen, die eine verringerte Aktivität eines BEI, eines SSIII und eines BEII Proteins aufweisen, wurden zunächst transgene Pflanzen erzeugt, die eine verringerte Aktivität eines BEI und eines SSIII Proteins aufweisen. Zu diesem Zwecke wurde die T-DNA des Plasmids pB33-αBEI-αSSIII-Kan mit Hilfe von Agrobakterien, wie bei Rocha-Sosa et al. (EMBO J. 8, (1989), 23-29) beschrieben, in Kartoffelpflanzen transferiert.
Zur Konstruktion des Plasmids pB33-αBEI-αSSIII-Kan (siehe Fig 7) wurde zunächst der Expressionsvektor pBin33-Kan konstruiert. Dazu wurde der Promotor des Patatin Gens B33 aus *Solanum tuberosum* (Rocha-Sosa et al., 1989, siehe oben) als *Dra*I-Fragment (Nukleotide -1512 - +14) in den mit *Sst*l geschnittenen Vektor pUC19 (Genbank Acc. No. M77789), dessen Enden mit Hilfe der T4 DNA-Polymerase geglättet worden waren, ligiert. Daraus entstand das Plasmid pUC19-B33. Aus diesem Plasmid wurde der B33-Promotor mit *Eco*RI und *Sma*l herausgeschnitten und in den entsprechend geschnittenen Vektor pBinAR ligiert. Hieraus entstand der pflanzliche Expressionsvektor pBin33-Kan. Das Plasmid pBinAR ist ein Derivat des Vektorplasmids pBin19 (Bevan, Nucl. Acid Research 12, (1984), 8711-8721) und wurde von Höfgen and Willmitzer (Plant Sci. 66, (1990), 221-230) konstruiert. Anschließend wurde ein 1631 Bp langes Hindll-Fragment, welches eine partielle cDNA codierend für das BEI-Enzym aus Kartoffel enthält (Kossmann et al., 1991, Mol. & Gen. Genetics 230(1-2):39-44), geglättet und in "antisense"-Orientierung bezüglich des B33 Promotors (Promotor des Patatin Gens B33 aus Solanum tuberosum; Rocha-Sosa et al., 1989) in den mit Smal vorgeschnittenen Vektor pBinB33 eingeführt. Das erhaltene Plasmid wurde mit BamHI aufgeschnitten. In die Schnittstelle wurde ein 1363 Bp langes BamHI-Fragment, enthaltend eine partielle cDNA codierend für das SS III-Enzym aus Kartoffel (Abel et al., 1996, loc.cit.), ebenfalls in "antisense"-Orientierung bezüglich des B33-Promotors eingeführt.

Nach der Transformation konnten verschiedene Linien transgener Kartoffelpflanzen identifiziert werden, deren Knollen einen deutlich verringerte Aktivität eines BEI und SSIII-Proteins aufwiesen. Die aus dieser Transformation resultierenden Pflanzen wurden mit 038VL bezeichnet.
Zum Nachweis der Aktivität löslicher Stärkesynthasen (SSIII) durch nichtdenaturierende Gelelektrophorese wurden Gewebeproben von Kartoffelknollen in 50 mM Tris-HCl pH 7,6, 2 mM DTT, 2,5 mM EDTA, 10 % Glycerin und 0,4 mM PMSF aufgeschlossen. Die Elektrophorese wurde in einer MiniProtean II Kammer (BioRAD) durchgeführt. Die Monomerkonzentration der 1,5 mm dicken Gele war 7,5 % (w/v), als Gel- und Laufpuffer diente 25 mM Tris-Glycin pH 8,4. Gleiche Mengen an Proteinextrakt wurden aufgetragen und für 2 h bei 10 mA je Gel aufgetrennt.
Anschließend erfolgte die Inkubation der Aktivitäts-Gele in 50 mM Tricine-NaOH pH 8,5, 25 mM Kaliumacetat, 2 mM EDTA, 2 mM DTT, 1 mM ADP-Glukose, 0,1 % (w/v) Amylopektin und 0,5 M Natriumcitrat. Gebildete Glukane wurden mit Lugolscher Lösung angefärbt.
Der Nachweis der BEI Aktivität erfolgte ebenfalls mit Hilfe der nicht denaturierenden Gelelektrophorese:

Zur Isolierung von Proteinen aus Pflanzen wurde das Probenmaterial in flüssigem Stickstoff gemörsert, in Extraktionspuffer (50 mM Na-Citrat, pH 6.5; 1 mM EDTA, 4 mM DTT) aufgenommen und nach Zentrifugation (10 min, 14.000 g, 4 °C) direkt zur Messung der Proteinkonzentration nach Bradford eingesetzt. Anschließend wurde je nach Bedarf 5 bis 20 µg Gesamt-Proteinextrakt mit 4-fach Loading-Buffer (20% Glycerin, 125 mM Tris HCl, pH 6,8) versetzt und auf ein "BE-Aktivitätsgel" geladen. Der Laufpuffer (RB) setzte sich wie folgt zusammen: RB = 30,2 g Tris-Base, pH 8.0, 144 g Glycine auf 1 L H₂O.
Nach Beendigung des Gellaufes wurden die Gele in je 25 ml "Phosphorylase - Puffer" (25 ml 1M Na-Citrat pH 7,0, 0,47 g Glucose-1-Phosphat, 12,5 mg AMP, 2,5mg Phosphorylase a/b aus "rabbit") über Nacht bei 37 °C inkubiert. Die Färbung der Gele wurde mit Lugol'sche Lösung durchgeführt.

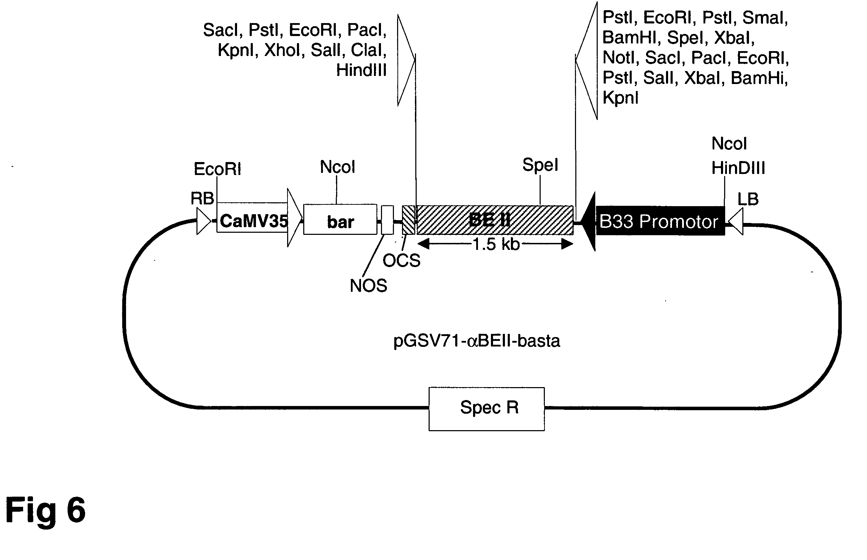

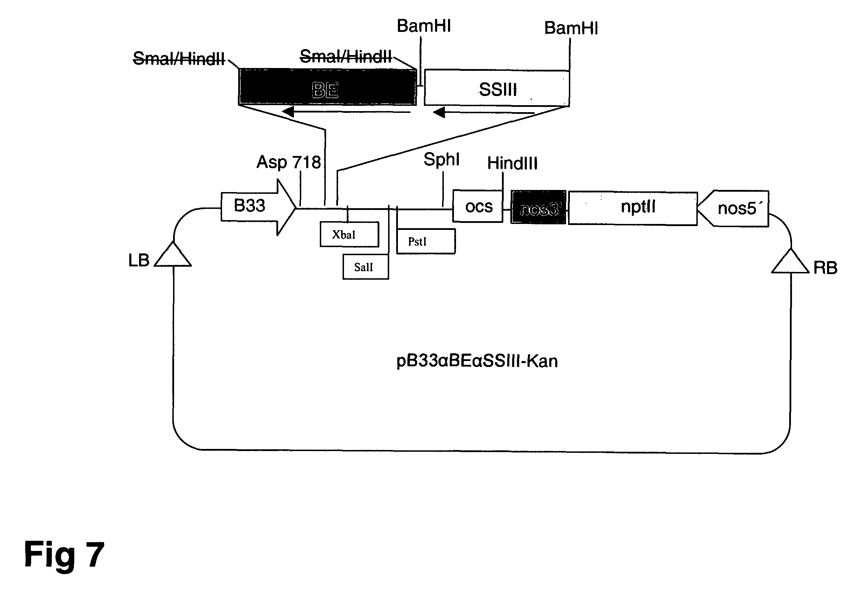

Weitergehende Analysen zeigten, dass isolierte Stärken der Linie 038VL008 und 038VL107, welche eine Reduzierung sowohl des BEI-, als auch des SSIII-Proteins aufweisen, den höchsten Phosphatgehalt aller untersuchten unabhängigen Transformanten aufwiesen.
Pflanzen dieser Linien wurden anschließend wie beschrieben bei Rocha-Sosa et al. (EMBO J. 8, (1989), 23-29) mit dem Plasmid pGSV71-αBEII-basta transformiert. Plasmid pGSV71-αBEII-basta wurde konstruiert, indem eine knollenspezifische Kartoffel cDNA Bank mit einem durch RT-PCR (Primer: 5'- gggggtgttggctttgacta und 5'cccttctcctcctaatccca; Stratagene ProSTAR™ HF Single-Tube RT-PCR System) mit Gesamt-RNA aus Knollen als Vorlage amplifizierten DNA Fragment nach Standardmethoden durchmustert wurde. Auf diese Weise wurde ein etwa 1250 bp großes DNA Fragment isoliert (SEQ ID No. 8), welches dann als EcoRV-SmaI Fragment in die EcoRV Schnittstelle des Klonierungsvektor pSK-Pac (siehe oben) subkloniert und abschließend als PacI Fragment in antisense Orientierung, bezogen auf den Promotor, in den Expressionsvektor ME5/6 ligiert wurde. Somit entstand Plasmid pGSV71-αBEII-basta (siehe Fig 6).

Von den durch Transformation mit dem Plasmid pGSV71-αBEII-basta erhaltenen Pflanzen, die als 108CF bzw. 110CF bezeichnet wurden, wurden Gewebeproben von Knollen der unabhängigen Transformanten genommen und deren Amylosegehalt ermittelt (siehe Methoden). Die Stärken der unabhängigen Linien, deren Knollen den höchsten Amylosegehalt aufwiesen, wurden für eine weitere Analyse der Stärkeeigenschaften herangezogen. Zum Nachweis, dass diese Pflanzen zusätzlich zu einer reduzierte Aktivität eines BEI- und SSIII-Protein auch eine reduzierte Aktivität eines BEII-Proteins aufweisen, wurde ebenfalls eine Analyse mit Hilfe der nicht denaturierenden Gelelektrophorese durchgeführt. Die Analyse wurde mit der gleichen Methode wie oben bereits für die Analyse der reduzierten BEI-Aktivität durchgeführt, außer, dass das nicht denaturierende Polyacrylamidgel zusätzlich zur oben beschriebenen Zusammensetzung 0,5% Maltodextrin (Beba, Maltodextrin-Lösung 15%ig für Neugeborene, Nestle) enthielt. Durch Zusatz des Dextrins konnten die unterschiedlichen Aktivitäten der BEI- und BEII- Proteine nach Inkubation der Gele in "Phosphorylase - Puffer" (25 ml 1 M Na-Citrat pH 7,0, 0,47 g Glucose-1-Phosphat, 12,5 mg AMP, 2,5mg Phosphorylase a/b aus "rabbit") über Nacht bei 37 °C und anschließender Färbung mit Lugol'scher Lösung in einem Gel dargestellt werden.

### Extraktionsprozess der Stärke aus Kartoffeln

Alle Knollen einer Linie (4 bis 5 kg) werden gemeinsam in einem handelsüblichen Entsafter (Multipress automatic MP80, Braun) aufgearbeitet. Das stärkehaltige Fruchtwasser wird in einem 10 L Eimer (Verhältnis Höhe des Eimers/Durchmesser des Eimers = ca. 1,1), in dem 200 ml Leitungswasser mit einer Löffelspitze (ca. 3-4 g) Natrium-Disulfit vorgelegt wurden, aufgefangen. Im Anschluss wird der Eimer mit Leitungswasser vollständig aufgefüllt. Nach einem 2 stündigen Absetzen der Stärke wird der Überstand abdekantiert, die Stärke erneut in 10 I Leitungswasser aufgeschwemmt und über ein Sieb mit 125 µm Maschenweite gegeben. Nach 2 Stunden (Stärke hat sich erneut am Boden des Eimers abgesetzt) wird erneut der wässrige Überstand dekantiert. Dieser Waschvorgang wird noch 3 mal wiederholt, so dass die Stärke insgesamt fünf mal in frischem Leitungswasser resuspendiert wird. Im Anschluss werden die Stärken bei 37 °C auf einen Wassergehalt von 12 - 17% getrocknet und im Mörser homogenisiert. Die Stärken stehen nun für Analysen zu Verfügung.

### Beispiel 2

### Analyse der Stärke von Pflanzen mit verringerter BEI-, SSIII- und BEII-Genexpression

Die Stärke verschiedener unabhängiger Linien der in Beispiel 1 beschriebenen Transformationen 108CF und 110CF wurden aus Kartoffelknollen isoliert. Anschließend wurden die physico-chemischen Eigenschaften dieser Stärke analysiert. Die Ergebnisse der Charakterisierung der modifizierten Stärken sind in Tabelle 2 (Tab.2) für eine beispielhafte Auswahl bestimmter Pflanzenlinien dargestellt. Die Analysen wurden nach den oben beschriebenen Methoden durchgeführt.

Die folgende Tabellen 2, 3 und 4 fassen die Ergebnisse der RVA Analyse bezogen auf Stärke aus Wildtyppflanzen zusammen:

**Tabelle 2:**

| Angabe der charakteristischen Werte der RVA-Analyse von Stärke, isoliert aus Wildtyppflanzen (cv. Desiree), Pflanzen mit einer verringerten Aktivität eines SSIII- und eines BEI-Proteins (038VL008, 038VL107), bzw. von Pflanzen mit einer verringerten Aktivität eines SSIII- und eines BEI- und eines BEII-Proteins (108CF041, 110CF003) in Prozent, bezogen auf Werte von Stärke des Wildtyps. Die RVA Analyse wurde nach Analysemethode 1 durchgeführt. | | | | | | |
|---|---|---|---|---|---|---|
| RVA-Analysemethode 1 | | | | | | |
| | RVA Max (%) | RVA Min (%) | RVA Fin (%) | RVA Set (%) | RVA T (%) | Gelfestigkeit |
| cv.Desiree | 100 | 100 | 100 | 100 | 100 | 100 |
| 038VL008 | 158.7 | 69.8 | 72.0 | 79.5 | 73.0 | 55.4 |
| 108CF041 | 59.6 | 89.9 | 227.5 | 693.7 | 150.2 | 532.3 |
| 038VL107 | 151.1 | 94.3 | 94.0 | 93.0 | 82.2 | 52.2 |
| 110CF003 | 106.4 | 158.6 | 265.0 | 625.7 | 151.5 | 737.1 |

**Tabelle 3:**

| Angabe der charakteristischen Werte der RVA-Analyse von Stärke, isoliert aus Wildtyppflanzen (cv. Desiree), Pflanzen mit einer verringerten Aktivität eines SSIII- und eines BEI-Proteins (038VL008, 038VL107), bzw. von Pflanzen mit einer verringerten Aktivität eines SSIII- und eines BEI- und eines BEII- (108CF041, 110CF003) in Prozent, bezogen auf Werte von Stärke des Wildtyps. Die RVA Analyse wurde nach Analysemethode 2 durchgeführt. | | | | | | |
|---|---|---|---|---|---|---|
| RVA-Analysemethode 2 | | | | | | |
| | RVA Max (%) | RVA Min (%) | RVA Fin (%) | RVA Set (%) | RVA T (%) | Gelfestigkeit |
| cv. Desiree | 100 | 100 | 100 | 100 | 100 | 100 |
| 038VL008 | 167.1 | 40.4 | 52.6 | 77.6 | 54.2 | 63.0 |
| 108CF041 | 44.5 | 82.5 | 187.5 | 402.7 | 137.4 | 412.2 |
| 038VL107 | 152.0 | 76.1 | 81.9 | 93.8 | 76.9 | 51.7 |
| 110CF003 | 92.4 | 172.2 | n.d. | n.d. | 139.0 | 795.0 |

**Tabelle 4:**

| Angabe der charakteristischen Werte der RVA-Analyse von Stärke, isoliert aus Wildtyppflanzen (cv. Desiree), Pflanzen mit einer verringerten Aktivität eines SSIII- und eines BEI-Proteins (038VL008, 038VL107), bzw. von Pflanzen mit einer verringerten Aktivität eines SSIII- und eines BEI- und eines BEII-Proteins (108CF041, 110CF003) in Prozent, bezogen auf Werte von Stärke des Wildtyps. Die RVA Analyse wurde nach Analysemethode 3 durchgeführt. | | | | | | |
|---|---|---|---|---|---|---|
| RVA-Analysemethode 3 | | | | | | |
| | RVA Max (%) | RVA Min (%) | RVA Fin (%) | RVA Set (%) | RVA T (%) | Gelfestigkeit |
| cv. Desiree | 100 | 100 | 100 | 100 | 100 | 100 |
| 038VL008 | n.d. | 50.2 | 76.5 | 127.8 | 77.0 | 100.5 |
| 108CF041 | 74.7 | 291.0 | n.d. | 205.7 | 236.0 | 630.3 |
| 038VL107 | n.d. | 84.5 | 86.4 | 90.1 | 102.3 | 58.1 |
| 110CF003 | 89.8 | 259.7 | n.d. | n.d. | 196.6 | 663.9 |

Die folgende Tabellen 5,6 und 7 fassen die Ergebnisse der RVA Analyse zusammen. Die Werte beziehen sich nicht auf den Wildtyp, sondern sind die gemessenen Werte:

**Tabelle 5:**

| Angabe der charakteristischen Werte der RVA-Analyse von Stärke, isoliert aus Wildtyppflanzen (cv. Desiree), Pflanzen mit einer verringerten Aktivität eines SSIII- und eines BEI-Proteins (038VL008, 038VL107), bzw. von Pflanzen mit einer verringerten Aktivität eines SSIII- und eines BEI- und eines BEII-Proteins (108CF041, 110CF003) in RVUs. Die RVA Analyse wurde nach Analysemethode 1 durchgeführt. | | | | | | |
|---|---|---|---|---|---|---|
| RVA-Analysemethode 1 (siehe auch Fig 1) | | | | | | |
| | RVA Max (RVU) | RVA Min (RVU) | RVA Fin (RVU) | RVA Set (RVU) | RVA T (RVU) | Gelfestigkeit |
| cv. Desiree | 255.05 | 162.33 | 210.25 | 47.92 | 4.6 | 25.1 |
| 038VL008 | 404.83 | 113.25 | 151.33 | 38.08 | 3.36 | 13.9 |
| 108CF041 | 152.08 | 145.92 | 478.33 | 332.42 | 6.91 | 133.6 |
| 038VL107 | 385.5 | 153 | 197.58 | 44.58 | 3.78 | 13.1 |
| 110CF003 | 271.5 | 257.42 | 557.25 | 299.83 | 6.97 | 185 |

**Tabelle 6:**

| Angabe der charakteristische Werte der RVA-Analyse von Stärke, isoliert aus Wildtyppflanzen (cv. Desiree), Pflanzen mit einer verringerten Aktivität eines SSIII- und eines BEI-Proteins (038VL008, 038VL107), bzw. von Pflanzen mit einer verringerten Aktivität eines SSIII- und eines BEI- und eines BEII-Proteins (108CF041, 110CF003) in RVUs. Die RVA Analyse wurde nach Analysemethode 2 durchgeführt. | | | | | | |
|---|---|---|---|---|---|---|
| RVA-Analysemethode 2 (siehe auch Fig 2) | | | | | | |
| | RVA Max (RVU) | RVA Min (RVU) | RVA Fin (RVU) | RVA Set (RVU) | RVA T (RVU) | Gelfestigkeit |
| cv. Desiree | 212.17 | 113.75 | 169.25 | 55.5 | 28.78 | 23.8 |
| 038VL008 | 354.58 | 45.92 | 89 | 43.08 | 15.61 | 15 |
| 108CF041 | 94.33 | 93.83 | 317.33 | 223.5 | 39.53 | 98.1 |
| 038VL107 | 322.58 | 86.58 | 138.67 | 52.08 | 22.13 | 12.3 |
| 110CF003 | 196.08 | 195.92 | n.d. | n.d. | 39.99 | 189.2 |

**Tabelle 7:**

| Angabe der charakteristische Werte der RVA-Analyse von Stärke, isoliert aus Wildtyppflanzen (cv. Desiree), Pflanzen mit einer verringerten Aktivität eines SSIII- und eines BEI-Proteins (038VL008, 038VL107), bzw. von Pflanzen mit einer verringerten Aktivität eines SSIII-Proteins und eines BEI-Proteins und eines BEII-Proteins (108CF041, 110CF003) in RVUs. Die RVA Analyse wurde nach Analysemethode 3 durchgeführt. | | | | | | |
|---|---|---|---|---|---|---|
| RVA-Analysemethode 3 (siehe auch Fig 3) | | | | | | |
| | RVA Max (RVU) | RVA Min (RVU) | RVA Fin (RVU) | RVA Set (RVU) | RVA T (RVU) | Gelfestigkeit |
| Desiree | 819.67 | 207.67 | 314.25 | 106.58 | 16.88 | 56.5 |
| 038VL008 | n.d. | 104.17 | 240.33 | 136.17 | 12.99 | 56.8 |
| 108CF041 | 612.33 | 604.25 | 823.5 | 219.25 | 39.83 | 356.1 |
| 038VL107 | n.d. | 175.42 | 271.5 | 96.08 | 17.27 | 32.8 |
| 110CF003 | 736.08 | 539.42 | n.d. | n.d. | 33.18 | 375.1 |

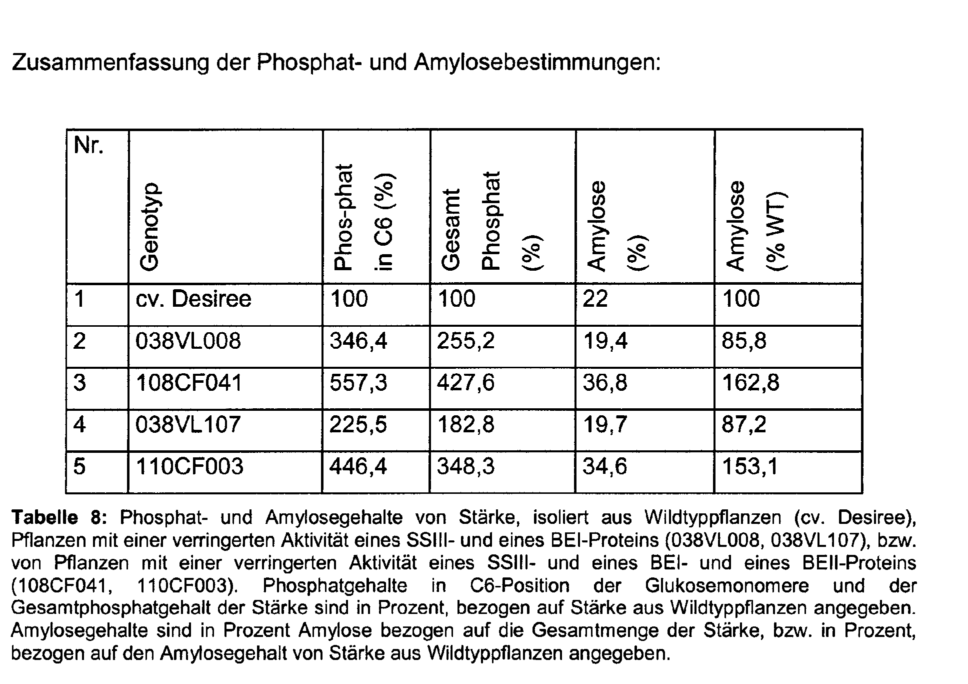

Die Analyse der Seitenkettenverteilung des Amylopektins wurde wie oben beschrieben durchgeführt. Die folgende Tabelle enthält eine Übersicht über die Anteile der einzelnen Peakflächen:

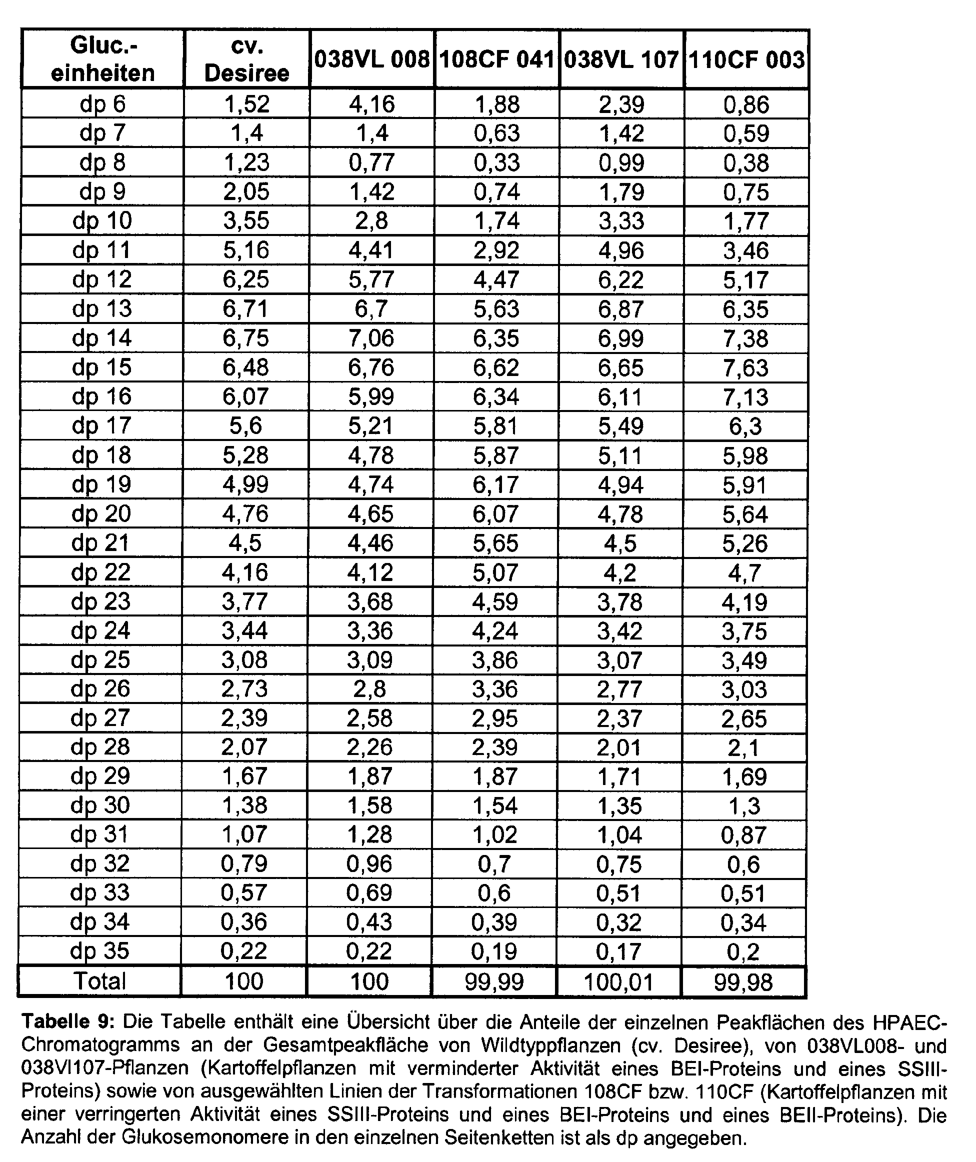

Die sogenannte "Peak Chain Length", deren Wert sich aus der Bildung des Mittelwertes der beiden Kettenlängen (angegeben in DP) ergibt, die den größten Anteil an der Gesamtfläche unter den Peaks des HPAEC Chromatogramms ausmachen, liegt bei entzweigtem Amylopektin von Wildtyppflanzen bei DP= 13 bei 038VL-Pflanzen ebenfalls bei DP= 13 und bei den 108CF bzw. 110CF-Pflanzen im Durchschnitt bei 15.

Setzt man die Peak Chain Length der transgenen Pflanzen ins Verhältnis zur Peak Chain Length von Amylopektin von Wildtyppflanzen, so erhält man folgende Werte für das Peak Chain Length- Verhältnis (PCL-Verhältnis):
PCL-Verhältnis für 038VL = 13 / 13 = 1
PCL-Verhältnis für 108/110CF = 15 / 13 = 1,15

Darüberhinaus wurde die Morphologie der Stärkekörner durch Rasterelektronenmikroskopie (REM) untersucht:

Die Oberfläche der Stärkekörner von 108/110CF-Pflanzen erscheint belegt oder aufgerauht mit Porenbildung.

Ferner wurde die Korngrößenbestimmung mit einem Fotosedimentometer des Typs "Lumosed" der Firma Retsch GmbH, Deutschland durchgeführt.
Die mittlere Korngröße wurde von unbehandelten Stärke-Proben bestimmt. (Tab. 3).

**Tabelle 10:**

| Werte der mittleren Korngröße von Stärke, isoliert aus Wildtyppflanzen (cv. Desiree), Pflanzen mit einer verringerten Aktivität eines SSIII- und eines BEI-Proteins (038VL008, 038VL107), bzw. von Pflanzen mit einer verringerten Aktivität eines SSIII- und eines BEI- und eines BEII-Proteins (108CF041, 110CF003). | |
|---|---|
| mittlere Korngröße [µm] | |
| Probe | mittlere Korngröße |
| cv. Desiree | 29,7 |
| 038VL008 | 21,5 |
| 108CF041 | 20,8 |
| 038VL107 | 22,9 |
| 110CF003 | 20,7 |

## Patentansprüche

1. Pflanzenzelle, die genetisch modifiziert ist, wobei die genetische Modifikation zur Verringerung der Aktivität eines oder mehrerer endogen in der Pflanzenzelle vorkommenden SSIII-Proteine und zur Verringerung der Aktivität eines oder mehrerer endogen in der Pflanzenzelle vorkommenden BEI-Proteine und zur Verringerung der Aktivität eines oder mehrerer endogen in der Pflanzenzelle vorkommenden BEII-Proteine führt im Vergleich zu entsprechenden nicht genetisch modifizierten Pflanzenzellen von Wildtyppflanzen.

2. Pflanzenzelle nach Anspruch 1, wobei die genetische Modifikation in der Einführung eines oder mehrerer fremder Nukleinsäuremoleküle besteht, deren Vorhandensein und/oder Expression zur Verringerung der Aktivität von einem oder mehreren in der Pflanzenzelle vorkommenden SSIII-und BEI- und BEII-Proteinen führt im Vergleich zu entsprechenden nicht genetisch modifizierten Pflanzenzellen von Wildtyppflanzen.

3. Transgene Pflanzenzellen nach Anspruch 2, worin besagte fremde Nukleinsäuremoleküle ausgewählt sind aus der Gruppe bestehend aus
a) DNA-Molekülen, die mindestens eine antisense-RNA codieren, welche eine Verringerung der Expression von mindestens einem endogenen Gen bewirkt, das SSIII-Protein(e) und/oder BEI-Protein(e) und/oder BEII-Protein(e) codiert;
b) DNA-Molekülen, die über einen Cosuppressionseffekt zu Verringerung der Expression von mindestens einem endogenen Gen führen, das SSIII-Protein(e) und/oder BEI-Protein(e) und/oder BEII-Protein(e) codiert;
c) DNA-Molekülen, die mindestens ein Ribozym codieren, das spezifisch Transkripte von mindestens einem endogenen Gen spaltet, das SSIII-Protein(e) und/oder BEI-Protein(e) und/oder BEII-Protein(e) codiert; und
d) Mittels in vivo-Mutagenese eingeführte Nukleinsäuremoleküle, die zu einer Mutation oder einer Insertion einer heterologen Sequenz in mindestens einem endogenen SSIII-Protein(e) und/oder BEI-Protein(e) und/oder BEII-Protein(e) codierenden Gen führen, wobei die Mutation oder Insertion eine Verringerung der Expression von mindestens einem SSIII-Protein(e) und/oder BEI-Protein(e) und/oder BEII-Protein(e) codierenden Gen bewirkt, oder die Synthese von inaktiven SSIII- und/oder BEIund/oder BEII-Proteinen zur Folge hat;
e) DNA-Molekülen, die simultan mindestens eine antisense-RNA und mindestens eine sense-RNA codieren, wobei besagte antisense-RNA und besagte sense-RNA ein doppelsträngiges RNA-Molekül ausbilden, das eine Verringerung der Expression von mindestens einem endogenen Gen bewirkt, das SSIII-Protein(e) und/oder BEI-Protein(e) und/oder BEII-Protein(e) codiert;
f) DNA Molekülen, die Transposons enthalten, wobei die Integration der Transposonsequenzen zu einer Mutation oder einer Insertion in mindestens einem endogenen SSIII-Protein(e) und/oder BEI-Protein(e) und/oder BEII-Protein(e) codierenden Gen führt, welche eine Verringerung der Expression von mindestens einem SSIII-Protein(e) und/oder BEI-Protein(e) und/oder BEII-Protein(e) codierenden Gen bewirkt, oder die Synthese von inaktiven SSIII- und/oder BEI- und/oder BEII-Proteinen zur Folge hat; und/oder
g) T-DNA Moleküle, die durch Insertion in mindestens einem endogenen SSIII-Protein(e) und/oder BEI-Protein(e) und/oder BEII-Protein(e) codierenden Gen eine Verringerung der Expression von mindestens einem SSIII-Protein(e) und/oder BEI-Protein(e) und/oder BEII-Protein(e) codierenden Gen bewirken, oder die Synthese von inaktiven SSIII- und/oder BEI- und/oder BEII-Proteinen zur Folge haben.

4. Pflanzenzelle nach einem der Ansprüche 1 bis 3, die eine modifizierte Stärke im Vergleich zu einer nicht genetisch modifizierten Wildtyp-Pflanzenzelle synthetisiert.

5. Pflanzenzelle nach Anspruch 4, wobei die modifizierte Stärke **dadurch gekennzeichnet ist, dass** sie
a) einen Amylosegehalt von mindestens 30% enthält,
b) in Vergleich zu Stärke aus entsprechenden nicht genetisch modifizierten Wildtyp-Pflanzenzellen einen erhöhten Phosphatgehalt aufweist und
c) in der RVA Analyse im Vergleich zu nicht genetisch modifizierten Wildtyp-Pflanzenzellen eine erhöhte Endviskosität aufweist.

6. Pflanzenzelle nach Anspruch 4 oder 5, wobei die modifizierte Stärke **dadurch gekennzeichnet ist, dass** sie im Vergleich zur Stärke aus nicht genetisch modifizierten Wildtyp-Pflanzenzellen eine erhöhte Gelfestigkeit aufweist.

7. Pflanzenzelle nach einem der Ansprüche 4 bis 6, wobei die modifizierte Stärke **dadurch gekennzeichnet ist, dass** sie im Vergleich zur Stärke aus nicht genetisch modifizierten Wildtyp-Pflanzenzellen eine veränderte Seitenkettenverteilung und/oder eine veränderte Stärkekornmorphologie aufweist.

8. Pflanze enthaltend Pflanzenzellen nach einem der Ansprüche 1 bis 7.

9. Verfahren zur Herstellung einer Pflanzenzelle, die eine modifizierte Stärke synthetisiert, umfassend die genetische Modifikation der Pflanzenzelle, wobei die genetische Modifikation zur Verringerung der Aktivität eines oder mehrerer endogen in der Pflanzenzelle vorkommenden SSIII-Proteine und zur Verringerung der Aktivität eines oder mehrerer endogen in der Pflanzenzelle vorkommenden BEI-Proteine und zur Verringerung der Aktivität eines oder mehrerer endogen in der Pflanzenzelle vorkommenden BEII-Proteine führt im Vergleich zu entsprechenden nicht genetisch modifizierten Pflanzenzellen von Wildtyp-Pflanzen.

10. Verfahren zur Herstellung einer Pflanzenzelle gemäß Anspruch 9, die eine modifizierte Stärke synthetisiert, worin die pflanzliche Zelle genetisch modifiziert wird durch die Einführung eines oder mehrerer fremder Nukleinsäuremoleküle, deren Vorhandensein und/oder Expression zur Verringerung der Aktivität von jeweils mindestens einem SSIII-, BEI- und BEII-Protein führt im Vergleich zu entsprechenden nicht genetisch modifizierten Wildtyp-Pflanzenzellen.

11. Verfahren nach Anspruch 9 oder 10, worin die modifizierte Stärke **dadurch gekennzeichnet ist, dass** sie
a) einen Amylosegehalt von mindestens 30% enthält,
b) im Vergleich zu Stärke aus entsprechenden nicht genetisch modifizierten Wildtyp-Pflanzenzellen einen erhöhten Phosphatgehalt aufweist und
c) in der RVA Analyse im Vergleich zu Stärke aus entsprechenden nicht genetisch modifizierten Wildtyp-Pflanzenzellen eine erhöhte Endviskosität aufweist.

12. Verfahren zur Herstellung einer genetisch modifizierten Pflanze, wobei
a) eine Pflanzenzelle gemäß einem der Ansprüche 9 bis 11 hergestellt wird;
b) aus oder mit der gemäß a) hergestellten Pflanzenzelle eine Pflanze regeneriert wird; und
c) gegebenenfalls aus der gemäß Schritt b) erzeugten Pflanze weitere Pflanzen erzeugt werden.

13. Verfahren zur Herstellung einer transgenen Pflanze gemäß Anspruch 12, die eine modifizierte Stärke synthetisiert, worin
a) eine pflanzliche Zelle genetisch modifiziert wird durch die Einführung eines oder mehrerer fremder Nukleinsäuremoleküle, deren Vorhandensein und/oder Expression zur Verringerung der Aktivität von jeweils mindestens einem SSIII-, BEI- und BEII-Protein führt im Vergleich zu entsprechenden nicht genetisch modifizierten Wildtyp-Pflanzenzellen;
b) aus oder mit der gemäß a) hergestellten Zelle eine Pflanze regeneriert wird; und
c) aus der gemäß Schritt b) erzeugten Pflanzen gegebenenfalls weitere Pflanzen erzeugt werden.

14. Verfahren nach Anspruch 12 oder 13, worin die modifizierte Stärke **dadurch gekennzeichnet ist, dass** sie
a) einen Amylosegehalt von mindestens 30% enthält und
b) im Vergleich zu Stärke aus entsprechenden nicht genetisch modifizierten Wildtyp Pflanzen einen erhöhten Phosphatgehalt aufweist und
c) im Vergleich zu Stärke aus entsprechenden nicht genetisch modifizierten Wildtyp Pflanzen eine erhöhte Endviskosität in der RVA Analyse aufweist.

15. Pflanze nach Anspruch 8 oder erhältlich nach dem Verfahren gemäß einem der Ansprüche 12 bis 14, die eine stärkespeichernde Pflanze ist.

16. Pflanze nach Anspruch 15, die eine Kartoffelpflanze ist.

17. Vermehrungsmaterial von Pflanzen nach einem der Ansprüche 8 oder 15 bis 16, enthaltend mindestens eine Pflanzenzelle nach einem der Ansprüche 1 bis 7.

18. Verwendung eines oder mehrerer Nukleinsäuremoleküle, die Proteine mit der enzymatischen Aktivität von mindestens einem SSIII-, mindestens einem BEI, und/oder mindestens einem BEII-Protein oder deren Fragmente codieren, zur Herstellung von Pflanzenzellen nach einem der Ansprüche 1 bis 7 oder von Pflanzen nach einem der Ansprüche 8 oder 15 bis 16.

19. Verwendung eines oder mehrerer Nukleinsäuremoleküle zur Herstellung von Pflanzenzellen nach einem der Ansprüche 1 bis 7 oder von Pflanzen nach einem der Ansprüche 8 oder 15 bis 16, wobei das fremde Nukleinsäuremolekül oder die fremden Nukleinsäuremoleküle ausgewählt sind aus der Gruppe bestehend aus :
a) DNA-Molekülen, die mindestens eine antisense-RNA codieren, welche eine Verringerung der Expression von mindestens einem endogenen Gen bewirkt, das SSIII-Protein(e) und/oder BEI-Protein(e) und /oder BEII-Protein(e) codiert;
b) DNA-Moleküle, die über einen Cosuppressionseffekt zu Verringerung der Expression von mindestens einem endogenen Gen führen, das SSIII-Protein(e) und/oder BEI-Protein(e) und/oder BEII-Protein(e) codiert;
c) DNA-Moleküle, die mindestens ein Ribozym codieren, das spezifisch Transkripte von mindestens einem endogenen Gen spaltet, das SSIII-Protein(e) und/oder BEI-Protein(e) und/oder BEII-Protein(e) codiert;
d) Mittels in vivo Mutagenese eingeführte Nukleinsäuremoleküle, die zu einer Mutation oder Insertion einer heterologen Sequenz in mindestens einem endogenen SSIII- Protein(e) und/oder BEI-Protein(e) und/oder BEII-Protein(e) codierenden Gen führen, wobei die Mutation oder Insertion eine Verringerung der Expression von mindestens einem SSIII-Protein(e) und/oder BEI-Protein(e) und/oder BEII-Protein(e) codierenden Gen bewirkt, oder die Synthese von inaktiven SSIII-Proteinen und/oder BEI-Proteinen und/oder BEII-Proteinen zur Folge hat;
e) DNA-Moleküle, die simultan mindestens eine antisense-RNA und mindestens eine sense-RNA codieren, wobei besagte antisense-RNA und besagte sense-RNA ein doppelsträngiges RNA-Molekül ausbilden, das eine Verringerung der Expression von mindestens einem endogenen Gen bewirkt, das SSIII-Proteine und/oder BEI-Proteine und/oder BEII-Proteine codiert;
f) DNA Molekülen, die Transposons enthalten, wobei die Integration der Transposonsequenzen zu einer Mutation oder einer Insertion in mindestens einem endogenen SSIII-Protein(e) und/oder BEI-Protein(e) und/oder BEII-Protein(e) codierenden Gen führt, welche eine Verringerung der Expression von mindestens einem SSIII-Protein(e) und/oder BEI-Protein(e) und/oder BEII-Protein(e) codierenden Gen bewirkt, oder die Synthese von inaktiven SSIII- und/oder BEI- und/oder BEII-Proteinen zur Folge haben; und
g) T-DNA Moleküle, die durch Insertion in mindestens einem endogenen SSIII-Proteine und/oder BEI-Proteine und/oder BEII-Proteine codierenden Gen eine Verringerung der Expression von mindestens einem SSIII-Protein(e) und/oder BEI-Protein(e) und/oder BEII-Protein(e) codierenden Gen bewirken, oder die Synthese von inaktiven SSIII- und/oder BEI- und/oder BEII-Proteinen zur Folge haben.

20. Zusammensetzung, enthaltend mindestens eines der in Anspruch 19 definierten Nukleinsäuremoleküle, wobei das mindestens eine Nukleinsäuremolekül nach Einführung in eine Pflanzenzelle zur Verringerung mindestens eines endogen in der Pflanzenzelle vorkommenden SSIII-Proteins und mindestens eines endogen in der Pflanzenzelle vorkommenden BEII-Proteins, und vorzugsweise weiterhin zur Verringerung mindestens eines endogen in der Pflanzenzelle vorkommenden BEI-Proteins führt.

21. Zusammensetzung nach Anspruch 20, **dadurch gekennzeichnet, dass** das Vorhandensein des mindestens einen Nukleinsäuremoleküls in den besagten Pflanzenzellen zur Verringerung der Aktivität von jeweils mindestens einem SSIII- und BEI und BEII-Protein führt im Vergleich zu entsprechenden nicht genetisch modifizierten Wildtyp-Pflanzenzellen.

22. Zusammensetzung nach einem der Ansprüche 20 bis 21, wobei die Nukleinsäuremoleküle in einem rekombinanten Nukleinsäuremolekül enthalten sind.

23. Verwendung einer Zusammensetzung nach einem der Ansprüche 20 bis 22 oder enthaltend mindestens eines der in Anspruch 19 definierten Nukleinsäuremoleküle zur Herstellung einer Pflanzenzelle mit verringerter Aktivität eines oder mehrerer endogen in der Pflanzenzelle vorkommenden SSIII-Proteine und verringerter Aktivität eines oder mehrerer endogen in der Pflanzenzelle vorkommenden BEI-Proteine und verringerter Aktivität eines oder mehrerer endogen in der Pflanzenzelle vorkommenden BEII-Proteine, im Vergleich zu entsprechenden nicht genetisch modifizierten Pflanzenzellen von Wildtyppflanzen.

24. Transformationssystem für Pflanzenzellen, enthaltend mindestens ein Nukleinsäuremolekül und mindestens eine Pflanzenzelle, wobei das mindestens eine Nukleinsäuremolekül zur Verringerung der Aktivität jeweils mindestens eines der endogen in der Pflanzenzelle vorkommenden SSIII-, BEI- und BE-II-Proteine führt, soweit diese nicht bereits durch eine vorherige genetische Modifikation der besagten Pflanzenzelle in ihrer Aktivität verringert wurden.

25. Wirtszelle, enthaltend eine Zusammensetzung nach einem der Ansprüche 20 bis 22.

26. Wirtszelle nach Anspruch 25, die eine transgene Pflanzenzelle enthaltend die Zusammensetzung nach einem der Ansprüche 20 bis 22 ist.

27. Stärke, erhältlich aus Pflanzenzellen nach einem der Ansprüche 1 bis 7 oder 26 oder aus einer Pflanze nach einem der Ansprüche 8 oder 15 bis 16 oder aus Vermehrungsmaterial nach Anspruch 17.

28. Stärke nach Anspruch 27, **dadurch gekennzeichnet, dass** sie
a) einen Amylosegehalt von mindestens 30% enthält,
b) in Vergleich zu Stärke aus entsprechenden nicht genetisch modifizierten Wildtyp-Pflanzenzellen einen erhöhten Phosphatgehalt aufweist und
c) in der RVA Analyse im Vergleich zu nicht genetisch modifizierten Wildtyp-Pflanzenzellen eine erhöhte Endviskosität aufweist.

29. Stärke nach Anspruch 27 oder 28, **dadurch gekennzeichnet, dass** sie im Vergleich zu Stärke aus entsprechenden nicht genetisch modifizierten Wildtyp-Pflanzenzellen eine veränderte Seitenkettenverteilung aufweist.

30. Stärke nach einem der Ansprüche 27 bis 29, **dadurch gekennzeichnet, dass** die Stärkekörner in Vergleich zu nicht genetisch modifizierten Wildtyp-Pflanzenzellen eine veränderte Stärkekornmorphologie und/oder eine veränderte mittlere Stärkekorngröße aufweisen.

31. Stärke nach einem der Ansprüche 27 bis 30, die eine Kartoffelstärke ist.

32. Verfahren zur Herstellung einer Stärke nach einem der Ansprüche 27 bis 31, umfassend die Extraktion der Stärke aus einer Pflanze nach einem der Ansprüche 8 oder 15 bis 16 und/oder aus stärkespeichernden Teilen einer solchen Pflanze und/oder aus einer Pflanzenzelle nach einem der Ansprüche 1 bis 7 oder 26 und/oder aus Vermehrungsmaterial nach Anspruch 17.

33. Stärke nach einem der Ansprüche 27 bis 31, erhältlich durch ein Verfahren nach Anspruch 32.

34. Stärke nach einem der Ansprüche 27 bis 31 oder 33, **dadurch gekennzeichnet, dass** sie mindestens eine der folgenden Eigenschaften aufweist:
a) eine Endviskosität in der RVA-Analyse mit einer 6 %igen (w/w) wässrigen Stärkesuspension von mindestens 300 RVU, bevorzugt von mindestens 400 RVU, insbesondere von mindestens 500 RVU;
b) in der C6-Position der Glucosemonomere der Stärke einen Phosphatgehalt von 40 bis 120 nmol, bevorzugt von 60 bis 100 nmol, und insbesondere von 80 bis 100 nmol C6-P pro mg Stärke;
c) in nativer Form eine poröse Oberfläche der Stärkekörner.

35. Verarbeitungsprodukt, insbesondere Nahrungs- oder Futtermittel, Farbe, Klebstoff, Bau- oder Dämmstoff, enthaltend eine Stärke gemäß einem der Ansprüche 27 bis 31, 33 oder 34.

36. Stärkehaltiger Teil einer Pflanze gemäß einem der Ansprüche 8, 15 oder 16.

37. Verfahren zur Modifikation der Stärke einer Pflanze, umfassend das Verfahren zur Herstellung einer Pflanze gemäß einem der Ansprüche 12 bis 14 sowie die Gewinnung von Stärke aus der Pflanze oder stärkehaltigen Teilen davon.

38. Verfahren gemäß Anspruch 37, **dadurch gekennzeichnet, dass** die Modifikation der Stärke umfasst:
a) die Erhöhung des Amylosegehaltes der Stärke;
b) die Erhöhung des Phosphatgehaltes der Stärke, insbesondere auf einen Phosphatgehalt in der C6-Position der Glucosemonomere der Stärke von 40 bis 120 nmol, bevorzugt von 60 bis 100 nmol, und insbesondere von 80 bis 100 nmol C6-P pro mg Stärke;
c) die Erhöhung der Endviskosität der Stärke, insbesondere auf eine Endviskosität in der RVA-Analyse mit einer 6 %igen (w/w) wässrigen Stärkesuspension von mindestens 300 RVU, bevorzugt von mindestens 400 RVU, insbesondere von mindestens 500 RVU;
d) die Erhöhung der Gelfestigkeit der gelatinisierten Stärke und/oder
e) die Morphologie, insbesondere die Oberflächenstruktur, Porosität und/oder Korngrößenverteilung der nativen Stärkekörner.


SEQUENCE LISTING

<110> Bayer CropScience GmbH

<120> Pflanzen, die eine Stärke mit erhöhter Endviskosität
synthetisieren

<130> BCS 02 5002

<160> 8

<170> PatentIn version 3.1

<210> 1
<211> 4167
<212> DNA
<213> Solanum tuberosum

<220>
<221> CDS
<222> (207)..(3899)
<223>

<300>
<301> Abel,G.J., Springer,F., Willmitzer,L. and Kossmann,J.
<302> Cloning and functional analysis of a cDNA encoding a novel
139 kDa

<303>  Plant J.

<304>  10

<305>  6

<306>  981-991

<307>  1996

<308>  X94400

<309>  1995-12-22

<313>  (1)..(4167)


<300>

<308>  EMBL / X94400

<309>  1997-04-16

<313>  (1)..(4167)


<400>  1
ttttttaata gatttttaaa accccattaa agcaaatacg tatataattg cagcacagat
   60

acagagaggg agagagaaag atagtgtgtt gatgaaggag aagagagata tttcacatgg
  120

gatgttctat ttgattctgt ggtgaacaag agttttacaa agaacattcc tttttctttt
  180

tttcttggtt cttgtgtggg tcagcc atg gat gtt cca ttt cca ctg cat aga
  233
                             Met Asp Val Pro Phe Pro Leu His Arg
                             1               5

cca ttg agt tgc aca agt gtc tcc aat gca ata acc cac ctc aag atc
  281
Pro Leu Ser Cys Thr Ser Val Ser Asn Ala Ile Thr His Leu Lys Ile

10                  15                  20                  25

aaa cct ttt ctt ggg ttt gtc tct cat gga acc aca agt cta tca gta
329
Lys Pro Phe Leu Gly Phe Val Ser His Gly Thr Thr Ser Leu Ser Val
                30                  35                  40

caa tct tct tca tgg agg aag gat gga atg gtt act ggg gtt tca ttt
377
Gln Ser Ser Ser Trp Arg Lys Asp Gly Met Val Thr Gly Val Ser Phe
            45                  50                  55

cca ttt tgt gca aat ctc tcg gga aga aga cgg aga aaa gtt tca act
425
Pro Phe Cys Ala Asn Leu Ser Gly Arg Arg Arg Arg Lys Val Ser Thr
        60                  65                  70

act agg agt caa gga tct tca cct aag ggg ttt gtg cca agg aag ccc
473
Thr Arg Ser Gln Gly Ser Ser Pro Lys Gly Phe Val Pro Arg Lys Pro
    75                  80                  85

tca ggg atg agc acg caa aga aag gtt cag aag agc aat ggt gat aaa
521
Ser Gly Met Ser Thr Gln Arg Lys Val Gln Lys Ser Asn Gly Asp Lys
90                  95                  100                 105

gaa agt caa agt act tca aca tct aaa gaa tct gaa att tcc aac cag
569
Glu Ser Gln Ser Thr Ser Thr Ser Lys Glu Ser Glu Ile Ser Asn Gln
                110                 115                 120

aag acg gtt gaa gca aga gtt gaa act agt gac gat gac act aaa gta
617
Lys Thr Val Glu Ala Arg Val Glu Thr Ser Asp Asp Asp Thr Lys Val
            125                 130                 135

gtg gtg agg gac cac aag ttt ctg gag gat gag gat gaa atc aat ggt
665
Val Val Arg Asp His Lys Phe Leu Glu Asp Glu Asp Glu Ile Asn Gly

        140                 145                 150

tct act aaa tca ata agt atg tca cct gtt cgt gta tca tct caa ttt
713
Ser Thr Lys Ser Ile Ser Met Ser Pro Val Arg Val Ser Ser Gln Phe

    155                 160                 165

gtt gaa agt gaa gaa act ggt ggt gat gac aag gat gct gta aag tta
761
Val Glu Ser Glu Glu Thr Gly Gly Asp Asp Lys Asp Ala Val Lys Leu

170                 175                 180                 185

aac aaa tca aag aga tcg gaa gag agt gat ttt cta att gat tct gta
809
Asn Lys Ser Lys Arg Ser Glu Glu Ser Asp Phe Leu Ile Asp Ser Val

                190                 195                 200

ata aga gaa caa agt gga tct cag ggg gaa act aat gcc agt agc aag
857
Ile Arg Glu Gln Ser Gly Ser Gln Gly Glu Thr Asn Ala Ser Ser Lys

            205                 210                 215

gga agc cat gct gtg ggt aca aaa ctt tat gag ata ttg cag gtg gat
905
Gly Ser His Ala Val Gly Thr Lys Leu Tyr Glu Ile Leu Gln Val Asp

        220                 225                 230

gtt gag cca caa caa ttg aaa gaa aat aat gct ggg aat gtt gaa tac
953
Val Glu Pro Gln Gln Leu Lys Glu Asn Asn Ala Gly Asn Val Glu Tyr

    235                 240                 245

aaa gga cct gta gca agt aag cta ttg gaa att act aag gct agt gat
1001
Lys Gly Pro Val Ala Ser Lys Leu Leu Glu Ile Thr Lys Ala Ser Asp

250                 255                 260                 265

gtg gaa cac act gaa agc aat gag att gat gac tta gac act aat agt
1049
Val Glu His Thr Glu Ser Asn Glu Ile Asp Asp Leu Asp Thr Asn Ser
                270                 275                 280

ttc ttt aaa tca gat tta att gaa gag gat gag cca tta gct gca gga
1097
Phe Phe Lys Ser Asp Leu Ile Glu Glu Asp Glu Pro Leu Ala Ala Gly
            285                 290                 295

aca gtg gag act gga gat tct tct cta aac tta aga ttg gag atg gaa
1145
Thr Val Glu Thr Gly Asp Ser Ser Leu Asn Leu Arg Leu Glu Met Glu
        300                 305                 310

gca aat cta cgt agg cag gct ata gaa agg ctt gcc gag gaa aat tta
1193
Ala Asn Leu Arg Arg Gln Ala Ile Glu Arg Leu Ala Glu Glu Asn Leu
    315                 320                 325

ttg caa ggg atc aga tta ttt tgt ttt cca gag gtt gta aaa cct gat
1241
Leu Gln Gly Ile Arg Leu Phe Cys Phe Pro Glu Val Val Lys Pro Asp
330                 335                 340                 345

gaa gat gtc gag ata ttt ctt aac aga ggt ctt tcc act ttg aag aat
1289
Glu Asp Val Glu Ile Phe Leu Asn Arg Gly Leu Ser Thr Leu Lys Asn
                350                 355                 360

gag tct gat gtc ttg att atg gga gct ttt aat gag tgg cgc tat agg
1337
Glu Ser Asp Val Leu Ile Met Gly Ala Phe Asn Glu Trp Arg Tyr Arg
            365                 370                 375

tct ttt act aca agg cta act gag act cat ctc aat gga gat tgg tgg
1385

Ser Phe Thr Thr Arg Leu Thr Glu Thr His Leu Asn Gly Asp Trp Trp

        380                 385                 390

tct tgc aag atc cat gtt ccc aag gaa gca tac agg gct gat ttt gtg
1433
Ser Cys Lys Ile His Val Pro Lys Glu Ala Tyr Arg Ala Asp Phe Val

    395                 400                 405

ttt ttt aat gga caa gat gtc tat gac aac aat gat gga aat gac ttc
1481
Phe Phe Asn Gly Gln Asp Val Tyr Asp Asn Asn Asp Gly Asn Asp Phe

410                 415                 420                 425

agt ata act gtg aaa ggt ggt atg caa atc att gac ttt gaa aat ttc
1529
Ser Ile Thr Val Lys Gly Gly Met Gln Ile Ile Asp Phe Glu Asn Phe

                430                 435                 440

ttg ctt gag gag aaa tgg aga gaa cag gag aaa ctt gct aaa gaa caa
1577
Leu Leu Glu Glu Lys Trp Arg Glu Gln Glu Lys Leu Ala Lys Glu Gln

            445                 450                 455

gct gaa aga gaa aga cta gcg gaa gaa caa aga cga ata gaa gca gag
1625
Ala Glu Arg Glu Arg Leu Ala Glu Glu Gln Arg Arg Ile Glu Ala Glu

        460                 465                 470

aaa gct gaa att gaa gct gac aga gca caa gca aag gaa gag gct gca
1673
Lys Ala Glu Ile Glu Ala Asp Arg Ala Gln Ala Lys Glu Glu Ala Ala

    475                 480                 485

aag aaa aag aaa gta ttg cga gaa ttg atg gta aaa gcc acg aag act
1721
Lys Lys Lys Lys Val Leu Arg Glu Leu Met Val Lys Ala Thr Lys Thr

490                 495                 500                 505

cgt gat atc acg tgg tac ata gag cca agt gaa ttt aaa tgc gag gac
1769
Arg Asp Ile Thr Trp Tyr Ile Glu Pro Ser Glu Phe Lys Cys Glu Asp

                510                 515                 520

aag gtc agg tta tac tat aac aaa agt tca ggt cct ctc tcc cat gct
1817
Lys Val Arg Leu Tyr Tyr Asn Lys Ser Ser Gly Pro Leu Ser His Ala

            525                 530                 535

aag gac ttg tgg atc cac gga gga tat aat aat tgg aag gat ggt ttg
1865
Lys Asp Leu Trp Ile His Gly Gly Tyr Asn Asn Trp Lys Asp Gly Leu

        540                 545                 550

tct att gtc aaa aag ctt gtt aaa tct gag aga ata gat ggt gat tgg
1913
Ser Ile Val Lys Lys Leu Val Lys Ser Glu Arg Ile Asp Gly Asp Trp

    555                 560                 565

tgg tat aca gag gtt gtt att cct gat cag gca ctt ttc ttg gat tgg
1961
Trp Tyr Thr Glu Val Val Ile Pro Asp Gln Ala Leu Phe Leu Asp Trp

570                 575                 580                 585

gtt ttt gct gat ggt cca ccc aag cat gcc att gct tat gat aac aat
2009
Val Phe Ala Asp Gly Pro Pro Lys His Ala Ile Ala Tyr Asp Asn Asn

                590                 595                 600

cac cgc caa gac ttc cat gcc att gtc ccc aac cac att ccg gag gaa
2057
His Arg Gln Asp Phe His Ala Ile Val Pro Asn His Ile Pro Glu Glu

            605                 610                 615

tta tat tgg gtt gag gaa gaa cat cag atc ttt aag aca ctt cag gag

2105
Leu Tyr Trp Val Glu Glu Glu His Gln Ile Phe Lys Thr Leu Gln Glu

        620                 625                 630

gag aga agg ctt aga gaa gcg gct atg cgt gct aag gtt gaa aaa aca
2153
Glu Arg Arg Leu Arg Glu Ala Ala Met Arg Ala Lys Val Glu Lys Thr

    635                 640                 645

gca ctt ctg aaa act gaa aca aag gaa aga act atg aaa tca ttt tta
2201
Ala Leu Leu Lys Thr Glu Thr Lys Glu Arg Thr Met Lys Ser Phe Leu

650                 655                 660                 665

ctg tct cag aag cat gta gta tat act gag cct ctt gat atc caa gct
2249
Leu Ser Gln Lys His Val Val Tyr Thr Glu Pro Leu Asp Ile Gln Ala

                670                 675                 680

gga agc agc gtc aca gtt tac tat aat ccc gcc aat aca gta ctt aat
2297
Gly Ser Ser Val Thr Val Tyr Tyr Asn Pro Ala Asn Thr Val Leu Asn

            685                 690                 695

ggt aaa cct gaa att tgg ttc aga tgt tca ttt aat cgc tgg act cac
2345
Gly Lys Pro Glu Ile Trp Phe Arg Cys Ser Phe Asn Arg Trp Thr His

        700                 705                 710

cgc ctg ggt cca ttg cca cct cag aaa atg tcg cct gct gaa aat ggc
2393
Arg Leu Gly Pro Leu Pro Pro Gln Lys Met Ser Pro Ala Glu Asn Gly

    715                 720                 725

acc cat gtc aga gca act gtg aag gtt cca ttg gat gca tat atg atg
2441
Thr His Val Arg Ala Thr Val Lys Val Pro Leu Asp Ala Tyr Met Met

730                 735                 740                 745

gat ttt gta ttt tcc gag aga gaa gat ggt ggg att ttt gac aat aag
2489
Asp Phe Val Phe Ser Glu Arg Glu Asp Gly Gly Ile Phe Asp Asn Lys

                750                 755                 760

agc gga atg gac tat cac ata cct gtg ttt gga gga gtc gct aaa gaa
2537
Ser Gly Met Asp Tyr His Ile Pro Val Phe Gly Gly Val Ala Lys Glu

            765                 770                 775

cct cca atg cat att gtc cat att gct gtc gaa atg gca cca att gca
2585
Pro Pro Met His Ile Val His Ile Ala Val Glu Met Ala Pro Ile Ala

        780                 785                 790

aag gtg gga ggc ctt ggt gat gtt gtt act agt ctt tcc cgt gct gtt
2633
Lys Val Gly Gly Leu Gly Asp Val Val Thr Ser Leu Ser Arg Ala Val

    795                 800                 805

caa gat tta aac cat aat gtg gat att atc tta cct aag tat gac tgt
2681
Gln Asp Leu Asn His Asn Val Asp Ile Ile Leu Pro Lys Tyr Asp Cys

810                 815                 820                 825

ttg aag atg aat aat gtg aag gac ttt cgg ttt cac aaa aac tac ttt
2729
Leu Lys Met Asn Asn Val Lys Asp Phe Arg Phe His Lys Asn Tyr Phe

                830                 835                 840

tgg ggt ggg act gaa ata aaa gta tgg ttt gga aag gtg gaa ggt ctc
2777
Trp Gly Gly Thr Glu Ile Lys Val Trp Phe Gly Lys Val Glu Gly Leu

            845                 850                 855

tcg gtc tat ttt ttg gag cct caa aac ggg tta ttt tcg aaa ggg tgc
2825
Ser Val Tyr Phe Leu Glu Pro Gln Asn Gly Leu Phe Ser Lys Gly Cys

        860                 865                 870

gtc tat ggt tgt agc aat gat ggt gaa cga ttt ggt ttc ttc tgt cac
2873
Val Tyr Gly Cys Ser Asn Asp Gly Glu Arg Phe Gly Phe Phe Cys His

    875                 880                 885

gcg gct ttg gag ttt ctt ctg caa ggt gga ttt agt ccg gat atc att
2921
Ala Ala Leu Glu Phe Leu Leu Gln Gly Gly Phe Ser Pro Asp Ile Ile

890                 895                 900                 905

cat tgc cat gat tgg tct agt gct cct gtt gct tgg ctc ttt aag gaa
2969
His Cys His Asp Trp Ser Ser Ala Pro Val Ala Trp Leu Phe Lys Glu

                910                 915                 920

caa tat aca cac tat ggt cta agc aaa tct cgt ata gtc ttc acg ata
3017
Gln Tyr Thr His Tyr Gly Leu Ser Lys Ser Arg Ile Val Phe Thr Ile

            925                 930                 935

cat aat ctt gaa ttt ggg gca gat ctc att ggg aga gca atg act aac
3065
His Asn Leu Glu Phe Gly Ala Asp Leu Ile Gly Arg Ala Met Thr Asn

        940                 945                 950

gca gac aaa gct aca aca gtt tca cca act tac tca cag gag gtg tct
3113
Ala Asp Lys Ala Thr Thr Val Ser Pro Thr Tyr Ser Gln Glu Val Ser

    955                 960                 965

gga aac cct gta att gcg cct cac ctt cac aag ttc cat ggt ata gtg
3161
Gly Asn Pro Val Ile Ala Pro His Leu His Lys Phe His Gly Ile Val

970                 975                 980                 985

aat ggg att gac cca gat att tgg gat cct tta aac gat aag ttc att
3209
Asn Gly Ile Asp Pro Asp Ile Trp Asp Pro Leu Asn Asp Lys Phe Ile

                990                 995                 1000

ccg att ccg tac acc tca gaa aac gtt gtt gaa ggc aaa aca gca
3254
Pro Ile Pro Tyr Thr Ser Glu Asn Val Val Glu Gly Lys Thr Ala

            1005                1010                1015

gcc aag gaa gct ttg cag cga aaa ctt gga ctg aaa cag gct gac
3299
Ala Lys Glu Ala Leu Gln Arg Lys Leu Gly Leu Lys Gln Ala Asp

            1020                1025                1030

ctt cct ttg gta gga att atc acc cgc tta act cac cag aaa gga
3344
Leu Pro Leu Val Gly Ile Ile Thr Arg Leu Thr His Gln Lys Gly

            1035                1040                1045

atc cac ctc att aaa cat gct att tgg cgc acc ttg gaa cgg aac
3389
Ile His Leu Ile Lys His Ala Ile Trp Arg Thr Leu Glu Arg Asn

            1050                1055                1060

gga cag gta gtc ttg ctt ggt tct gct cct gat cct agg gta caa
3434
Gly Gln Val Val Leu Leu Gly Ser Ala Pro Asp Pro Arg Val Gln

            1065                1070                1075

aac gat ttt gtt aat ttg gca aat caa ttg cac tcc aaa tat aat
3479
Asn Asp Phe Val Asn Leu Ala Asn Gln Leu His Ser Lys Tyr Asn

            1080                1085                1090

gac cgc gca cga  ctc tgt cta aca tat  gac gag cca ctt tct  cac
 3524
Asp Arg Ala Arg  Leu Cys Leu Thr Tyr  Asp Glu Pro Leu Ser  His

              1095                  1100                  1105

ctg ata tat gct  ggt gct gat ttt att  cta gtt cct tca ata  ttt
 3569
Leu Ile Tyr Ala  Gly Ala Asp Phe Ile  Leu Val Pro Ser Ile  Phe

              1110                  1115                  1120

gag cca tgt gga  cta aca caa ctt acc  gct atg aga tat ggt  tca
 3614
Glu Pro Cys Gly  Leu Thr Gln Leu Thr  Ala Met Arg Tyr Gly  Ser

              1125                  1130                  1135

att cca gtc gtg  cgt aaa act gga gga  ctt tat gat act gta  ttt
 3659
Ile Pro Val Val  Arg Lys Thr Gly Gly  Leu Tyr Asp Thr Val  Phe

              1140                  1145                  1150

gat gtt gac cat  gac aaa gag aga gca  caa cag tgt ggt ctt  gaa
 3704
Asp Val Asp His  Asp Lys Glu Arg Ala  Gln Gln Cys Gly Leu  Glu

              1155                  1160                  1165

cca aat gga ttc  agc ttt gat gga gca  gat gct ggc gga gtt  gat
 3749
Pro Asn Gly Phe  Ser Phe Asp Gly Ala  Asp Ala Gly Gly Val  Asp

              1170                  1175                  1180

tat gct ctg aat  aga gct ctc tct gct  tgg tac gat ggt cgg  gat
 3794
Tyr Ala Leu Asn  Arg Ala Leu Ser Ala  Trp Tyr Asp Gly Arg  Asp

              1185                  1190                  1195

tgg ttc aac tct  tta tgc aag cag gtc  atg gaa caa gat tgg  tct
 3839

Trp Phe Asn Ser Leu Cys Lys Gln Val Met Glu Gln Asp Trp Ser
            1200                1205                1210

tgg aac cga cct gct ctt gat tat ttg gag ctt tac cat gct gct
3884
Trp Asn Arg Pro Ala Leu Asp Tyr Leu Glu Leu Tyr His Ala Ala
            1215                1220                1225

aga aag tta gaa tag ttagtttgtg agatgctagc agaaaaattc acgagatctg
3939
Arg Lys Leu Glu
            1230

caatctgtac aggttcagtg tttgcgtctg gacagctttt ttatttccta tatcaaagta
3999

taaatcaagt ctacactgag atcaatagca gacagtcctc agttcatttc attttttgtg
4059

caacatatga aagagcttag cctctaataa tgtagtcatt gatgattatt tgttttggga
4119

agaaatgaga aatcaaagga tgcaaaatac tctgaaaaaa aaaaaaaa
4167

<210> 2

<211> 1230

<212> PRT

<213> Solanum tuberosum

<400> 2
   Met Asp Val Pro Phe Pro Leu His Arg Pro Leu Ser Cys Thr Ser Val
   1 5 10 15
   Ser Asn Ala Ile Thr His Leu Lys Ile Lys Pro Phe Leu Gly Phe Val
   20 25 30
   Ser His Gly Thr Thr Ser Leu Ser Val Gln Ser Ser Ser Trp Arg Lys
           35                  40                  45
   Asp Gly Met Val Thr Gly Val Ser Phe Pro Phe Cys Ala Asn Leu Ser
       50                  55                  60
   Gly Arg Arg Arg Arg Lys Val Ser Thr Thr Arg Ser Gln Gly Ser Ser
   65                  70                  75                  80
   Pro Lys Gly Phe Val Pro Arg Lys Pro Ser Gly Met Ser Thr Gln Arg
                   85                  90                  95
   Lys Val Gln Lys Ser Asn Gly Asp Lys Glu Ser Gln Ser Thr Ser Thr
               100                 105                 110
   Ser Lys Glu Ser Glu Ile Ser Asn Gln Lys Thr Val Glu Ala Arg Val
           115                 120                 125
   Glu Thr Ser Asp Asp Asp Thr Lys Val Val Val Arg Asp His Lys Phe
       130                 135                 140
   Leu Glu Asp Glu Asp Glu Ile Asn Gly Ser Thr Lys Ser Ile Ser Met
   145                 150                 155                 160
   Ser Pro Val Arg Val Ser Ser Gln Phe Val Glu Ser Glu Glu Thr Gly
                   165                 170                 175
   Gly Asp Asp Lys Asp Ala Val Lys Leu Asn Lys Ser Lys Arg Ser Glu
               180                 185                 190
   Glu Ser Asp Phe Leu Ile Asp Ser Val Ile Arg Glu Gln Ser Gly Ser
           195                 200                 205
   Gln Gly Glu Thr Asn Ala Ser Ser Lys Gly Ser His Ala Val Gly Thr
       210                 215                 220
   Lys Leu Tyr Glu Ile Leu Gln Val Asp Val Glu Pro Gln Gln Leu Lys
   225                 230                 235                 240
   Glu Asn Asn Ala Gly Asn Val Glu Tyr Lys Gly Pro Val Ala Ser Lys
   245 250 255
   Leu Leu Glu Ile Thr Lys Ala Ser Asp Val Glu His Thr Glu Ser Asn
   260 265 270
   Glu Ile Asp Asp Leu Asp Thr Asn Ser Phe Phe Lys Ser Asp Leu Ile
   275 280 285
   Glu Glu Asp Glu Pro Leu Ala Ala Gly Thr Val Glu Thr Gly Asp Ser
   290 295 300
   Ser Leu Asn Leu Arg Leu Glu Met Glu Ala Asn Leu Arg Arg Gln Ala
   305 310 315 320
   Ile Glu Arg Leu Ala Glu Glu Asn Leu Leu Gln Gly Ile Arg Leu Phe
   325 330 335
   Cys Phe Pro Glu Val Val Lys Pro Asp Glu Asp Val Glu Ile Phe Leu
   340 345 350
   Asn Arg Gly Leu Ser Thr Leu Lys Asn Glu Ser Asp Val Leu Ile Met
   355 360 365
   Gly Ala Phe Asn Glu Trp Arg Tyr Arg Ser Phe Thr Thr Arg Leu Thr
   370 375 380
   Glu Thr His Leu Asn Gly Asp Trp Trp Ser Cys Lys Ile His Val Pro
   385 390 395 400
   Lys Glu Ala Tyr Arg Ala Asp Phe Val Phe Phe Asn Gly Gln Asp Val
   405 410 415
   Tyr Asp Asn Asn Asp Gly Asn Asp Phe Ser Ile Thr Val Lys Gly Gly
   420 425 430
   Met Gln Ile Ile Asp Phe Glu Asn Phe Leu Leu Glu Glu Lys Trp Arg
   435 440 445
   Glu Gln Glu Lys Leu Ala Lys Glu Gln Ala Glu Arg Glu Arg Leu Ala
       450                 455                 460
   Glu Glu Gln Arg Arg Ile Glu Ala Glu Lys Ala Glu Ile Glu Ala Asp
   465                 470                 475                 480
   Arg Ala Gln Ala Lys Glu Glu Ala Ala Lys Lys Lys Lys Val Leu Arg
                   485                 490                 495
   Glu Leu Met Val Lys Ala Thr Lys Thr Arg Asp Ile Thr Trp Tyr Ile
               500                 505                 510
   Glu Pro Ser Glu Phe Lys Cys Glu Asp Lys Val Arg Leu Tyr Tyr Asn
           515                 520                 525
   Lys Ser Ser Gly Pro Leu Ser His Ala Lys Asp Leu Trp Ile His Gly
       530                 535                 540
   Gly Tyr Asn Asn Trp Lys Asp Gly Leu Ser Ile Val Lys Lys Leu Val
   545                 550                 555                 560
   Lys Ser Glu Arg Ile Asp Gly Asp Trp Trp Tyr Thr Glu Val Val Ile
                   565                 570                 575
   Pro Asp Gln Ala Leu Phe Leu Asp Trp Val Phe Ala Asp Gly Pro Pro
               580                 585                 590
   Lys His Ala Ile Ala Tyr Asp Asn Asn His Arg Gln Asp Phe His Ala
           595                 600                 605
   Ile Val Pro Asn His Ile Pro Glu Glu Leu Tyr Trp Val Glu Glu Glu
       610                 615                 620
   His Gln Ile Phe Lys Thr Leu Gln Glu Glu Arg Arg Leu Arg Glu Ala
   625                 630                 635                 640
   Ala Met Arg Ala Lys Val Glu Lys Thr Ala Leu Leu Lys Thr Glu Thr
                   645                 650                 655
   Lys Glu Arg Thr Met Lys Ser Phe Leu Leu Ser Gln Lys His Val Val
   660 665 670
   Tyr Thr Glu Pro Leu Asp Ile Gln Ala Gly Ser Ser Val Thr Val Tyr
   675 680 685
   Tyr Asn Pro Ala Asn Thr Val Leu Asn Gly Lys Pro Glu Ile Trp Phe
   690 695 700
   Arg Cys Ser Phe Asn Arg Trp Thr His Arg Leu Gly Pro Leu Pro Pro
   705 710 715 720
   Gln Lys Met Ser Pro Ala Glu Asn Gly Thr His Val Arg Ala Thr Val
   725 730 735
   Lys Val Pro Leu Asp Ala Tyr Met Met Asp Phe Val Phe Ser Glu Arg
   740 745 750
   Glu Asp Gly Gly Ile Phe Asp Asn Lys Ser Gly Met Asp Tyr His Ile
   755 760 765
   Pro Val Phe Gly Gly Val Ala Lys Glu Pro Pro Met His Ile Val His
   770 775 780
   Ile Ala Val Glu Met Ala Pro Ile Ala Lys Val Gly Gly Leu Gly Asp
   785 790 795 800
   Val Val Thr Ser Leu Ser Arg Ala Val Gln Asp Leu Asn His Asn Val
   805 810 815
   Asp Ile Ile Leu Pro Lys Tyr Asp Cys Leu Lys Met Asn Asn Val Lys
   820 825 830
   Asp Phe Arg Phe His Lys Asn Tyr Phe Trp Gly Gly Thr Glu Ile Lys
   835 840 845
   Val Trp Phe Gly Lys Val Glu Gly Leu Ser Val Tyr Phe Leu Glu Pro
   850 855 860
   Gln Asn Gly Leu Phe Ser Lys Gly Cys Val Tyr Gly Cys Ser Asn Asp
   865                 870                 875                 880
   Gly Glu Arg Phe Gly Phe Phe Cys His Ala Ala Leu Glu Phe Leu Leu
                   885                 890                 895
   Gln Gly Gly Phe Ser Pro Asp Ile Ile His Cys His Asp Trp Ser Ser
               900                 905                 910
   Ala Pro Val Ala Trp Leu Phe Lys Glu Gln Tyr Thr His Tyr Gly Leu
           915                 920                 925
   Ser Lys Ser Arg Ile Val Phe Thr Ile His Asn Leu Glu Phe Gly Ala
       930                 935                 940
   Asp Leu Ile Gly Arg Ala Met Thr Asn Ala Asp Lys Ala Thr Thr Val
   945                 950                 955                 960
   Ser Pro Thr Tyr Ser Gln Glu Val Ser Gly Asn Pro Val Ile Ala Pro
                   965                 970                 975
   His Leu His Lys Phe His Gly Ile Val Asn Gly Ile Asp Pro Asp Ile
               980                 985                 990
   Trp Asp Pro Leu Asn Asp Lys Phe  Ile Pro Ile Pro Tyr  Thr Ser Glu
           995                 1000                 1005
   Asn Val  Val Glu Gly Lys Thr  Ala Ala Lys Glu Ala  Leu Gln Arg
       1010                 1015                 1020
   Lys Leu  Gly Leu Lys Gln Ala  Asp Leu Pro Leu Val  Gly Ile Ile
       1025                 1030                 1035
   Thr Arg  Leu Thr His Gln Lys  Gly Ile His Leu Ile  Lys His Ala
       1040                 1045                 1050
   Ile Trp  Arg Thr Leu Glu Arg  Asn Gly Gln Val Val  Leu Leu Gly
       1055                 1060                 1065
   Ser Ala Pro Asp Pro Arg Val Gln Asn Asp Phe Val Asn Leu Ala
   1070 1075 1080
   Asn Gln Leu His Ser Lys Tyr Asn Asp Arg Ala Arg Leu Cys Leu
   1085 1090 1095
   Thr Tyr Asp Glu Pro Leu Ser His Leu Ile Tyr Ala Gly Ala Asp
   1100 1105 1110
   Phe Ile Leu Val Pro Ser Ile Phe Glu Pro Cys Gly Leu Thr Gln
   1115 1120 1125
   Leu Thr Ala Met Arg Tyr Gly Ser Ile Pro Val Val Arg Lys Thr
   1130 1135 1140
   Gly Gly Leu Tyr Asp Thr Val Phe Asp Val Asp His Asp Lys Glu
   1145 1150 1155
   Arg Ala Gln Gln Cys Gly Leu Glu Pro Asn Gly Phe Ser Phe Asp
   1160 1165 1170
   Gly Ala Asp Ala Gly Gly Val Asp Tyr Ala Leu Asn Arg Ala Leu
   1175 1180 1185
   Ser Ala Trp Tyr Asp Gly Arg Asp Trp Phe Asn Ser Leu Cys Lys
   1190 1195 1200
   Gln Val Met Glu Gln Asp Trp Ser Trp Asn Arg Pro Ala Leu Asp
   1205 1210 1215
   Tyr Leu Glu Leu Tyr His Ala Ala Arg Lys Leu Glu
   1220 1225 1230

<210> 3

<211> 61

<212> PRT

<213> Solanum tuberosum

<400> 3

Arg Ser Phe Thr Thr Arg Leu Thr Glu Thr His Leu Asn Gly Asp Trp
1               5                   10                  15

Trp Ser Cys Lys Ile His Val Pro Lys Glu Ala Tyr Arg Ala Asp Phe
            20                  25                  30

Val Phe Phe Asn Gly Gln Asp Val Tyr Asp Asn Asn Asp Gly Asn Asp
        35                  40                  45

Phe Ser Ile Thr Val Lys Gly Gly Met Gln Ile Ile Asp
    50                  55                  60

<210> 4

<211> 1641

<212> DNA

<213> Solanum tuberosum

<400> 4
atgaagcaca gttcagctat ttccgctgtt ttgaccgatg acaattcgac aatggcaccc
   60

ctagaggaag atgtcaacac tgaaaatatt ggcctcctaa atttggatcc aactttggaa
  120

ccttatctag atcacttcag acacagaatg aagagatatg tggatcagaa aatgctcatt
  180

gaaaaatatg agggacccct tgaggaattt gctcaaggtt atttaaaatt tggattcaac
  240

agggaagatg gttgcatagt ctatcgtgaa tgggctcctg ctgctcagga agcagaagtt
  300

attggcgatt tcaatggtag gaacggttct aaccacatga tggagaagga ccagtttggt
  360

gtttggagta ttagaattcc tgatgttgac agtaagccag tcattccaca caactccaga
  420

gttaagtttc gtttcaaaca tggtaatgga gtgtgggtag atcgtatccc tgcttggata
480

aagtatgcca ctgcagacgc cacaaagttt gcagcaccat atgatggtgt ctactgggac
540

ccaccacctt cagaaaggta ccacttcaaa taccctcgcc ctcccaaacc ccgagcccca
600

cgaatctacg aagcacatgt cggcatgagc agctctgagc cacgtgtaaa ttcgtatcgt
660

gagtttgcag atgatgtttt acctcggatt aaggcaaata actataatac tgtccagttg
720

atggccataa tggaacattc ttactatgga tcatttggat atcatgttac aaactttttt
780

gctgtgagca atagatatgg aaacccggag gacctaaagt atctgataga taaagcacat
840

agcttgggtt tacaggttct ggtggatgta gttcacagtc atgcaagcaa taatgtcact
900

gatggcctca atggctttga tattggccaa ggttctcaag aatcctactt tcatgctgga
960

gagcgagggt accataagtt gtgggatagc aggctgttca actatgccaa ttgggaggtt
1020

cttcgtttcc ttctttccaa cttgaggtgg tggctagaag agtataactt tgacggattt
1080

cgatttgatg gaataacttc tatgctgtat gttcatcatg gaatcaatat gggatttaca
1140

ggaaactata atgagtattt cagcgaggct acagatgttg atgctgtggt ctatttaatg
1200

ttggccaata atctgattca caagattttc ccagacgcaa ctgttattgc cgaagatgtt
1260

tctggtatgc cgggccttag ccggcctgtt tctgagggag gaattggttt tgattaccgc
1320

ctggcaatgg caatcccaga taagtggata gattatttaa agaataagaa tgatgaagat
1380

tggtccatga aggaagtaac atcgagtttg acaaatagga gatatacaga gaagtgtata
1440

gcatatgcgg agagccatga tcagtctatt gtcggtgaca agaccattgc atttctccta

1500

atgaacaaag agatgtattc tggcatgtct tgcttgacag atgcttctcc tgttgttgat
1560

gcaggaattg cgcttgacaa gatgatccat ttttttcaca atggccttgg gaggagaggg
1620

gtacctcaat ttcatgggta a
1641

<210> 5

<211> 546

<212> PRT

<213> Solanum tuberosum

<300>

<308> Swiss Prot / P30924

<309> 1993-07-26

<400> 5

Met Lys His Ser Ser Ala Ile Ser Ala Val Leu Thr Asp Asp Asn Ser
1               5                   10                  15

Thr Met Ala Pro Leu Glu Glu Asp Val Asn Thr Glu Asn Ile Gly Leu
            20                  25                  30

Leu Asn Leu Asp Pro Thr Leu Glu Pro Tyr Leu Asp His Phe Arg His
        35                  40                  45

Arg Met Lys Arg Tyr Val Asp Gln Lys Met Leu Ile Glu Lys Tyr Glu
    50                  55                  60

Gly Pro Leu Glu Glu Phe Ala Gln Gly Tyr Leu Lys Phe Gly Phe Asn
65                  70                  75                  80

Arg Glu Asp Gly Cys Ile Val Tyr Arg Glu Trp Ala Pro Ala Ala Gln
                85                  90                  95

Glu Ala Glu Val Ile Gly Asp Phe Asn Gly Arg Asn Gly Ser Asn His
            100                 105                 110

Met Met Glu Lys Asp Gln Phe Gly Val Trp Ser Ile Arg Ile Pro Asp
        115                 120                 125

Val Asp Ser Lys Pro Val Ile Pro His Asn Ser Arg Val Lys Phe Arg
    130                 135                 140

Phe Lys His Gly Asn Gly Val Trp Val Asp Arg Ile Pro Ala Trp Ile
145                 150                 155                 160

Lys Tyr Ala Thr Ala Asp Ala Thr Lys Phe Ala Ala Pro Tyr Asp Gly
                165                 170                 175

Val Tyr Trp Asp Pro Pro Pro Ser Glu Arg Tyr His Phe Lys Tyr Pro
            180                 185                 190

Arg Pro Pro Lys Pro Arg Ala Pro Arg Ile Tyr Glu Ala His Val Gly
        195                 200                 205

Met Ser Ser Ser Glu Pro Arg Val Asn Ser Tyr Arg Glu Phe Ala Asp
    210                 215                 220

Asp Val Leu Pro Arg Ile Lys Ala Asn Asn Tyr Asn Thr Val Gln Leu
225                 230                 235                 240

Met Ala Ile Met Glu His Ser Tyr Tyr Gly Ser Phe Gly Tyr His Val
                245                 250                 255

Thr Asn Phe Phe Ala Val Ser Asn Arg Tyr Gly Asn Pro Glu Asp Leu
            260                 265                 270

Lys Tyr Leu Ile Asp Lys Ala His Ser Leu Gly Leu Gln Val Leu Val
        275                 280                 285

Asp Val Val His Ser His Ala Ser Asn Asn Val Thr Asp Gly Leu Asn
    290                 295                 300

Gly Phe Asp Ile Gly Gln Gly Ser Gln Glu Ser Tyr Phe His Ala Gly
305                 310                 315                 320

Glu Arg Gly Tyr His Lys Leu Trp Asp Ser Arg Leu Phe Asn Tyr Ala
                325                 330                 335

Asn Trp Glu Val Leu Arg Phe Leu Leu Ser Asn Leu Arg Trp Trp Leu
            340                 345                 350

Glu Glu Tyr Asn Phe Asp Gly Phe Arg Phe Asp Gly Ile Thr Ser Met
        355                 360                 365

Leu Tyr Val His His Gly Ile Asn Met Gly Phe Thr Gly Asn Tyr Asn
    370                 375                 380

Glu Tyr Phe Ser Glu Ala Thr Asp Val Asp Ala Val Val Tyr Leu Met
385                 390                 395                 400

Leu Ala Asn Asn Leu Ile His Lys Ile Phe Pro Asp Ala Thr Val Ile
                405                 410                 415

Ala Glu Asp Val Ser Gly Met Pro Gly Leu Ser Arg Pro Val Ser Glu
            420                 425                 430

Gly Gly Ile Gly Phe Asp Tyr Arg Leu Ala Met Ala Ile Pro Asp Lys
        435                 440                 445

Trp Ile Asp Tyr Leu Lys Asn Lys Asn Asp Glu Asp Trp Ser Met Lys
    450                 455                 460

Glu Val Thr Ser Ser Leu Thr Asn Arg Arg Tyr Thr Glu Lys Cys Ile
465                 470                 475                 480

Ala Tyr Ala Glu Ser His Asp Gln Ser Ile Val Gly Asp Lys Thr Ile
                485                 490                 495

Ala Phe Leu Leu Met Asn Lys Glu Met Tyr Ser Gly Met Ser Cys Leu
            500                 505                 510

Thr Asp Ala Ser Pro Val Val Asp Ala Gly Ile Ala Leu Asp Lys Met
        515                 520                 525

Ile His Phe Phe His Asn Gly Leu Gly Arg Arg Gly Val Pro Gln Phe
    530                 535                 540

His Gly
545

<210>  6

<211>  2649

<212>  DNA

<213>  Solanum tuberosum

<300>

<308>  EMBL / AJ011890

<309>  1999-04-07

<300>

<302>  Improvments in or relating to plant starch composition

<308>  EMBL / A58164

<309>  1998-03-05

<310>  WO 96 34968

<311>  1996-05-03

<312>  1996-11-07

<400>  6
atggtgtata cactctctgg agttcgtttt cctactgttc catcagtgta caaatctaat

60

ggattcagca gtaatggtga tcggaggaat gctaatgttt ctgtattctt gaaaaagcac
120

tctctttcac ggaagatctt ggctgaaaag tcttcttaca attccgaatt ccgaccttct
180

acagttgcag catcggggaa agtccttgtg cctggaaccc agagtgatag ctcctcatcc
240

tcaacagacc aatttgagtt cactgagaca tctccagaaa attccccagc atcaactgat
300

gtagatagtt caacaatgga acacgctagc cagattaaaa ctgagaacga tgacgttgag
360

ccgtcaagtg atcttacagg aagtgttgaa gagctggatt ttgcttcatc actacaacta
420

caagaaggtg gtaaactgga ggagtctaaa acattaaata cttctgaaga gacaattatt
480

gatgaatctg ataggatcag agagaggggc atccctccac ctggacttgg tcagaagatt
540

tatgaaatag accccctttt gacaaactat cgtcaacacc ttgattacag gtattcacag
600

tacaagaaac tgagggaggc aattgacaag tatgagggtg gtttggaagc cttttctcgt
660

ggttatgaaa aaatgggttt cactcgtagt gctacaggta tcacttaccg tgagtgggct
720

cttggtgccc agtcagctgc cctcattgga gatttcaaca attgggacgc aaatgctgac
780

attatgactc ggaatgaatt tggtgtctgg gagatttttc tgccaaataa tgtggatggt
840

tctcctgcaa ttcctcatgg gtccagagtg aagatacgta tggacactcc atcaggtgtt
900

aaggattcca ttcctgcttg gatcaactac tctttacagc ttcctgatga aattccatat
960

aatggaatac attatgatcc acccgaagag gagaggtata tcttccaaca cccacggcca
1020

aagaaaccaa agtcgctgag aatatatgaa tctcatattg gaatgagtag tccggagcct
1080

aaaattaact catacgtgaa ttttagagat gaagttcttc ctcgcataaa aaagcttggg
1140

tacaatgcgc tgcaaattat ggctattcaa gagcattctt attacgctag ttttggttat
1200

catgtcacaa atttttttgc accaagcagc cgttttggaa cgcccgacga ccttaagtct
1260

ttgattgata aagctcatga gctaggaatt gttgttctca tggacattgt tcacagccat
1320

gcatcaaata atactttaga tggactgaac atgtttgact gcaccgatag ttgttacttt
1380

cactctggag ctcgtggtta tcattggatg tgggattccc gcctctttaa ctatggaaac
1440

tgggaggtac ttaggtatct tctctcaaat gcgagatggt ggttggatgc gttcaaattt
1500

gatggattta gatttgatgg tgtgacatca atgatgtata ttcaccacgg attatcggtg
1560

ggattcactg ggaactacga ggaatacttt ggactcgcaa ctgatgtgga tgctgttgtg
1620

tatctgatgc tggtcaacga tcttattcat gggcttttcc cagatgcaat taccattggt
1680

gaagatgtta gcggaatgcc gacattttgt attcccgtcc aagagggggg tgttggcttt
1740

gactatcggc tgcatatggc aattgctgat aaacggattg agttgctcaa gaaacgggat
1800

gaggattgga gagtgggtga tattgttcat acactgacaa atagaagatg gtcggaaaag
1860

tgtgtttcat acgctgaaag tcatgatcaa gctctagtcg gtgataaaac tatagcattc
1920

tggctgatgg acaaggatat gtatgatttt atggctctgg atagaccgtc aacatcatta
1980

atagatcgtg ggatagcatt gcacaagatg attaggcttg taactatggg attaggagga
2040

gaagggtacc taaatttcat gggaaatgaa ttcggccacc ctgagtggat tgatttccct
2100

agggctgaac aacacctctc tgatggctca gtaatccccg gaaaccaatt ccgttatgat
2160

aaatgcagac ggagatttga cctgggagat gcagaatatt taagataccg tgggttgcaa
2220

gaatttgacc ggcctatgca gtatcttgaa gataaatatg agtttatgac ttcagaacac
2280

cagttcatat cacgaaagga tgaaggagat aggatgattg tatttgaaaa aggaaaccta
2340

gtttttgtct ttaattttca ctggacaaaa agctattcag actatcgcat agcctgcctg
2400

aagcctggaa aatacccggt tgccttggac tcagatgatc cactttttgg tggcttcggg
2460

agaattgatc ataatgccga atatttcacc tttgaaggat ggtatgatga tcgtcctcgt
2520

tcaattatgg tgtatgcacc ttgtaaaaca gcagtggtct atgcactagt agacaaagaa
2580

gaagaagaag aagaagaaga agaagaagaa gtagcagcag tagaagaagt agtagtagaa
2640

gaagaatga
2649

<210> 7

<211> 882

<212> PRT

<213> Solanum tuberosum

<400> 7

Met Val Tyr Thr Leu Ser Gly Val Arg Phe Pro Thr Val Pro Ser Val
1               5                   10                  15

Tyr Lys Ser Asn Gly Phe Ser Ser Asn Gly Asp Arg Arg Asn Ala Asn
            20                  25                  30

Val Ser Val Phe Leu Lys Lys His Ser Leu Ser Arg Lys Ile Leu Ala

35                  40                  45

Glu Lys Ser Ser Tyr Asn Ser Glu Phe Arg Pro Ser Thr Val Ala Ala
    50                  55                  60

Ser Gly Lys Val Leu Val Pro Gly Thr Gln Ser Asp Ser Ser Ser Ser
65                  70                  75                  80

Ser Thr Asp Gln Phe Glu Phe Thr Glu Thr Ser Pro Glu Asn Ser Pro
                85                  90                  95

Ala Ser Thr Asp Val Asp Ser Ser Thr Met Glu His Ala Ser Gln Ile
            100                 105                 110

Lys Thr Glu Asn Asp Asp Val Glu Pro Ser Ser Asp Leu Thr Gly Ser
        115                 120                 125

Val Glu Glu Leu Asp Phe Ala Ser Ser Leu Gln Leu Gln Glu Gly Gly
    130                 135                 140

Lys Leu Glu Glu Ser Lys Thr Leu Asn Thr Ser Glu Glu Thr Ile Ile
145                 150                 155                 160

Asp Glu Ser Asp Arg Ile Arg Glu Arg Gly Ile Pro Pro Pro Gly Leu
                165                 170                 175

Gly Gln Lys Ile Tyr Glu Ile Asp Pro Leu Leu Thr Asn Tyr Arg Gln
            180                 185                 190

His Leu Asp Tyr Arg Tyr Ser Gln Tyr Lys Lys Leu Arg Glu Ala Ile
        195                 200                 205

Asp Lys Tyr Glu Gly Gly Leu Glu Ala Phe Ser Arg Gly Tyr Glu Lys
    210                 215                 220

Met Gly Phe Thr Arg Ser Ala Thr Gly Ile Thr Tyr Arg Glu Trp Ala
225                 230                 235                 240

Leu Gly Ala Gln Ser Ala Ala Leu Ile Gly Asp Phe Asn Asn Trp Asp

                245                 250                 255

Ala Asn Ala Asp Ile Met Thr Arg Asn Glu Phe Gly Val Trp Glu Ile
            260                 265                 270

Phe Leu Pro Asn Asn Val Asp Gly Ser Pro Ala Ile Pro His Gly Ser
        275                 280                 285

Arg Val Lys Ile Arg Met Asp Thr Pro Ser Gly Val Lys Asp Ser Ile
    290                 295                 300

Pro Ala Trp Ile Asn Tyr Ser Leu Gln Leu Pro Asp Glu Ile Pro Tyr
305                 310                 315                 320

Asn Gly Ile His Tyr Asp Pro Pro Glu Glu Glu Arg Tyr Ile Phe Gln
                325                 330                 335

His Pro Arg Pro Lys Lys Pro Lys Ser Leu Arg Ile Tyr Glu Ser His
            340                 345                 350

Ile Gly Met Ser Ser Pro Glu Pro Lys Ile Asn Ser Tyr Val Asn Phe
        355                 360                 365

Arg Asp Glu Val Leu Pro Arg Ile Lys Lys Leu Gly Tyr Asn Ala Leu
    370                 375                 380

Gln Ile Met Ala Ile Gln Glu His Ser Tyr Tyr Ala Ser Phe Gly Tyr
385                 390                 395                 400

His Val Thr Asn Phe Phe Ala Pro Ser Ser Arg Phe Gly Thr Pro Asp
                405                 410                 415

Asp Leu Lys Ser Leu Ile Asp Lys Ala His Glu Leu Gly Ile Val Val
            420                 425                 430

Leu Met Asp Ile Val His Ser His Ala Ser Asn Asn Thr Leu Asp Gly
        435                 440                 445

Leu Asn Met Phe Asp Cys Thr Asp Ser Cys Tyr Phe His Ser Gly Ala

450                 455                 460

Arg Gly Tyr His Trp Met Trp Asp Ser Arg Leu Phe Asn Tyr Gly Asn
465                 470                 475                 480

Trp Glu Val Leu Arg Tyr Leu Leu Ser Asn Ala Arg Trp Trp Leu Asp
                485                 490                 495

Ala Phe Lys Phe Asp Gly Phe Arg Phe Asp Gly Val Thr Ser Met Met
            500                 505                 510

Tyr Ile His His Gly Leu Ser Val Gly Phe Thr Gly Asn Tyr Glu Glu
        515                 520                 525

Tyr Phe Gly Leu Ala Thr Asp Val Asp Ala Val Val Tyr Leu Met Leu
    530                 535                 540

Val Asn Asp Leu Ile His Gly Leu Phe Pro Asp Ala Ile Thr Ile Gly
545                 550                 555                 560

Glu Asp Val Ser Gly Met Pro Thr Phe Cys Ile Pro Val Gln Glu Gly
                565                 570                 575

Gly Val Gly Phe Asp Tyr Arg Leu His Met Ala Ile Ala Asp Lys Arg
            580                 585                 590

Ile Glu Leu Leu Lys Lys Arg Asp Glu Asp Trp Arg Val Gly Asp Ile
        595                 600                 605

Val His Thr Leu Thr Asn Arg Arg Trp Ser Glu Lys Cys Val Ser Tyr
    610                 615                 620

Ala Glu Ser His Asp Gln Ala Leu Val Gly Asp Lys Thr Ile Ala Phe
625                 630                 635                 640

Trp Leu Met Asp Lys Asp Met Tyr Asp Phe Met Ala Leu Asp Arg Pro
                645                 650                 655

Ser Thr Ser Leu Ile Asp Arg Gly Ile Ala Leu His Lys Met Ile Arg

660                 665                 670

Leu Val Thr Met Gly Leu Gly Gly Glu Gly Tyr Leu Asn Phe Met Gly
        675                 680                 685

Asn Glu Phe Gly His Pro Glu Trp Ile Asp Phe Pro Arg Ala Glu Gln
    690                 695                 700

His Leu Ser Asp Gly Ser Val Ile Pro Gly Asn Gln Phe Arg Tyr Asp
705                 710                 715                 720

Lys Cys Arg Arg Arg Phe Asp Leu Gly Asp Ala Glu Tyr Leu Arg Tyr
                725                 730                 735

Arg Gly Leu Gln Glu Phe Asp Arg Pro Met Gln Tyr Leu Glu Asp Lys
            740                 745                 750

Tyr Glu Phe Met Thr Ser Glu His Gln Phe Ile Ser Arg Lys Asp Glu
        755                 760                 765

Gly Asp Arg Met Ile Val Phe Glu Lys Gly Asn Leu Val Phe Val Phe
    770                 775                 780

Asn Phe His Trp Thr Lys Ser Tyr Ser Asp Tyr Arg Ile Ala Cys Leu
785                 790                 795                 800

Lys Pro Gly Lys Tyr Pro Val Ala Leu Asp Ser Asp Asp Pro Leu Phe
                805                 810                 815

Gly Gly Phe Gly Arg Ile Asp His Asn Ala Glu Tyr Phe Thr Phe Glu
            820                 825                 830

Gly Trp Tyr Asp Asp Arg Pro Arg Ser Ile Met Val Tyr Ala Pro Cys
        835                 840                 845

Lys Thr Ala Val Val Tyr Ala Leu Val Asp Lys Glu Glu Glu Glu Glu
    850                 855                 860

Glu Glu Glu Glu Glu Glu Val Ala Ala Val Glu Glu Val Val Val Glu

865                 870                 875                 880

Glu Glu

<210>  8

<211>  1255

<212>  DNA

<213>  Solanum tuberosum

<400>  8
attttgtatt cccgttcaag atgggggtgt tggctttgac tatcggctgc atatggcaat
    60

tgctgataaa tggattgagt tgctcaagaa acgggatgag gattggagag tgggtgatat
   120

tgttcataca ctgacaaata gaagatggtc ggaaaagtgt gtttcatacg ctgaaagtca
   180

tgatcaagct ctagtcggtg ataaaactat agcattctgg ctgatggaca aggatatgta
   240

tgattttatg gctttggata gaccgtcaac atcattaata gatcgtggga tagcattgca
   300

caagatgatt aggcttgtaa ctatgggatt aggaggagaa gggtacctaa atttcatggg
   360

aaatgaattc ggccaccctg agtggattga tttccctagg gctgaacaac acctctctga
   420

tggctcagta attcccggaa accaattcag ttatgataaa tgcagacgga gatttgacct
   480

gggagatgca gaatatttaa gataccgtgg gttgcaagaa tttgaccggg ctatgcagta
   540

tcttgaagat aaatatgagt ttatgacttc agaacaccag ttcatatcac gaaaggatga
   600

aggagatagg atgattgtat ttgaaaaagg aaacctagtt tttgtcttta attttcactg
   660

gacaaaaagc tattcagact atcgcatagg ctgcctgaag cctggaaaat acaaggttgc

720

cttggactca gatgatccac tttttggtgg cttcgggaga attgatcata atgccgaatg
780

tttcaccttt gaaggatggt atgatgatcg tcctcgttca attatggtgt atgcacctag
840

tagaacagca gtggtctatg cactagtaga caaagaagaa gaagaagaag aagtagcagt
900

agtagaagaa gtagtagtag aagaagaatg aacgaacttg tgatcgcgtt gaaagatttg
960

aacgctacat agagcttctt gacgtatctg gcaatattgc atcagtcttg gcggaatttc
1020

atgtgacaaa aggtttgcaa ttctttccac tattagtagt gcaacgatat acgcagagat
1080

gaagtgctga acaaacatat gtaaaatcga tgaatttatg tcgaatgctg ggacgggctt
1140

cagcaggttt tgcttagtga gttctgtaaa ttgtcatctc tttatatgta cagccaacta
1200

gaaatcaatt atgtgagacc taaaatacaa taaccataaa atggaaatag tgctg
1255